# EUROPEAN PATENT APPLICATION

(11) **EP 4 588 489 A1**
(43) Date of publication of application: **23.07.2025**
(21) Application number: 23864829.9
(22) Date of filing: 18.09.2023
(51) Int. Cl.: A61K 47/68, A61K 47/51, A61K 31/4745, A61P 35/00, C07D 309/02, C07D 307/02, C07D 207/00, C07D 247/00

(54) **LINKER FOR ANTIBODY-DRUG CONJUGATE AND USE THEREOF**

(30) Priority: 16.09.2022 CN 202211168480
(71) Applicant: Shanghai Junshi Biosciences Co., Ltd., Pilot Free Trade Zone Shanghai 201210 (CN); Shanghai Allink Biotherapeutics Co., Ltd., Shanghai 201208 (CN)
(72) Inventor: JI, Ao, Suzhou, Jiangsu 215002 (CN); LI, Wenli, Suzhou, Jiangsu 215002 (CN); SUN, Chuchu, Suzhou, Jiangsu 215002 (CN); ZHAI, Shizhong, Shanghai 201400 (CN); LI, Yuying, Suzhou, Jiangsu 215002 (CN); ZHANG, Pengqi, Suzhou, Jiangsu 215002 (CN); GAO, Cuicui, Suzhou, Jiangsu 215002 (CN); LI, Xueting, Shanghai 201400 (CN); ZHANG, Mei, Suzhou, Jiangsu 215002 (CN); FENG, Hui, Shanghai 201210 (CN)
(74) Representative: V.O.
(86) International application number: PCT/CN2023/119531
(87) International publication number: WO 2024/056101

(57) **Abstract**

The present invention relates to a linker for an antibody-drug conjugate and a use thereof. Specifically, a linker represented by formula I is provided. An antibody-drug conjugate prepared by means of the linker are more stable and more effective, and can be effectively used for treating various diseases such as tumors. Further provided are an antibody-drug conjugate prepared using the linker and a use of the antibody-drug conjugate in treating tumors or other diseases.

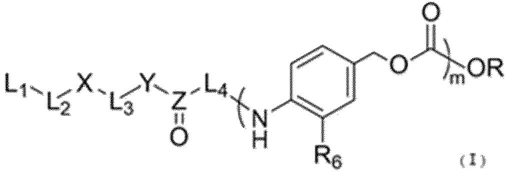

## Description

### TECHNICAL FIELD

The present disclosure relates to the technical field of bio-pharmaceuticals and particularly to a linker for an antibody-drug conjugate, an antibody-drug conjugate prepared using the linker, and use of the antibody-drug conjugate in the treatment of tumors or other diseases.

### BACKGROUND

Antibody-drug conjugates (ADCs) are a novel class of targeted-therapy drugs primarily used in the treatment of diseases such as cancers, autoimmune diseases, and infections. Antibody-drug conjugates typically consist of three components: an antibody or antibody ligand, a small-molecule drug, and a linker that conjugates the ligand to the drug. The mechanism of action of antibody-drug conjugates is as follows: the antibody or antibody ligand specifically recognizes and binds to a cell surface antigen; the resulting complex enters the cell via endocytosis, carrying the small-molecule drug along with it; the antibody is degraded by enzymes or the linker is cleaved, releasing the small-molecule drug in an appropriate active form to kill the target cell.

The linker is a core component in ADC drug design and is a key to achieving targeted drug release. When delivering hydrophobic cytotoxic drugs, hydrophobic linkers can increase conjugate aggregation or reduce antibody affinity, especially when the drug load is high. Meanwhile, drug-resistant tumor cells may limit the activity of ADCs, often due to increases in expression or activity of drug transport proteins that accelerate the efflux of hydrophobic compounds. Thus, one of the challenges in ADC design and development is the creation of hydrophilic linkers suitable for conjugating the antibody to the drug. By using hydrophilic linkers, it is possible to achieve higher drug loads while avoiding the local intensive distribution of drugs (mostly hydrophobic), which can lead to hydrophobic aggregation and thus reduced efficacy.

Given the above limitations of existing antibody-drug conjugate preparation technologies, there is a need to develop new linker technologies to produce conjugates that are more stable and more effective.

### SUMMARY

A first aspect of the present disclosure provides a linker having a structure represented by formula A: 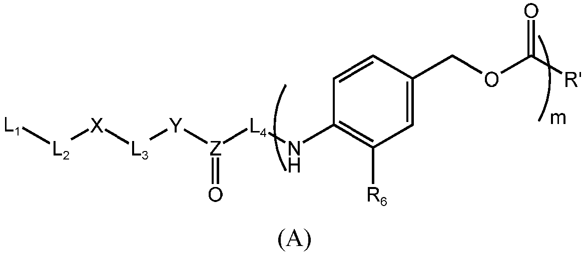 wherein L₁ is a functional group capable of reacting with mercapto; L₂ and Y are each independently selected from -(CH₂)ₚ-, -(OCH_{2C}H₂)ₚ-, and -(CH₂CH₂O)ₚ-; L₃ is a hydrophilic group; L₄ is an amino acid group; R' is -OR or -NR"-C₁-C₆ alkylene-NR"-R‴; R" and R"' are each independently H and C₁-C₆ alkyl; R is selected from H and phenyl optionally substituted with one or more nitro groups; R₆ is selected from H, C₁-C₆ alkoxy, -(OCH₂CH₂)ₚO-C₁-C₆ alkyl, and -(CH₂CH₂O)ₚ-C₁-C₆ alkyl; X is selected from -NH-, -NH(CH₂)ₙC(=O)-, -C(=O)(CH₂)ₙNH-, and -(CH₂)ₙC(=O)-; Z is selected from C and S; m is selected from 0 and 1; n is selected from 0, 1, and 2; p is selected from an integer from 1 to 10.

A second aspect of the present application provides a linker-drug conjugate having a structure represented by formula XIII': 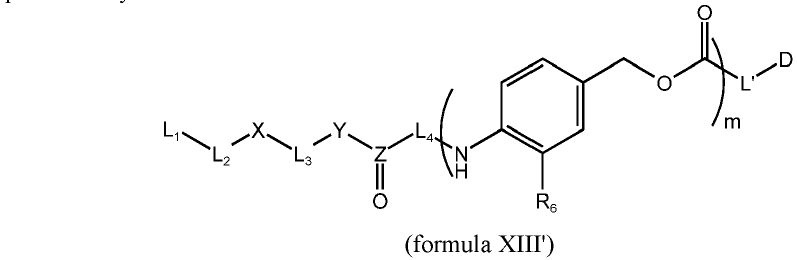 wherein L₁, L₂, L₃, L₄, X, Y, Z, R₆, and m are as described in the first aspect; L' is a bond, -NR"-C₁-C₆ alkylene-NR"-CO-*, or -NH-CH₂-O-; D is a drug moiety; each R" is independently H and C₁-C₆ alkyl; * indicates a position where L' is connected to D.

A third aspect of the present application provides an antibody-drug conjugate or a pharmaceutically acceptable salt thereof, having a structure represented by formula XV:

Ab-(L-D)ₛ (formula XV)

wherein L is the linker according to the first aspect; D is the drug according to the second aspect; s represents an average number of L-D units conjugated to Ab, and s ranges from 1 to 8; Ab is a targeting moiety.

A fourth aspect of the present application provides a connector for connecting a drug to a targeting moiety to form a targeting moiety-drug conjugate, having the following structure:

-L₁-L₂-X-L₃-Y-Z(=O)-L₄-(L₅)m-L₆-,

wherein in the formula, L₁ is selected from -(maleimido-3-yl-N)-, -C(=O)-, -(CH₂)ₙC(=O)-, - S(=O)-, -(CH₂)ₙS(=O)-, -CH₂-(CH₂)ₙ-, and -CH=CH-; L₂ and Y are each independently selected from -(CH₂)ₚ-, -(OCH_{2C}H₂)ₚ-, and -(CH₂CH₂O)ₚ-; L₃ is a hydrophilic group; L₄ is an amino acid group; L₅ is: 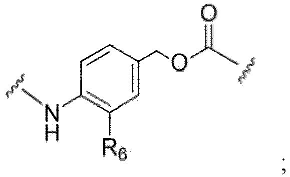 R₆ is selected from H, C₁-C₆ alkoxy, -(OCH₂CH₂)ₚO-C₁-C₆ alkyl, and -(CH₂CH₂O)ₚ-C₁-C₆ alkyl; L₆ is a bond, -NR"-C₁-C₆ alkylene-NR"-, or -NR"-C₁-C₆ alkylene-NR"-CO-; each R" is independently H or C₁-C₆ alkyl; preferably, each R" is independently H, methyl, or ethyl; more preferably, each R" is methyl; X is selected from -NH-, -NH(CH₂)ₙC(=O)-, -C(=O)(CH₂)ₙNH-, and -(CH₂)ₙC(=O)-; Z is selected from C and S; n is selected from 0, 1, and 2; p is selected from an integer from 1 to 10; m is selected from 0 and 1; wherein L₁ is used for connecting to the targeting moiety, and the other end of the connector is used for connecting to the drug; wherein when m is 0, the drug is connected to the carbonyl end (C(=O)) of L₄; when m is 1, the drug is connected to the carbonyl end (C(=O)) of L₅, and L₄ is connected to the amino end (NH) of L₅.

The present disclosure further provides a compound comprising the connector according to any one of the embodiments of the fourth aspect herein. The compound may be a linker-drug conjugate or an antibody-connector-drug conjugate. In the compound, the connector is a portion of the compound and is used for covalently connecting to the remainder of the compound. In some embodiments, the linker-drug conjugate or antibody-connector-drug conjugate is as described in any one of the embodiments of the present application.

The present application further provides a pharmaceutical composition comprising any one of the antibody-drug conjugates or pharmaceutically acceptable salts thereof described herein and a pharmaceutically acceptable carrier or auxiliary material.

The present application further provides use of any one of the linkers described herein in the preparation of a linker-drug conjugate or an antibody-drug conjugate.

The present application further provides use of any one of the antibody-drug conjugates or pharmaceutically acceptable salts thereof described herein, or any one of the pharmaceutical compositions described herein, in the preparation of a medicament for treating and/or preventing a cancer, an autoimmune disease, an inflammatory disease, or an infectious disease.

The present application further provides any one of the antibody-drug conjugates or pharmaceutically acceptable salts thereof described herein or any one of the pharmaceutical compositions described herein for use in treating and/or preventing a cancer, an autoimmune disease, an inflammatory disease, or an infectious disease.

The present application further provides a method for treating and/or preventing a cancer, an autoimmune disease, an inflammatory disease, or an infectious disease, the method comprising administering to a subject in need thereof any one of the antibody-drug conjugates or pharmaceutically acceptable salts thereof described herein or any one of the pharmaceutical compositions described herein.

### BRIEF DESCRIPTION OF THE DRAWINGS

FIG. 1: the anti-tumor effects of the antibody-drug conjugates in a subcutaneous xenograft tumor model of human lung squamous carcinoma cells (EBC-1).
FIG. 2: body weight test results for the antibody-drug conjugates in a subcutaneous xenograft tumor model of human lung squamous carcinoma cells (EBC-1).
FIG. 3: the anti-tumor effects of the antibody-drug conjugates in a subcutaneous xenograft tumor model of human lung adenocarcinoma cells (Calu-3).
FIG. 4: body weight test results for the antibody-drug conjugates in a subcutaneous xenograft tumor model of human lung adenocarcinoma cells (Calu-3).
FIG. 5: a comparison of the proliferation-inhibiting effects of the candidate molecules and the negative control ADC on BxPC-3 cells *in vitro.*
FIG. 6: a comparison of the proliferation-inhibiting effects of the candidate molecules and the negative control ADC on BxPC-3 cells *in vitro.*
FIG. 7: a comparison of the proliferation-inhibiting effects of the candidate molecules and the negative control ADC on BxPC-3 cells *in vitro.*
FIG. 8: a comparison of the abilities of the candidate molecules and the negative control ADC to inhibit MM.1S cell proliferation.
FIG. 9: a comparison of the abilities of the candidate molecules and the negative control ADC to inhibit MM.1S cell proliferation.

### DETAILED DESCRIPTION

The present disclosure is described below in detail. Prior to the description, it should be understood that the terms used in the specification and the appended claims should not be interpreted as being limited to the general meanings and dictionary meanings but should be interpreted according to the corresponding meanings and concepts in the technical aspects of the present disclosure on the basis of the principle that the inventors are allowed to define the terms appropriately to achieve the best interpretation. Therefore, the description proposed here is only a preferred example for illustrative purposes and is not intended to limit the scope of the present disclosure. Hence, it should be understood that other equivalents or improvements can be obtained from it without departing from the spirit and scope of the present disclosure.

### Terminology

In order to facilitate the understanding of the present disclosure, certain technical and scientific terms are specifically defined below. Unless otherwise specifically stated herein, all technical and scientific terms used herein have the meanings as commonly understood by those of ordinary skill in the art to which the present disclosure pertains.

As used herein, "a" or "an" may mean one or more. As used in one or more claims, when used in conjunction with the word "comprise", the words "a" or "an" may mean one or more than one. As used herein, "another" may mean at least a second or more.

As used herein, when referring to a measurable value (such as an amount or a duration), "about" is meant to encompass variations of ±20% or ±10%, including ±5%, ±1%, and ±0.1%, relative to the specified value, as such variations are appropriate for performing the disclosed methods.

The term "and/or" should be construed as meaning any one of the options or a combination of any two or more of the options.

As used herein, "linker" has two reactive ends, one for binding to or associating with a biological moiety or a fragment thereof, such as an antibody or a fragment thereof, and the other for conjugating to an active agent, such as a cytotoxin.

The term "alkyl" as used herein refers to a straight-chain or branched-chain saturated hydrocarbon group that may have 1-12, e.g., 1-8, 1-6, or 1-4, carbon atoms. Exemplary alkyl groups include methyl, ethyl, propyl (e.g., 1-propyl or isopropyl), butyl (e.g., n-butyl, isobutyl, or tert-butyl), etc. "Alkenyl" is a straight-chain or branched-chain unsaturated hydrocarbon group containing at least one carbon-carbon sp2 double bond (C=C). The number of carbon atoms of alkenyl groups is usually 2-8, such as 2-6 or 2-4. Exemplary alkenyl groups include ethenyl, allyl, etc.

"Alkynyl" is a straight-chain or branched-chain unsaturated hydrocarbon group containing at least one carbon-carbon sp triple bond (C≡C). The number of carbon atoms of alkynyl groups is usually 2-8, such as 2-6 or 2-4. Exemplary alkynyl groups include ethynyl, propargyl, etc.

"Carbocyclyl" refers to a saturated or partially saturated cyclic hydrocarbon group, including saturated cycloalkyl groups and partially saturated cycloalkenyl and cycloalkynyl groups. The number of ring carbon atoms of carbocyclyl groups may be 3-9, such as 3-8 or 3-6. Exemplary cycloalkyl groups include cyclopropyl, cyclobutyl, cyclopentyl, cyclohexyl, etc.

"Aldehyde group" refers to the R-C(O)- group, where R can be an alkyl, alkenyl, or alkynyl group as described herein. Preferred aldehyde groups are C1-C6 aldehyde groups, i.e., C₁-C₅ alkyl-C(O)-. "Alkanone" refers to a straight-chain or branched-chain group or compound containing - CH₂COCH₂-, or a cyclic, saturated or unsaturated group or compound.

"Silane" may be represented as SiₙH₂ₙ₊₂, where n is an integer. For example, silyl may be represented as -SiH₃.

"Isocyanate group" generally refers to -N=C=O.

"Sulfonyl" refers to the R-S(O)₂- group, where R can be an alkyl, alkenyl, or alkynyl group as described herein.

"Halogen" refers to F, Cl, Br, or I.

"Alkoxy" refers to the R-O- group, where R can be an alkyl group as described herein, such as C₁-C₆ alkyl or C₁-C₃ alkyl.

"Aryl" refers to a monovalent aromatic hydrocarbon group of 6-14 carbon atoms resulting from the removal of one hydrogen atom from a carbon atom of the parent aromatic ring system, such as phenyl, naphthyl, anthryl, or biphenyl.

"Heteroaryl" refers to a group formed by substituting one or more carbon atoms of the skeleton of "aryl" as described above with one or more heteroatoms selected from N, O, P, and S. Heteroaryl groups may generally contain 5-14, preferably 5-10, ring atoms and have 6, 10, or 14 electrons shared in the ring system. Exemplary heteroaryl groups include, but are not limited to, pyridine, thiophene, furan, pyrrolyl, pyrazolyl, thiazolyl, etc.

"Heterocyclyl" refers to a group formed by substituting one or more carbon atoms of the parent aromatic or non-aromatic ring system with one or more heteroatoms selected from N, O, P, and S. Heterocyclyl groups may include saturated or partially saturated 3- to 7-membered monocyclic groups, 7- to 10-membered bicyclic groups, spiro-ring groups, or bridged-ring groups. Exemplary heterocyclyl groups include tetrahydrofuranyl, pyranyl, piperidinyl, piperazinyl, 1,4-diazepanyl, azetidinyl, oxetanyl, pyrrolidinyl, imidazolidinyl, imidazolinyl, indolinyl, isoindolinyl, morpholinyl, etc.

"Aralkyl" refers to an aliphatic alkyl group in which a hydrogen atom connected to the terminal or sp3 carbon atom is substituted with an aryl group, such as benzyl or 3-phenylpropyl. The alkyl portion of an aralkyl group may also include alkenyl or alkynyl groups.

"Heteroaralkyl" refers to an aliphatic alkyl group in which a hydrogen atom connected to the terminal or sp3 carbon atom is substituted with a heteroaryl group, such as 2-pyridylethyl or 3-furylpropyl. The alkyl portion of a heteroaralkyl group may also include alkenyl or alkynyl groups. "Alkylene" is a saturated hydrocarbon group containing a straight chain, a branched chain, or a carbocycle in which the two monovalent radical centers result from the removal of two hydrogen atoms from the same carbon atom or two different carbon atoms of the parent alkane, such as methylene (-CH₂-), 1,2-ethylene (-CH₂CH₂-), or 1,3-propylene (-CH₂CH₂CH₂-).

"Alkenylene" is an unsaturated hydrocarbon group containing a straight chain, a branched chain, or a carbocycle in which the two monovalent radical centers result from the removal of two hydrogen atoms from the same carbon atom or two different carbon atoms of the parent alkene, such as 1,2-ethenylene (-CH=CH-) or 1,3-propenylene (-CH₂CH=CH-).

"Alkynylene" is an unsaturated hydrocarbon group containing a straight chain, a branched chain, or a carbocycle in which the two monovalent radical centers result from the removal of two hydrogen atoms from the same carbon atom or two different carbon atoms of the parent alkyne, such as ethynylene (-CH=CH-) or 1,3-propynylene (-CH₂C≡CH-).

"Arylene" refers to an aromatic hydrocarbon group of 6-14 carbon atoms in which the two monovalent radical centers result from the removal of two hydrogen atoms from two different carbon atoms of the parent aromatic ring system, such as 1,2-phenylene, 1,3-phenylene, or 1,4-phenylene.

Whenever a group is described herein as "substituted", it is substituted with one or more (e.g., 1, 2, 3, 4, or 5 or more) specified substituents. If no substituent is indicated, it means that the indicated "substituted" group may be substituted with one or more (e.g., 1, 2, 3, 4, or 5 or more) groups individually and independently selected from alkyl, alkenyl, alkynyl, cycloalkyl, cycloalkenyl, cycloalkynyl, heteroalkyl, aryl, heteroaryl, heteroalicyclyl, aralkyl, heteroaralkyl, (heteroalicyclyl)alkyl, hydroxyl, alkoxy, aryloxy, acyl, mercapto, alkylthio, arylthio, cyano, halogen, thiocarbonyl, carbamoyl, thiocarbamoyl, amido, sulfonamido, sulfonamido, carboxyl, isocyanato, thiocyanato, isothiocyanato, nitro, silyl, sulfinyl, sulfinyl, sulfonyl, haloalkyl, haloalkoxy, trihalomethanesulfonyl, trihalomethanesulfonylamino, amino, mono-substituted amino and di-substituted amino, and protected derivatives thereof.

Where the number of substituents (e.g., haloalkyl) is not specified, one or more substituents may be present. For example, "haloalkyl" may comprise one or more of identical or different halogens. As another example, "C₁-C₃ alkoxyphenyl" may comprise one or more of identical or different alkoxy groups containing one, two, or three atoms.

In some embodiments, "substituted alkyl", "substituted alkenyl", "substituted alkynyl", "substituted aryl", "substituted heteroaryl", "substituted heterocyclyl", "substituted aralkyl", and "substituted heteroaralkyl" mean that one or more hydrogen atoms of the corresponding "alkyl", "alkenyl", "alkynyl", "aryl", "heteroaryl", "heterocyclyl", "aralkyl", and "heteroaralkyl" are each independently substituted with a substituent. Substituents generally include, but are not limited to, -X, -OR, -NR₂, -NO₂, -CN, -SO₃R, -CO₂R, etc., wherein X is a halogen atom; R is H, alkyl, aryl, heterocyclyl, a protecting group, or a prodrug moiety. The above alkylene, alkenylene, and alkynylene groups may also be similarly substituted.

"Any combination" refers to a new substituent formed by connecting two or more substituents in a certain way, such as benzyl (phenyl + methylene), 3-phenylpropyl (phenyl + 1,3-propylene), 2-cyclohexylpropyl (cyclohexyl + 1,2-propylene), or 3-(3-pyridyl)propyl (3-pyridyl + 1,3-propylene). When a group is defined as a "bond", it generally means that the group is a monovalent covalent bond. For example, when L₆ is a bond, the structural formula of the connector -L₁-L₂-X-L₃-Y-Z(=O)-L₄-(L₅)ₘ-L₆- is actually -L₁-L₂-X-L₃-Y-Z(=O)-L₄-(L₅)ₘ-.

The term "drug/antibody ratio (DAR)" as used herein refers to the number of drugs conjugated per antibody molecule. Because antibody-drug conjugate samples contain multiple components with different DAR values, the two concepts "average DAR value" and "DAR value distribution" are more suitable for describing the composition of an antibody-drug conjugate. The average DAR value is the ratio of the total number of drug molecules to the total number of antibodies in a sample, and the DAR value distribution refers to the content distribution of the components with different DAR values in a sample.

The term "drug" in the present disclosure loosely refers to any compound that has desirable bioactivities and has a reactive functional group so that the conjugates described herein can be prepared. Desirable bioactivities include diagnosing, curing, alleviating, treating, and preventing diseases in humans or other animals. As novel drugs are continually discovered and developed, these novel drugs shall also be incorporated into the drugs described herein. Specifically, the drugs include, but are not limited to, cytotoxic drugs, cell differentiation factors, stem cell trophic factors, steroid drugs, drugs for treating autoimmune diseases, anti-inflammatory drugs, or drugs for infectious diseases. More specifically, the drugs include, but are not limited to, tubulin inhibitors or DNA or RNA damaging agents.

The term "antibody" in the present disclosure is used in the broadest sense and encompasses a variety of antibody structures including, but not limited to, monoclonal antibodies, polyclonal antibodies, multispecific antibodies (e.g., bispecific antibodies), and antibody fragments. Particularly, "antibody" as used herein refers to a protein comprising at least two heavy (H) chains and two light (L) chains interconnected by disulfide bonds. Each of the heavy chains comprises a heavy chain variable region (abbreviated as VH) and a heavy chain constant region. The heavy chain constant region comprises three domains, i.e., CH1, CH2, and CH3. Each of the light chains comprises a light chain variable region (abbreviated as VL) and a light chain constant region. The light chain constant region comprises one domain, i.e., CL. The VH and VL regions can be further subdivided into multiple hypervariable regions known as complementarity determining regions (CDRs), between which more conservative regions known as framework regions (FRs) are distributed. Each of VH and VL is composed of three CDRs and four FRs, arranged from the amino terminus to the carboxyl terminus in the following order: FR1, CDR1, FR2, CDR2, FR3, CDR3, FR4. These variable regions of the heavy and light chains comprise binding domains that interact with antigens. The constant region of an antibody can mediate the binding of the immunoglobulin to host tissues or factors, including various cells of the immune system (e.g., effector cells) and the first component (Clq) of the classical complement system. Chimeric or humanized antibodies are also encompassed by the antibodies according to the present disclosure.

The term "humanized antibody" refers to an antibody comprising CDRs derived from a non-human antibody, and the remainder of the antibody molecule is derived from one human antibody (or more human antibodies). Moreover, some residues of the backbone (known as FR) segment may be modified for the retention of the binding affinity (Jones et al., Nature, 321:522-525, 1986; Verhoeyen et al., Science, 239:1534-1536, 1988; Riechmann et al., Nature, 332:323-327, 1988). Humanized antibodies or fragments thereof according to the present disclosure can be prepared using techniques known to those skilled in the art (e.g., as described in Singer et al., J. Immun., 150:2844-2857, 1992; Mountain et al., Biotechnol. Genet. Eng. Rev., 10:1-142, 1992; or Bebbington et al., Bio/Technology, 10:169-175, 1992).

The term "chimeric antibody" refers to an antibody in which the variable region sequences are from one species and the constant region sequences are from another species, e.g., an antibody in which the variable region sequences are from a mouse antibody and the constant region sequences are from a human antibody. The chimeric antibodies or fragments thereof according to the present disclosure can be prepared by using genetic recombination techniques. For example, the chimeric antibody can be produced by cloning a recombinant DNA comprising a promoter and sequences encoding variable regions of the non-human, especially murine, monoclonal antibody according to the present disclosure, and sequences encoding constant regions of a human antibody. The chimeric antibodies of the present disclosure encoded by such recombinant genes will be, for example, murine-human chimeras whose specificity is determined by the variable regions derived from murine DNA and whose isotypes are determined by the constant regions derived from human DNA. For a method of preparing a chimeric antibody, reference can be made, for example, to Verhoeyn et al. (BioEssays, 8:74, 1988).

The term "monoclonal antibody" refers to preparations of antibody molecules having a single molecular composition. A monoclonal antibody composition exhibits a single binding specificity and affinity for a particular epitope.

The term "functional fragment" in the present disclosure means that the antibody fragment is composed of or comprises a portion of the sequence of the heavy or light variable chain of the antibody from which it is derived, and that the portion of the sequence is sufficient for the retention of the same binding specificity as the antibody from which it is derived and sufficient affinity, preferably at least 1/100 of the affinity of the antibody from which it is derived, and more preferably at least 1/10. Such functional fragments will comprise at least 5 amino acids, preferably 10, 15, 25, 50, and 100 contiguous amino acids of the antibody sequence from which they are derived.

In the present disclosure, the antibodies constituting the antibody-drug conjugates preferably retain their original wild-state antigen-binding ability. Thus, the antibodies in the present disclosure are capable of, preferably specifically, binding to antigens. Involved antigens include, for example, tumor-associated antigens (TAAs), cell surface receptor proteins, and other cell surface molecules, cell survival regulators, cell proliferation regulators, molecules associated with tissue growth and differentiation (e.g., known or predicted to be functional), lymphokines, cytokines, molecules involved in the regulation of cell circulation, molecules involved in angiogenesis, and molecules associated with angiogenesis (e.g., known or predicted to be functional). A tumor-associated factor may be a cluster differentiation factor (e.g., a CD protein). The antigen to which the antibody of the present disclosure binds may be one or a subset of the above categories, and other subsets include other molecules/antigens (as compared to the target antigen) having special properties.

Antibodies used in the antibody-drug conjugates include, but are not limited to, antibodies against cell surface receptors and tumor-associated antigens. Such tumor-associated antigens are well known in the art and can be prepared by antibody preparation methods and information well known in the art. In order to develop effective cellular-level targets for cancer diagnosis and treatment, researchers have sought transmembrane or other tumor-associated polypeptides. These targets can be specifically expressed on the surface of one or more types of cancer cells, while being minimally expressed or not expressed at all on the surface of one or more types of non-cancer cells. Generally, such tumor-associated polypeptides are more overexpressed on the surface of cancer cells than on the surface of non-cancer cells. The identification of such tumor-associated factors can greatly improve the specific targeting property of antibody-based cancer treatment.

Tumor-associated antigens include, but are not limited to, the tumor-associated antigens (1)-(36) listed below. For convenience, information related to antigens well known in the art is shown below, including their names, alternative names, and Genbank accession numbers. The nucleic acid and protein sequences corresponding to tumor-associated antigens can be found in publicly available databases, such as Genbank. Corresponding tumor-associated antigens targeted by antibodies include all amino acid sequence variants and isoforms that have at least 70%, 80%, 85%, 90%, or 95% homology with the sequences identified in the references or have biological properties and characteristics identical to those of the tumor-associated antigen sequences described in the references.

Tumor-associated antigens (1)-(36) are:
(1) BMPR1B (bone morphogenetic protein receptor-type IB, Genbank accession No. NM_001203);
(2) E16 (LAT1, SLC7A5, Genbank accession No. NM_003486);
(3) STEAP1 (six-transmembrane epithelial antigen of prostate, Genbank accession No. NM_012449);
(4) 0772P (CA125, MUC16, Genbank accession No. AF361486);
(5) MPF (MPF, MSLN, SMR, megakaryocyte potentiating factor, mesothelin, Genbank accession No. NM_005823);
(6) Napi3b (NAPI-3B, NPTIIb, SLC34A2, solute carrier family 34 (sodium phosphate) member 2, type II sodium-dependent phosphate transporter 3b, Genbank accession No. NM_006424);
(7) Sema 5b (FLJ10372, KIAA1445, Mm.42015, SEMA5B, SEMAG, semaphorin 5bHlog, sema domain, seven thrombospondin repeats (type 1 and type 1-like), transmembrane domain (TM) and short cytoplasmic domain, (semaphorin) 5B, Genbank accession No. AB040878);
(8) PSCA hlg (2700050C12Rik, C530008016Rik, RIKEN cDNA 2700050C12, RIKENcDNA 2700050C12 gene, Genbank accession No. AY358628);
(9) ETBR (endothelin type B receptor, Genbank accession No. AY275463);
(10) MSG783 (RNF124, hypothetical protein FLJ20315, Genbank accession No. NM_017763);
(11) STEAP2 (HGNC_8639, IPCA-1, PCANAP1, STAMP1, STEAP2, STMP, prostate cancer associated gene 1, prostate cancer associated protein 1, six-transmembrane epithelial antigen of prostate 2, six-transmembrane protein of prostate, Genbank accession No. AF455138);
(12) TrpM4 (BR22450, FLJ20041, TRPM4, TRPM4B, transient receptor potential cation channel, subfamily M, member 4, Genbank accession No. NM_017636);
(13) CRIPTO (CR, CR1, CRGF, CRIPTO, TDGF1, teratocarcinoma-derived growth factor, Genbank accession No. NP_003203 or NM_003212);
(14) CD21 (CR2 (complement receptor 2) or C3DR (C3d/EB virus receptor) or Hs.73792, Genbank accession No. M26004);
(15) CD79b (CD79B, CD79β, IGb (immunoglobulin-associated β), B29, Genbank accession No. NM_000626);
(16) FcRH2 (IFGP4, IRTA4, SPAP1A (containing the SH2 domain of phosphatase anchor protein 1a), SPAP1B, SPAP1C, Genbank accession No. NM_030764);
(17) HER2 (ErbB2, Genbank accession No. M11730);
(18) NCA (CEACAM6, Genbank accession No. M18728);
(19) MDP (DPEP1, Genbank accession No. BC017023);
(20) IL20Rα (IL20Ra, ZCYTOR7, Genbank accession No. AF184971);
(21) Brevican (BCAN, BEHAB, Genbank accession No. AF229053);
(22) EphB2R (DRT, ERK, Hek5, EPHT3, Tyro5, Genbank accession No. NM_004442);
(23) ASLG659 (B7h, Genbank accession No. AX092328);
(24) PSCA (prostate stem cell antigen precursor, Genbank accession No. AJ297436);
(25) GEDA (Genbank accession No. AY260763);
(26) BAFF-R (B cell-activating factor receptor, BLyS receptor 3, BR3, Genbank accession No. AF116456);
(27) CD22 (B-cell receptor CD22-B isoform, Genbank accession No. AK026467);
(28) CD79a (CD79A, CD79α, immunoglobulin-associated α, a B cell-specific protein that covalently interacts with Ig β (CD79B), forms a complex on the surface with an Ig M molecule, and transduces a signal involved in B-cell differentiation, Genbank accession No. NP_001774.1);
(29) CXCR5 (Burkitt's lymphoma receptor 1, a G protein-coupled receptor that is activated by the CXCL13 chemokine, functions in lymphocyte migration and humoral defense, and plays a role in HIV-2 infection and perhaps AIDS, lymphoma, myeloma, and leukemia, Genbank accession No. NP_001701.1);
(30) HLA-DOB (Beta subunit of MHC class II molecule (Ia antigen) that binds to peptides and presents them to CD4+ small T lymphocytes, Genbank accession No. NP_002111.1);
(31) P2X5 (purinergic receptor P2X ligand-gated ion channel 5, an ion channel gated by extracellular ATP, may be involved in synaptic transmission and neurogenesis, and deficiency may result in the pathophysiology of idiopathic detrusor instability, Genbank accession No. NP_002552.2);
(32) CD72 (B-cell differentiation antigen CD72, Lyb-2, Genbank accession No. NP_001773.1);
(33) LY64 (lymphocyte antigen 64 (RP105), type I membrane protein of the leucine-rich repeat (LRR) family, regulates B-cell activation and apoptosis, and loss of function is associated with increased disease activity in patients with systemic lupus erythematosus, Genbank accession No. NP_005573.1);
(34) FcRH1 (Fc receptor-like protein 1, a putative receptor for the immunoglobulin Fc domain that contains C2 type Ig-like and ITAM domains and may play a role in B-lymphocyte differentiation, Genbank accession No. NP_443170.1);
(35) IRTA2 (immunoglobulin superfamily receptor translocation associated 2, a putative immunoreceptor that may play a role in B-cell development and lymphomagenesis; dysregulation of the gene by translocation occurs in some B-cell malignancies, Genbank accession No. NP_112571.1); and
(36) TENB2 (a putative transmembrane proteoglycan that is associated with the EGF/heregulin family of growth factors and follistatin, Genbank accession No. AF179274).

As used herein, "drug" or the code "D" loosely refers to any compound that has desirable bioactivities and has a reactive functional group so that the conjugates described herein can be prepared. Desirable bioactivities include diagnosing, curing, alleviating, treating, and preventing diseases in humans or other animals. Thus, the compounds to which the term "drug" relates include drugs identified in the official national pharmacopeia as well as, e.g., the official Homeopathic Pharmacopoeia of the United States, the official National Formulary, or any supplements thereof, as long as they have the needed reactive functional group. Typical drugs are set forth in the Physicians' Desk Reference (PDR) and the Orange Book by the U.S. Food and Drug Administration (FDA). As novel drugs are continually discovered and developed, the present application stipulates that these drugs shall also be incorporated into the prodrugs of the conjugated drugs described herein.

The term "preventing" as used herein refers to a compound or drug that, when used for treating a disease or condition (e.g., cancer), can reduce the frequency of or delay the onset of symptoms of a medical condition in a subject as compared with a subject who has not been administered the compound or drug (e.g., a combination product as claimed by the present application).

The term "treating" as used herein refers to relieving, alleviating, or ameliorating a symptom of a disease or condition, ameliorating an underlying metabolic-induced symptom, and inhibiting a disease or symptom, e.g., arresting the progression of a disease or condition, alleviating a disease or condition, causing regression of a disease or condition, alleviating a disorder caused by a disease or condition, or arresting a symptom of a disease or condition.

The term "effective amount" or "prophylactically and/or therapeutically effective amount" as used herein refers to a sufficient amount (e.g., dose) of the drug or compound administered that will alleviate to some extent one or more symptoms of the disease or disorder being treated. The result may be a reduction and/or alleviation in the cause of the disorder or disease or any other desired changes in the biological system. For example, an "effective amount" for therapeutic use is an amount of a compound or drug (e.g., a combination product as claimed in the present application) that is provided such that the clinical symptoms of the disease or disorder are significantly alleviated without undue toxic and side effects.

"Administer" and "give" refer to introducing a composition comprising a therapeutic agent into a subject using any one of a variety of methods or delivery systems known to those skilled in the art. The terms "subject" and "individual" include any organism, preferably an animal, more preferably a mammal (such as a rat, mouse, dog, cat, or rabbit), and most preferably a human. The terms "subject" and "patient" are used interchangeably herein.

The term "pharmaceutically acceptable carrier or auxiliary material" refers to a carrier or auxiliary material that can be administered to a subject along with the compound according to one aspect of the present disclosure and, when administered at a dose sufficient to deliver a therapeutic amount of the compound, does not destroy its pharmacological activity and is non-toxic. Exemplary pharmaceutically acceptable carriers or auxiliary materials include inert diluents such as water or other solvents, solubilizing agents, and emulsifiers such as ethanol, isopropanol, ethyl carbonate, EtOAc, benzyl alcohol, benzyl benzoate, propylene glycol, 1,3-butylene glycol, dimethylformamide, oils (including cottonseed oil, peanut oil, corn oil, germ oil, olive oil, castor oil, and sesame oil), glycerol, tetrahydrofurfuryl alcohol, polyethylene glycol, and fatty acid esters of sorbitol, and mixtures thereof; and auxiliaries such as wetting agents, emulsifying and suspending agents, sweeteners, flavoring agents, and perfuming agents. The terms "mode of administration" and "dosing regimen" are used interchangeably and refer to the dose and time of use of each therapeutic agent in the combination of the present disclosure.

"Cancer" and "cancerous" refer to or describe the physiological condition in mammals which is typically characterized by unregulated cell growth. Included in this definition are benign and malignant cancers as well as dormant tumors or micrometastases. Examples of cancers include, but are not limited to, carcinoma, lymphoma, blastoma, sarcoma, and leukemia. More specific examples of such cancers include squamous cell carcinoma, lung cancer (including small cell lung cancer, non-small cell lung cancer, adenocarcinoma of the lung and squamous carcinoma of the lung), peritoneal cancer, hepatocellular cancer, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland carcinoma, renal cancer or cancer of the kidney, liver cancer, prostatic cancer, vulval cancer, thyroid cancer, cancer of the liver, and various types of head and neck cancers, as well as B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphomas, and Waldenstrom's macroglobulinemia), chronic lymphocytic leukemia (CLL), acute lymphoblastic leukemia (ALL), hairy cell leukemia, chronic myeloblastic leukemia, post-transplant lymphoproliferative disorder (PTLD), and abnormal vascular proliferation associated with phakomatoses, edema (such as associated with brain tumors) and Meigs syndrome.

### Linker

In a first aspect, the present application provides a linker having a structure represented by formula A: 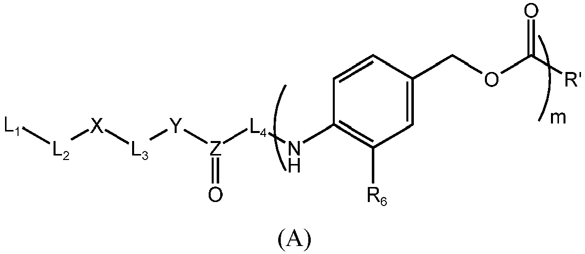 wherein L₁ is a functional group capable of reacting with mercapto; L₂ and Y are each independently selected from -(CH₂)ₚ-, -(OCH_{2C}H₂)ₚ-, and -(CH₂CH₂O)ₚ-; L₃ is a hydrophilic group; L₄ is an amino acid group; R' is -OR or -NR"-C₁-C₆ alkylene-NR"-R‴; R" and R"' are each independently H and C₁-C₆ alkyl; R is selected from H and phenyl optionally substituted with one or more nitro groups; R₆ is selected from H, C₁-C₆ alkoxy, -(OCH₂CH₂)ₚO-C₁-C₆ alkyl, and -(CH₂CH₂O)ₚ-C₁-C₆ alkyl; X is selected from -NH-, -NH(CH₂)ₙC(=O)-, -C(=O)(CH₂)ₙNH-, and -(CH₂)ₙC(=O)-; Z is selected from C and S; m is selected from 0 and 1; n is selected from 0, 1, and 2; p is selected from an integer from 1 to 10.

In some embodiments, the present disclosure provides a linker having a structure represented by formula I: 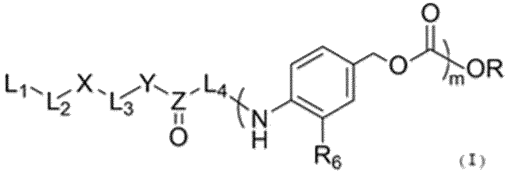 wherein L₁ is a functional group capable of reacting with mercapto; L₂ and Y are each independently selected from -(CH₂)ₚ-, -(OCH₂CH₂)ₚ-, and -(CH₂CH₂O)ₚ-; L₃ is a hydrophilic group; L₄ is an amino acid group; R is selected from H and phenyl optionally substituted with one or more nitro groups; R₆ is selected from H, C₁-C₆ alkoxy, -(OCH₂CH₂)ₚO-C₁-C₆ alkyl, and -(CH₂CH₂O)ₚ-C₁-C₆ alkyl; X is selected from -NH-, -NH(CH₂)ₙC(=O)-, -C(=O)(CH₂)ₙNH-, and -(CH₂)ₙC(=O)-; Z is selected from C and S; m is selected from 0 and 1; n is selected from 0, 1, and 2; p is selected from an integer from 1 to 10, e.g., 1-8 or 1-6.

In one or more embodiments, L₁ is selected from at least one of maleimide, halogen, halogen-substituted functional groups (e.g., halogen-substituted aldehyde groups or halogen-substituted - S(O)₂-), aldehyde groups, alkenyl, alkynyl, alkanones, sulfonyl, silane, isocyanate groups, and norbornenyl.

In some embodiments, the norbornenyl has the following structure: 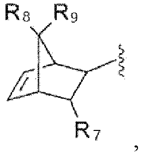 wherein in the formula, R₇, R₈, and R₉ are each independently selected from H, halogen, C₁-C₆ alkyl, C₂-C₆ alkenyl, C₂-C₆ alkynyl, aryl, heteroaryl, C₃-C₉ cycloalkyl, C₃-C₉ heterocyclyl, - (OCH₂CH₂)ₚOCH₃, -NO₂, -CN, -SCN, -OR₁₀, -SR₁₀, -N(R₁₀)₂, -C(=O)R₁₀, -C(=O)OR₁₀, - C(=O)N(R₁₀)₂, -C(=S)OR₁₀, -C(=S)N(R₁₀)₂, -C(=S)SR₁₀, -NR₁₀(C=O)R₁₀, -NR₁₀(C=S)N(R₁₀)₂, - O(C=O)N(R₁₀)₂, -S(=O)₂R₁₀, and -S(=O)₂OR₁₀, wherein each R₁₀ is independently selected from H, C₁-C₆ alkyl, C₂-C₆ alkenyl, and C₂-C₆ alkynyl.

In one or more embodiments, L₁ is selected from: 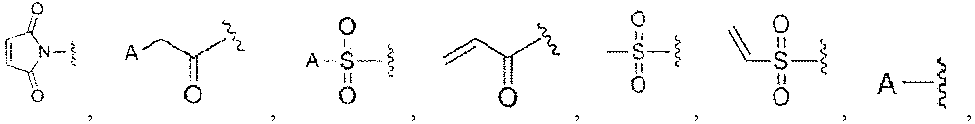 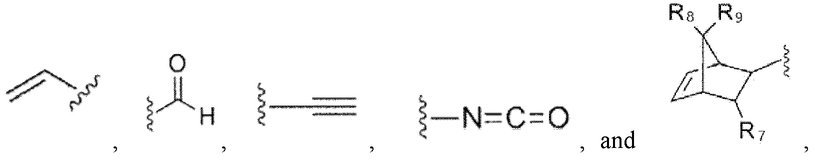 and derivatives thereof;
wherein R₇, R₈, and R₉ are each independently selected from H, halogen, C₁-C₆ alkyl, C₂-C₆ alkenyl, C₂-C₆ alkynyl, aryl, heteroaryl, C₃-C₉ cycloalkyl, C₃-C₉ heterocyclyl, -(OCH₂CH₂)ₚOCH₃, -NO₂, - CN, -SCN, -OR₁₀, -SR₁₀, -N(R₁₀)₂, -C(=O)R₁₀, -C(=O)OR₁₀, -C(=O)N(R₁₀)₂, -C(=S)OR₁₉, - C(=S)N(R₁₀)₂, -C(=S)SR₁₀, -NR₁₀(C=O)R₁₀, -NR₁₀(C=S)N(R₁₀)₂, -O(C=O)N(R₁₀)₂, -S(=O)₂R₁₀, and -S(=O)₂OR₁₀, wherein each R₁₀ is independently selected from H, C₁-C₆ alkyl, C₂-C₆ alkenyl, and C₂-C₆ alkynyl; p is an integer from 1 to 6; A is halogen; the wavy line indicates a position where L₁ is connected to L₂.

In one or more embodiments, L₁ is:

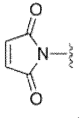

In one or more embodiments, L₂ is -(CH₂)ₚ-. In one or more embodiments, L₂ is -(CH₂)ₚ-, and p is an integer from 1 to 4. In one or more embodiments, L₂ is -CH₂CH₂- or -CH₂CH₂CH₂-.

In one or more embodiments, X is -NH- or -NH(CH₂)ₙC(=O)-. In some embodiments, X is -NH- or -NHC(=O)-.

In one or more embodiments, L₃ is selected from divalent hydrophilic groups with two monovalent radical centers resulting from the removal of two hydrogen atoms from a monosaccharide, a disaccharide, and a five- or six-membered saturated heterocycle containing 1-2 nitrogen atoms and a derivative thereof.

In one or more embodiments, the monosaccharide is selected from at least one of a triose, a tetrose, a pentose, a hexose, and a heptose; the disaccharide is selected from at least one of maltose, sucrose, and lactose; the five- or six-membered saturated heterocycle containing 1-2 nitrogen atoms may be piperazinyl, piperidinyl, pyrrolidinyl, etc. The derivative may be formed by substituting the H of one or more hydroxyl groups on the monosaccharide or disaccharide with a group selected from, e.g., C₁-C₆ alkyl, -(CH₂CH₂O)ₜCH₃, a sulfonic acid group, and a phosphoric acid group, wherein t is selected from an integer from 1 to 20; or one or more ring carbon atoms of the five- or six-membered saturated heterocycle containing 1-2 nitrogen atoms are substituted with one or more groups selected from -OR₂₀ and -CH₂R₂₀, and R₂₀ may be C₁-C₆ alkyl, -(CH₂CH₂O)ₜCH₃, a sulfonic acid group, or a phosphoric acid group, wherein t is selected from an integer from 1 to 20.

In one or more embodiments, L₃ is selected from divalent hydrophilic groups with two monovalent radical centers resulting from the removal of two hydrogen atoms from glucose, galactose, mannose, glucuronic acid, galactonic acid, mannuronic acid, N-acetylglucosamine, N-acetylgalactosamine, N-acetylmannosamine, and N-acetylmuramic acid.

In one or more embodiments, L₃ is selected from: 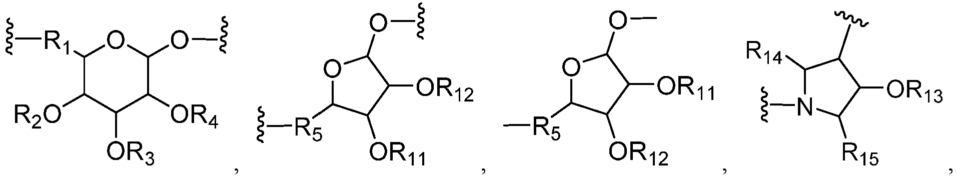 and 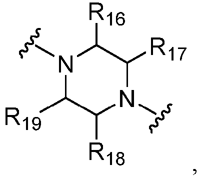 wherein in the formulas, R₁ is selected from -O(CH₂)ₚ- and -C(=O)-, and p is an integer from 1 to 6, preferably 1, 2, 3, or 4; R₅ is a bond or C₁-C₃ alkylene unsubstituted or substituted with one or more substituents selected from -OR₂₀ and -C(=O)OR₂₀-; R₂, R₃, R₄, R₁₁, R₁₂, and R₁₃ are independently selected from H, C₁-C₆ alkyl, -(CH₂CH₂O)ₜ-C₁-C₆ alkyl, a sulfonic acid group, and a phosphoric acid group; R₁₄ and R₁₅ are independently selected from H, -OR₂₀, and -CH₂R₂₀; R₁₆, R₁₇, R₁₈, and R₁₉ are independently selected from Hand -OR₂₀; R₂₀ is selected from H, C₁-C₆ alkyl, -(CH₂CH₂O)ₜ-C₁-C₆ alkyl, a sulfonic acid group, and a phosphoric acid group; t is an integer from 1 to 20; the wavy lines indicate positions where L₃ is connected to X and Y.

In one or more embodiments, R₁ is selected from -OCH₂- and -C(=O)-.

In one or more embodiments, R₅ is unsubstituted C₁-C₃ alkylene or is C₁-C₃ alkylene substituted with one or more substituents selected from -OR₂₀ and -C(=O)OR₂₀-, wherein R₂₀ is selected from H, C₁-C₆ alkyl, -(CH₂CH₂O)ₜCH₃, a sulfonic acid group, and a phosphoric acid group, wherein t is selected from an integer from 1 to 10.

In one or more embodiments, R₁₄ and R₁₅ are independently selected from H, -OR₂₀, and -CH₂R₂₀, wherein R₂₀ is selected from H, C₁-C₆ alkyl, -(CH₂CH₂O)ₜCH₃, a sulfonic acid group, and a phosphoric acid group, wherein t is selected from an integer from 1 to 10.

In one or more embodiments, R₁₆, R₁₇, R₁₈, and R₁₉ are independently selected from H and -OR₂₀, wherein R₂₀ is selected from H, C₁-C₆ alkyl, -(CH₂CH₂O)ₜCH₃, a sulfonic acid group, and a phosphoric acid group, wherein t is selected from an integer from 1 to 10.

In one or more embodiments, R₂, R₃, R₄, R₁₁, R₁₂, and R₁₃ are independently selected from H, C₁-C₆ alkyl, -(CH₂CH₂O)ₜCH₃, a sulfonic acid group, and a phosphoric acid group, wherein t is selected from an integer from 1 to 20, e.g., from 1 to 10.

In one or more embodiments, L₃ is: 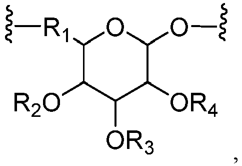 wherein in the formula, R₁ is selected from -O(CH₂)ₚ- and -C(=O)-, and p is 1, 2, or 3; R₂, R₃, and R₄ are all H.

In one or more embodiments, Y is -(CH₂)ₚ-, and p is 1, 2, or 3; preferably, Y is methylene.

In one or more embodiments, Z is C.

In one or more embodiments, L₄ is selected from the following amino acid residues: valine, citrulline, alanine, glycine, phenylalanine, asparagine, glutamic acid, lysine, serine, threonine, cysteine, and tyrosine; the amino acid groups are optionally substituted with one or more substituents selected from -OR₂₀, wherein R₂₀ is selected from H, C₁-C₆ alkyl, -(CH₂CH₂O)ₜ-C₁-C₆ alkyl, a sulfonic acid group, and a phosphoric acid group, and t is an integer from 1 to 20. Preferably, the amino acid residues are connected to the remainder of the compound by a peptide bond (-NH-CO-). Preferably, R₂₀ is selected from H, C₁-C₆ alkyl, -(CH₂CH₂O)ₜCH₃, a sulfonic acid group, and a phosphoric acid group, wherein t is selected from an integer from 1 to 10.

In one or more embodiments, L₄ is selected from the following peptide fragments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine, and lysine-phenylalanine, preferably from the following peptide fragments: valine-citrulline (VC), valine-alanine (VA), and glycine-glycine-phenylalanine-glycine (GGFG); each of the peptide fragments is optionally substituted with one or more substituents selected from -OR₂₀ and -(OCH₂CH₂)ₚO-C₁-C₆ alkyl, wherein R₂₀ is selected from H, C₁-C₆ alkyl, -(CH₂CH₂O)ₜ-C₁-C₆ alkyl, a sulfonic acid group, and a phosphoric acid group, t is an integer from 1 to 20, and p is an integer from 1 to 6. It should be understood that the peptide fragments are divalent groups, and that the two amino acid residues at their head and tail are connected to other parts of the compound by the -NH- moiety or the -CO- moiety in their peptide bonds. Preferably, R₂₀ is selected from H, C₁-C₆ alkyl, -(CH₂CH₂O)ₜCH₃, a sulfonic acid group, and a phosphoric acid group, wherein t is selected from an integer from 1 to 10. Preferably, the - (OCH₂CH₂)ₚO-C₁-C₆ alkyl is -(OCH₂CH₂)ₚO-C₁-C₃ alkyl, more preferably -(OCH₂CH₂)ₚOCH₃; preferably, p is 1, 2, or 3.

In one or more embodiments, L₄ is selected from:

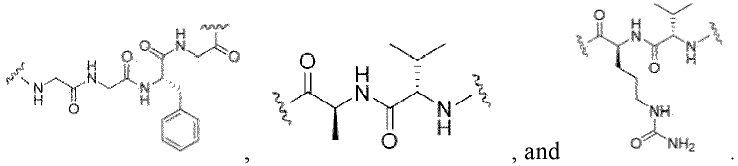

In one or more embodiments, m is 0.

In one or more embodiments, m is 1.

In one or more embodiments, R₆ is H, C₁-C₃ alkoxy, or -(OCH₂CH₂)ₚO-C₁-C₃ alkyl, wherein p is an integer from 1 to 6. In one or more embodiments, R₆ is H, methoxy, or -(OCH₂CH₂)ₚOCH₃, wherein p is an integer from 1 to 6 or from 1 to 4.

In one or more embodiments, R is selected from H and phenyl optionally substituted with 1-3 nitro groups and is preferably H or 4-nitrophenyl.

In one or more embodiments, the linker has a structure represented by any one of the following formulas II to XIV: 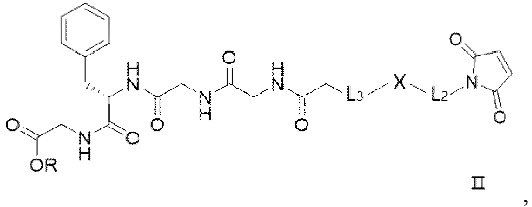 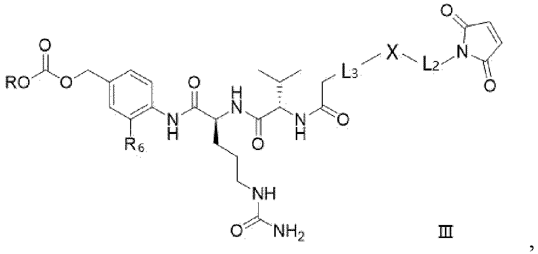 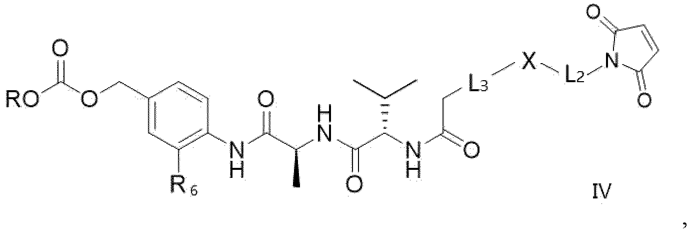 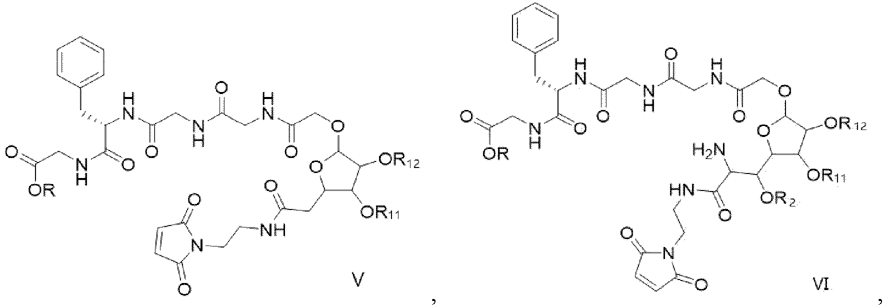 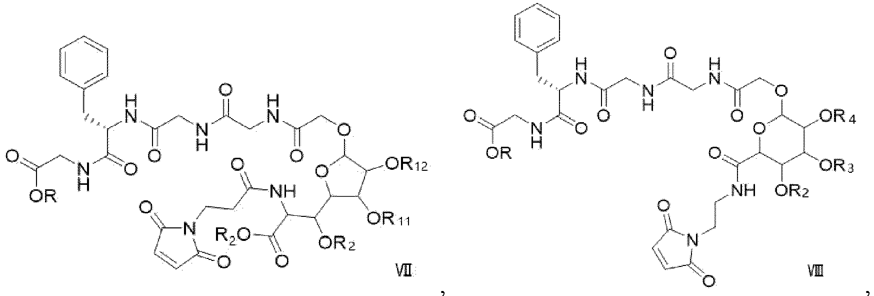 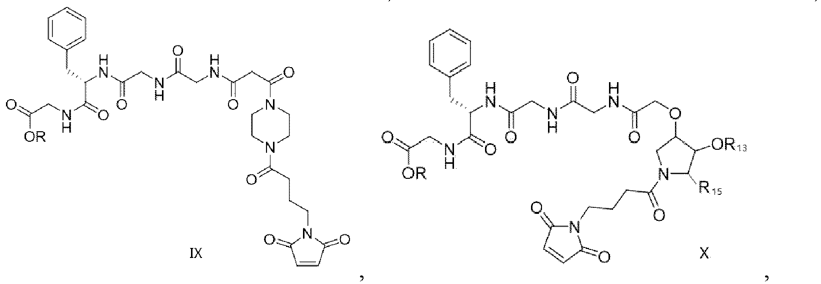 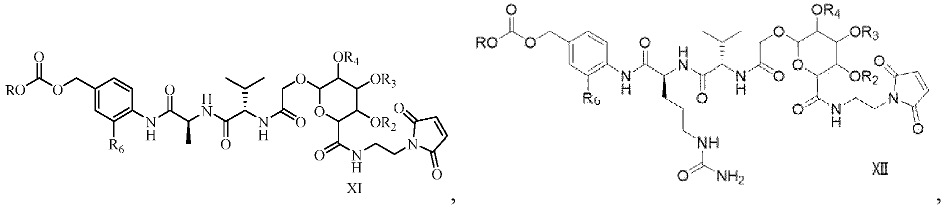 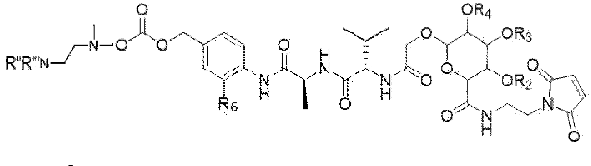 XIII, and 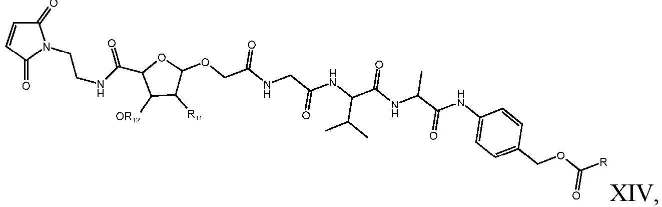 wherein in each of the formulas, R", R‴, R, L₂, X, L₃, R₂-R₄, R₁₁-R₁₃, and R₁₅ are as described in any one of the foregoing embodiments.

In one or more embodiments, the present application provides linkers represented by the following formulas 1-46:

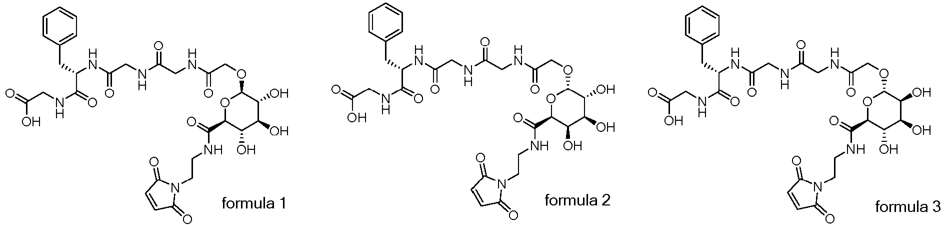

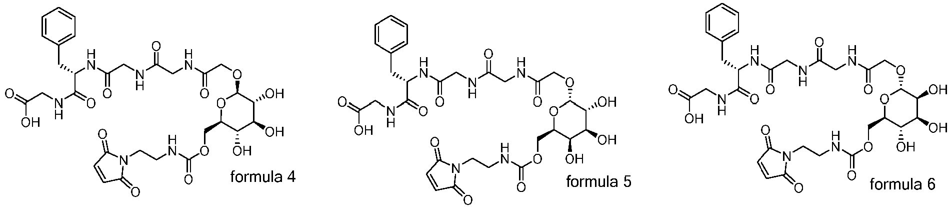

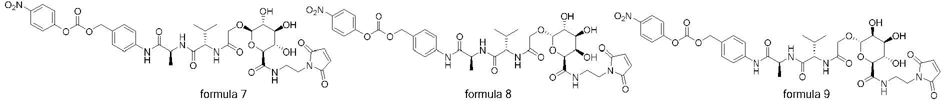

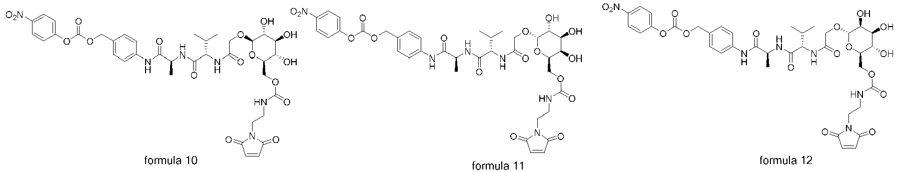

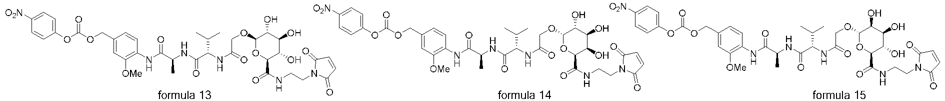

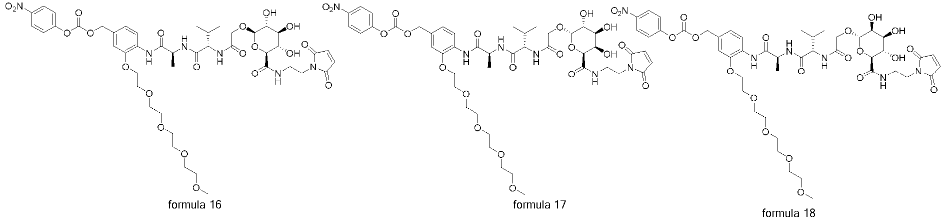

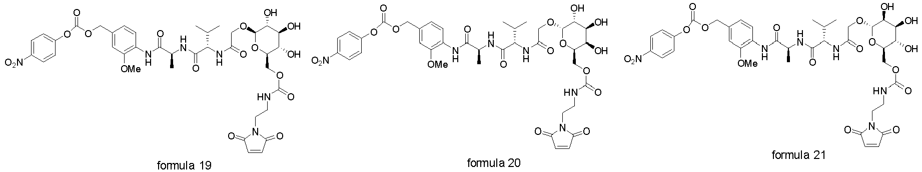

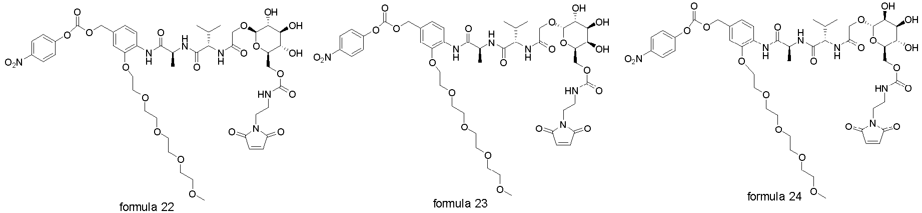

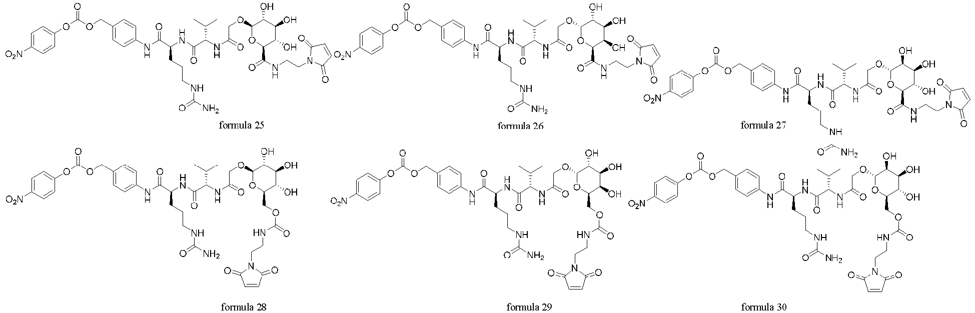

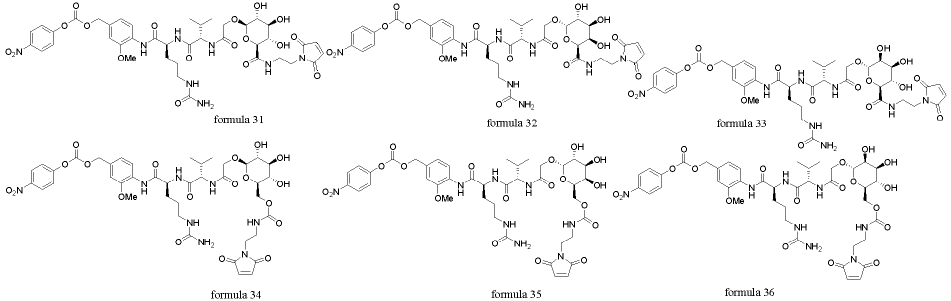

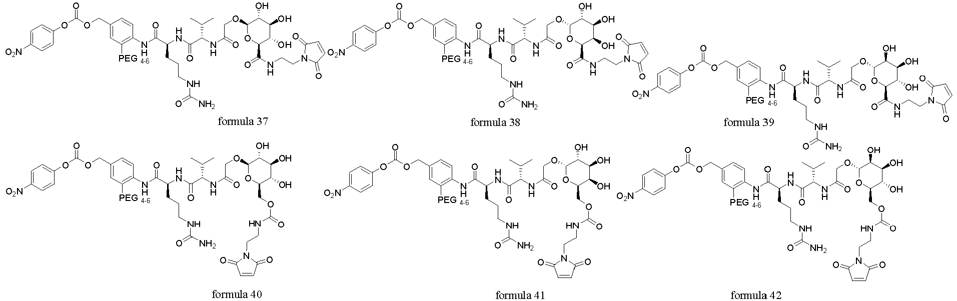

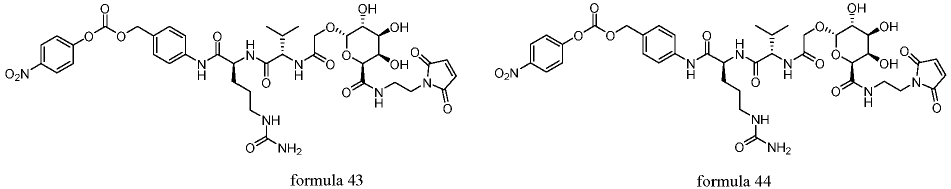

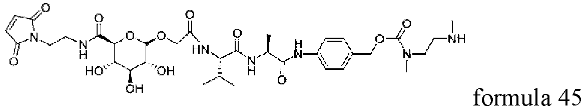

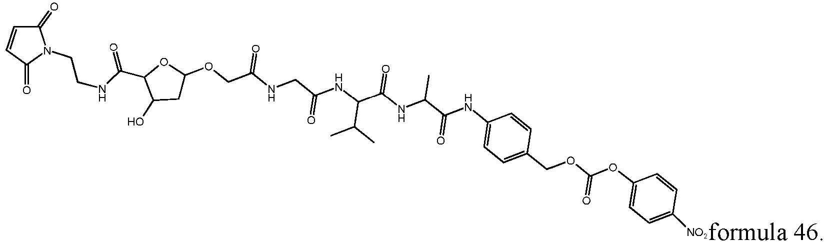

### Linker-Drug Conjugate

In a second aspect, the present application provides a linker-drug conjugate having a structural formula represented by formula XIII': 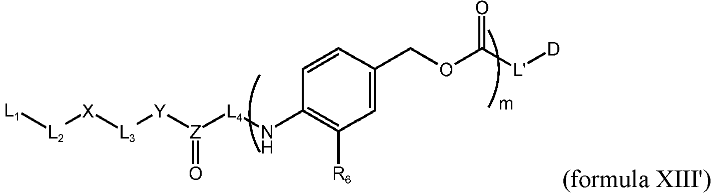 wherein in the formula, L₁, L₂, L₃, L₄, X, Y, Z, R₆, and m are as described in the first aspect; L' is a bond, -NR"-C₁-C₆ alkylene-NR"-CO-*, or -NH-CH₂-O-; D is a drug moiety; each R" is independently H and C₁-C₆ alkyl; * indicates a position where L' is connected to D.

In some embodiments, the linker-drug conjugate has a structure represented by formula XIII: 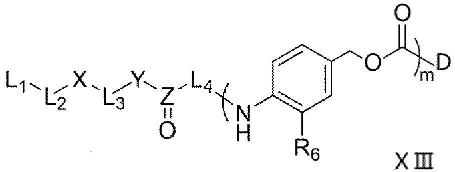 wherein L₁, L₂, L₃, L₄, X, Y, Z, R₆, and m are as described in the first aspect; D is a drug moiety.

Herein, the drugs include, but are not limited to, cytotoxic drugs, cell differentiation factors, stem cell trophic factors, steroid drugs, drugs for treating autoimmune diseases, anti-inflammatory drugs, and drugs for infectious diseases. Further, the drug moiety D includes, but is not limited to, tubulin inhibitors or DNA damaging agents. The tubulin inhibitors include, but are not limited to, dolastatins, auristatins, and maytansines; the DNA damaging agents include, but are not limited to, calicheamicins, duocarmycins, anthramycin derivative PBD (pyrrolobenzodiazepine), camptothecin derivative SN-38, and topoisomerase I inhibitors. The auristatin-based drugs include, but are not limited to, monomethyl auristatin E (MMAE), monomethyl auristatin F (MMAF), and auristatin F (AF), or derivatives thereof. The maytansine-based drugs include, but are not limited to, DM1, DM3, and DM4, or derivatives thereof ("Research Progress on Warhead Molecules of Antibody-Drug Conjugates", Hu Xinyue et al., Chinese Medicinal Biotechnology, December 2017, Vol. 12, No. 6; and "Research Progress on Maytansine-Based Antibody-Drug Conjugates", Zhou Lei et al., Chinese Journal of New Drugs, 2016, Vol. 25, No. 22, pp. 2521-2530). The topoisomerase I inhibitors include, but are not limited to, exatecan, topotecan, irinotecan, 9-nitrocamptothecin, and the macromolecular camptothecin conjugate PNU-166148 (compound A1 in WO99/17804). The drug moiety D may also be amanitins, anthracyclines, baccatins, camptothecins, cemadotins, colchicines, colcimids, combretastatins, cryptophycins, discodermolides, docetaxel, doxorubicin, echinomycins, eleutherobins, epothilones, estramustines, lexitropsins, maytansines, methotrexate, netropsins, puromycins, rhizoxins, taxanes, tubulysins, or vinca alkaloids. The drug moiety D may also be a vitamin A precursor, folic acid, etc. The drug moiety D is not limited to the above classes; it also includes all drugs available for ADCs.

It should be understood that the position where D is connected to the remainder of the linker-drug conjugate will generally not affect the biological activity of drug D itself. The active site of drug D can be readily determined by one skilled in the art based on the prior art, and the remainder of the linker-drug conjugate is covalently connected at the position of the active site. The modes of covalent connection are well known in the art, including via an amido group (-NRCO-, wherein R is H or C₁-C₄ alkyl), an ester bond (-COO-), -S-, etc.

In one or more embodiments, the drug moiety (D) has a structure represented by any one of the following formulas D-1 to D-9: 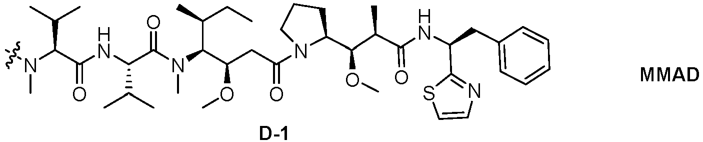 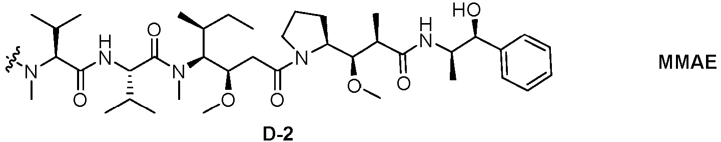 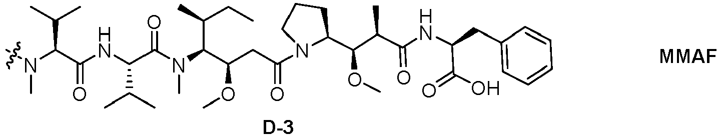 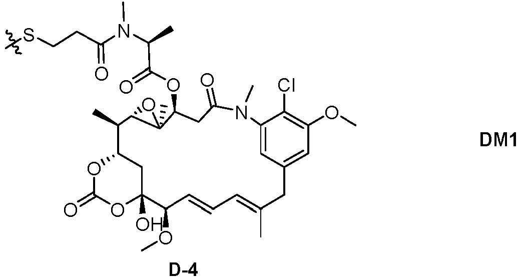 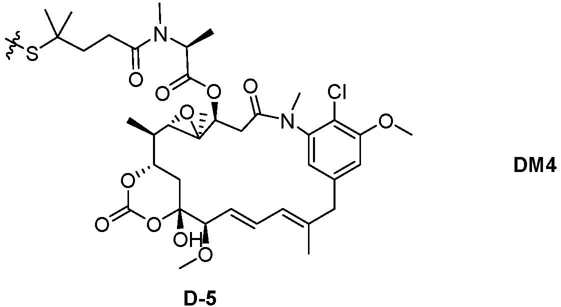 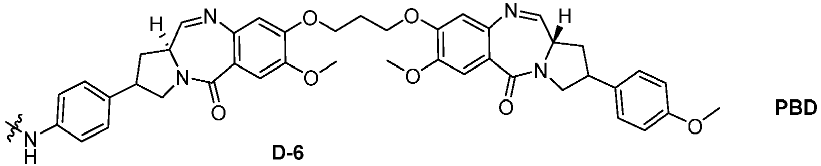 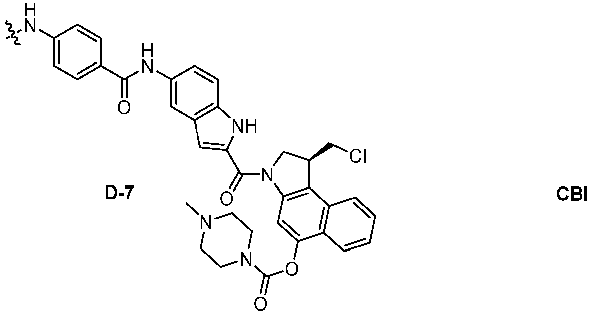 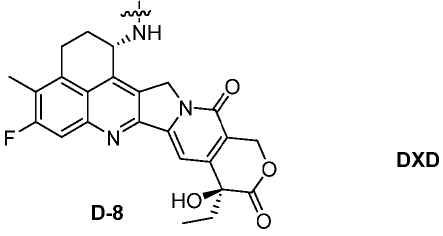 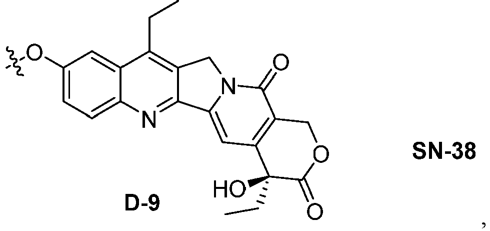 wherein in the formulas, the wavy line indicates a position where D is connected to the remainder of the linker-drug conjugate.

In one or more embodiments, the present application provides linker-drug conjugates numbered herein CPD1, CPD2, CPD3, CPD4, CPD5, CPD6, CPT004, CPT005, CPT006, CPT008, CPT009, CPT010, MMAE Derivate (MMAE derivative 1), MMAE Derivate (o-OMe-PAB) (MMAE derivative 2), Comp.5, Comp6, Comp7, Comp8, and Comp9.

### Antibody-Drug Conjugate

In a third aspect, the present application provides an antibody-drug conjugate having a structure represented by formula XV:

Ab-(L-D)ₛ (formula XV)

wherein L is the linker according to any one of the embodiments of the first aspect; D is the drug according to any one of the embodiments of the second aspect; s represents an average number of L-D units conjugated to Ab, and s ranges from 1 to 8; Ab is a targeting moiety. In some embodiments, s is 2-6.

In some embodiments, the antibody-drug conjugate of the present disclosure has a structure represented by the following formula: 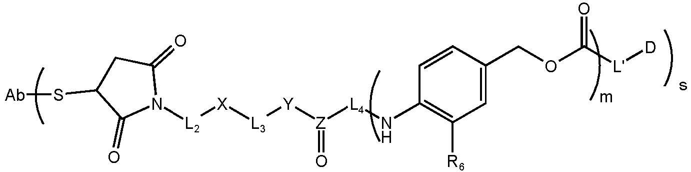 wherein L₂, X, L₃, Y, Z, L₄, R₆, L', and m are as described in any one of the embodiments herein; s represents an average number of L-D units conjugated to Ab, and s ranges from 1 to 8; Ab is a targeting moiety; S represents a sulfur atom in a cysteine residue in the antibody. In some embodiments, s is 2-6.

In one or more embodiments, Ab is any antibody or antibody functional fragment. Further, the antibody includes derivatives or analogs of the antibody; the "functional" fragment means that the fragment can recognize the same antigen and can recognize fragments, derivatives, or analogs derived from the antigen. The functional fragment includes, for example, but is not limited to: F(ab')2, Fab, Fab', Fv fragments, and heavy and light chain dimers of antibodies, or any of their smallest fragments such as Fvs or single-chain antibody (single-chain antibody fragment/single-chain variable fragment, scFv). In addition, the antibody may be a fusion protein of the antibody. The antibody may also include analogs and derivatives modified or unmodified (i.e., by covalent connection to any molecule), as long as such covalent connection allows the antibody to retain its antigen-binding immunospecificity, including, but not limited to: antibody analogs and derivatives, including those that have been further modified, for example: by glycosylation, acetylation, PEGylation, phosphorylation, amidation, derivatization with known protecting/blocking groups, protease cleavage, linking to cellular antibody units or other proteins, etc. Any of numerous chemical modifications can be achieved using known techniques including, but not limited to, specific chemical cleavage, acetylation, formylation, metabolic synthesis in the presence of tunicamycin, etc. In addition, the analogs or derivatives may comprise one or more unnatural amino acids. In some examples, the antibody may have a modification (e.g., substitution, deletion, or addition) in an amino acid residue that interacts with an Fc receptor; still further, the antibody is selected from a murine antibody, a mammal-derived antibody, a chimeric antibody, a humanized antibody, a human antibody, and a multispecific (e.g., bispecific) antibody. Still further, the antibody or antibody functional fragment specifically binds to a cell surface receptor or a tumor-associated antigen.

In some embodiments, the antibody is an anti-TROP2 antibody, such as hRS7 or datopotamab. The light chain sequence of an exemplary anti-TROP2 antibody is set forth in SEQ ID NO: 1, and its heavy chain is set forth in SEQ ID NO: 2; or its light chain sequence is set forth in SEQ ID NO: 3, and its heavy chain is set forth in SEQ ID NO: 4. In some embodiments, the antibody is anti-KLH hIgG1.

In some embodiments, the antibody is an anti-BCMA antibody. Preferably, the amino acid sequence of the heavy chain of the antibody is set forth in SEQ ID NO: 5, and the light chain amino acid sequence is set forth in SEQ ID NO: 6.

In one or more embodiments, the L-D is the linker-drug conjugate according to any one of the embodiments herein, especially the linker-drug conjugates numbered herein CPD1, CPD2, CPD3, CPD4, CPD5, CPD6, CPT004, CPT005, CPT006, CPT008, CPT009, CPT010, MMAE Derivate (MMAE derivative 1), MMAE Derivate (o-OMe-PAB) (MMAE derivative 2), Comp.5, Comp6, Comp7, Comp8, and Comp9.

In one or more embodiments, the antibody-drug conjugate (ADC) has the structure: 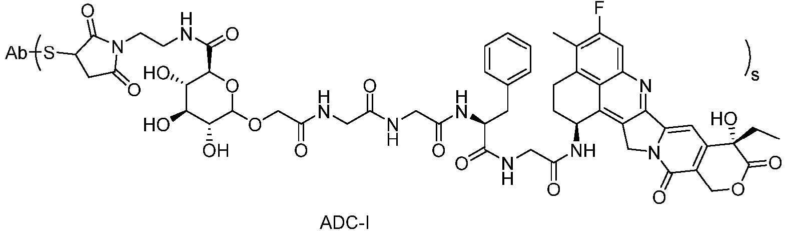 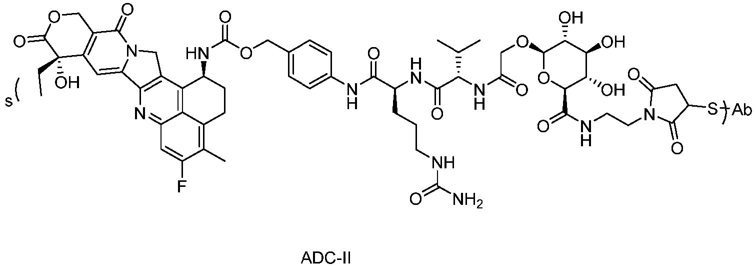 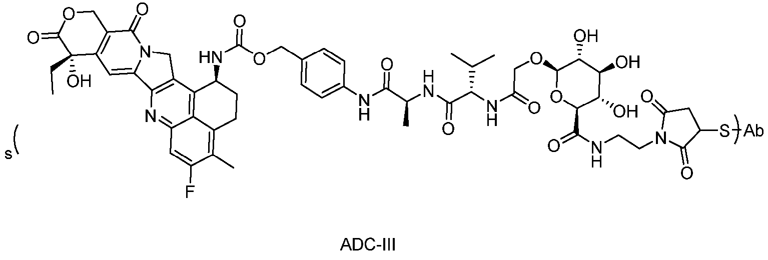 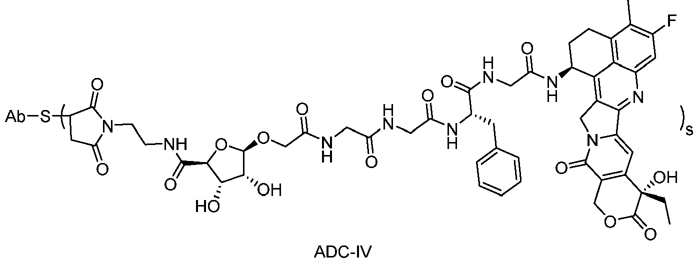 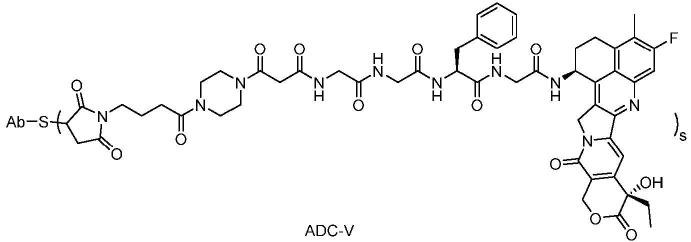 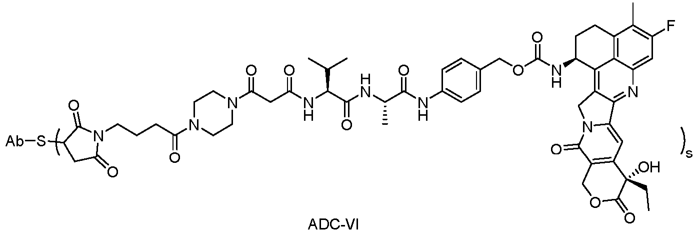 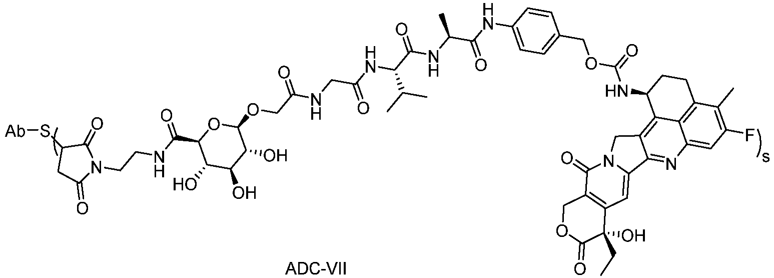 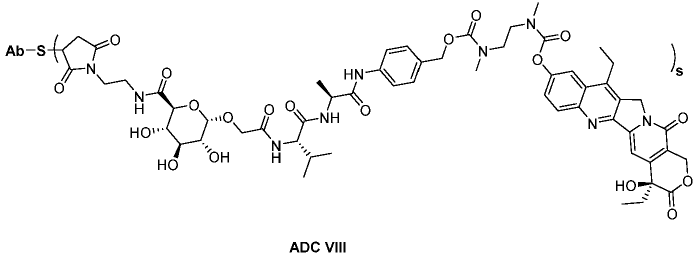 wherein in the formulas, s is 1-8.

### Connector

In a fourth aspect, the present application provides a connector for connecting a drug to a targeting moiety to form a targeting moiety-drug conjugate:

-L₁-L₂-X-L₃-Y-Z(=O)-L₄-(L₅)m-L₆-,

wherein in the formula, L₁ is selected from -(maleimido-3-yl-N)-, -C(=O)-, -(CH₂)ₙC(=O)-, - S(=O)-, -(CH₂)ₙS(=O)-, -CH₂-(CH₂)ₙ-, and -CH=CH-; L₂ and Y are each independently selected from -(CH₂)ₚ-, -(OCH_{2C}H₂)ₚ-, and -(CH₂CH₂O)ₚ-; L₃ is a hydrophilic group; L₄ is an amino acid group; L₅ is: 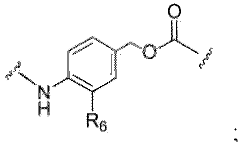 R₆ is selected from H, C₁-C₆ alkoxy, -(OCH₂CH₂)ₚO-C₁-C₆ alkyl, and -(CH₂CH₂O)ₚ-C₁-C₆ alkyl; L₆ is a bond, -NR"-C₁-C₆ alkylene-NR"-, or -NR"-C₁-C₆ alkylene-NR"-CO-; each R" is independently H or C₁-C₆ alkyl; preferably, each R" is independently H, methyl, or ethyl; more preferably, each R" is methyl; X is selected from -NH-, -NH(CH₂)ₙC(=O)-, -C(=O)(CH₂)ₙNH-, and -(CH₂)ₙC(=O)-; Z is selected from C and S; n is selected from 0, 1, and 2; p is selected from an integer from 1 to 10; m is selected from 0 and 1; wherein L₁ is used for connecting to the targeting moiety, and the other end of the connector is used for connecting to the drug; wherein when m is 0, the drug is connected to the carbonyl end (C(=O)) of L₄; when m is 1, the drug is connected to the carbonyl end (C(=O)) of L₅, and L₄ is connected to the amino end (NH) of L₅.

In one or more embodiments, L₁ is -(maleimido-3-yl-N)-, wherein the -(maleimido-3-yl-N)- is a structure represented by the following formula and is connected to the targeting moiety via locant 3,

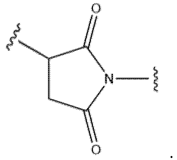

In one or more embodiments, L₂ is -(CH₂)ₚ-, and p is an integer from 1 to 4; preferably, L₂ is - CH₂CH₂- or -CH₂CH₂CH₂-.

In one or more embodiments, X is -NH- or -NH(CH₂)ₙC(=O)-; preferably, X is -NH- or -NHC(=O)-. In one or more embodiments, Y is -(CH₂)ₚ-, and p is 1, 2, or 3; preferably, Y is methylene.

In one or more embodiments, Z is C.

In one or more embodiments, L₃ is selected from divalent hydrophilic groups with two monovalent radical centers resulting from the removal of two hydrogen atoms from a monosaccharide, a disaccharide, and a five- or six-membered saturated heterocycle containing 1-2 nitrogen atoms and a derivative thereof; preferably, the monosaccharide is selected from at least one of a triose, a tetrose, a pentose, a hexose, and a heptose; the disaccharide is selected from at least one of maltose, sucrose, and lactose; the five- or six-membered saturated heterocycle containing 1-2 nitrogen atoms is selected from piperazinyl, piperidinyl, and pyrrolidinyl.

In one or more embodiments, L₃ is selected from divalent hydrophilic groups with two monovalent radical centers resulting from the removal of two hydrogen atoms from glucose, galactose, mannose, glucuronic acid, galactonic acid, mannuronic acid, N-acetylglucosamine, N-acetylgalactosamine, N-acetylmannosamine, and N-acetylmuramic acid.

In one or more embodiments, L₃ is selected from: 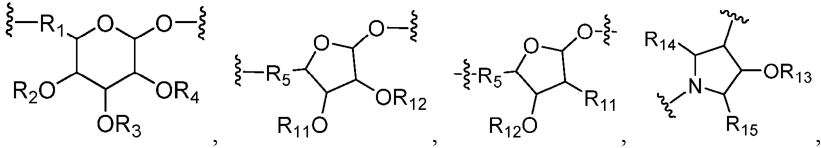 and 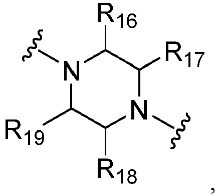 wherein in the formulas, R₁ is selected from -O(CH₂)ₚ- and -C(=O)-, and p is an integer from 1 to 6, preferably 1, 2, 3, or 4; R₅ is C₁-C₃ alkylene unsubstituted or substituted with one or more substituents selected from -OR₂₀ and -C(=O)OR₂₀-; R₂, R₃, R₄, R₁₁, R₁₂, and R₁₃ are independently selected from H, C₁-C₆ alkyl, -(CH₂CH₂O)ₜ-C₁-C₆ alkyl, a sulfonic acid group, and a phosphoric acid group; R₁₄ and R₁₅ are independently selected from H, -OR₂₀, and -CH₂R₂₀; R₁₆, R₁₇, R₁₈, and R₁₉ are independently selected from H and -OR₂₀; R₂₀ is selected from H, C₁-C₆ alkyl, -(CH₂CH₂O)ₜ-C₁-C₆ alkyl, a sulfonic acid group, and a phosphoric acid group; t is an integer from 1 to 20; the wavy lines indicate positions where L₃ is connected to X and Y.

In one or more embodiments, L₄ is selected from the following amino acid residues: valine, citrulline, alanine, glycine, phenylalanine, asparagine, glutamic acid, lysine, serine, threonine, cysteine, and tyrosine; the amino acid groups are optionally substituted with one or more substituents selected from -OR₂₀, wherein R₂₀ is selected from H, C₁-C₆ alkyl, -(CH₂CH₂O)ₜ-C₁-C₆ alkyl, a sulfonic acid group, and a phosphoric acid group, and t is an integer from 1 to 20.

In one or more embodiments, L₄ is selected from the following peptide fragments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine, and lysine-phenylalanine.

In one or more embodiments, L₄ is selected from the following peptide fragments: valine-citrulline (VC), valine-alanine (VA), and glycine-glycine-phenylalanine-glycine (GGFG); each of the peptide fragments is optionally substituted with one or more substituents selected from -OR₂₀ and - (OCH₂CH₂)ₚO-C₁-C₆ alkyl, wherein R₂₀ is selected from H, C₁-C₆ alkyl, -(CH₂CH₂O)ₜ-C₁-C₆ alkyl, a sulfonic acid group, and a phosphoric acid group, t is an integer from 1 to 20, and p is an integer from 1 to 6.

In one or more embodiments, L₄ is selected from:

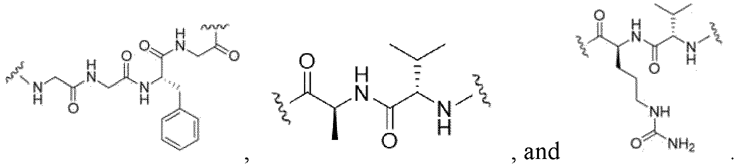

In one or more embodiments, R₆ is H, C₁-C₃ alkoxy, or -(OCH₂CH₂)ₚO-C₁-C₃ alkyl, wherein p is an integer from 1 to 6.

In one or more embodiments, R₆ is H, methoxy, or -(OCH₂CH₂)ₚOCH₃, wherein p is an integer from 1 to 6 or from 1 to 4.

In one or more embodiments, the connector is selected from connectors having structures represented by the following formulas II-1 to XIV-1: 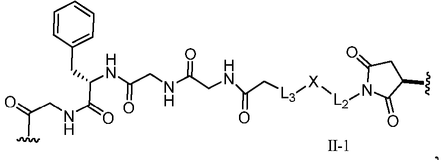 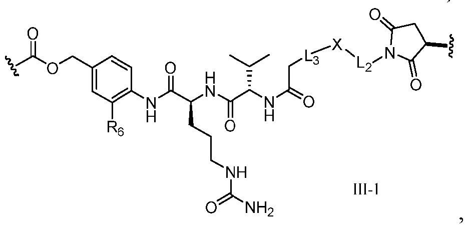 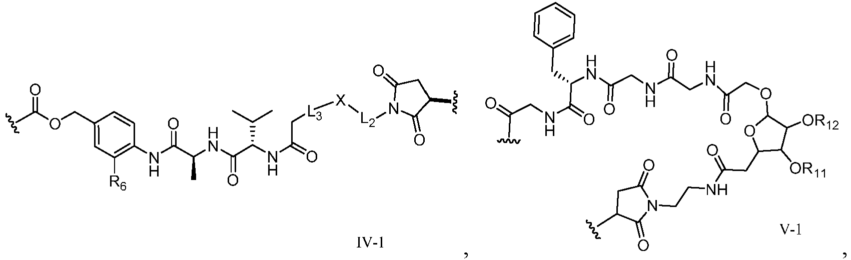 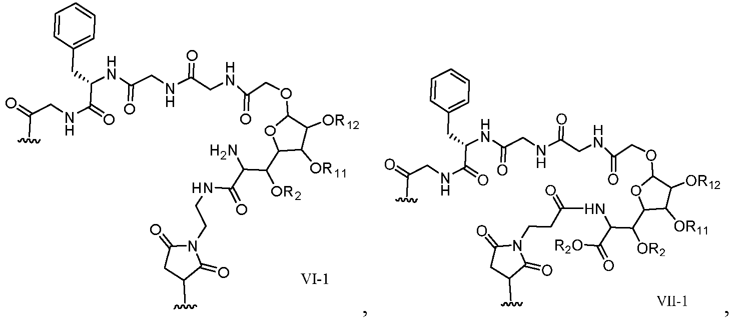 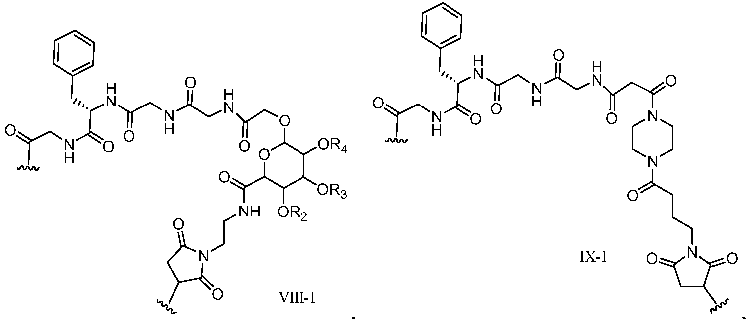 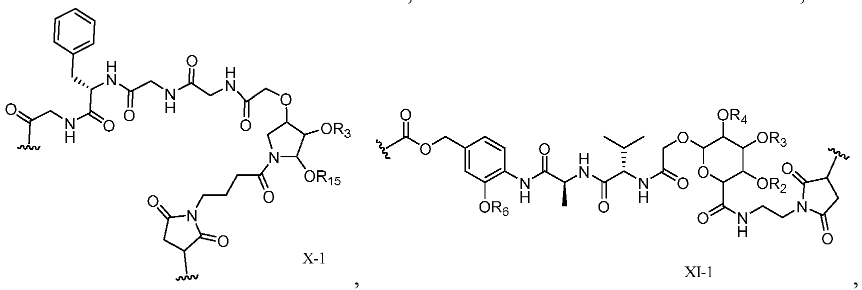 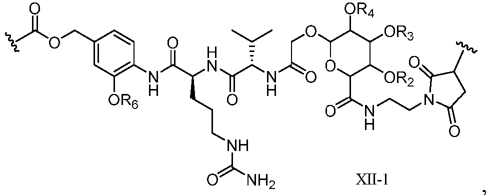 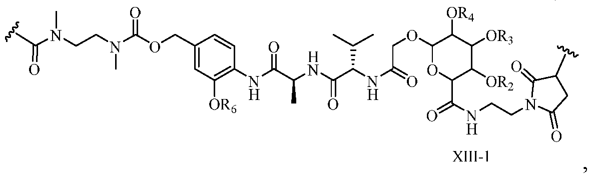 and 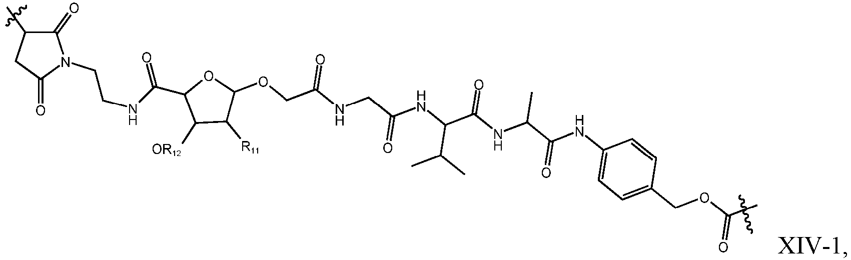 wherein in each of the formulas, R, L₂, X, L₃, R₂-R₄, R₁₁-R₁₃, and R₁₅ are as described in any one of the embodiments herein.

In one or more embodiments, the connector is selected from connectors having structures represented by the following formulas 1-1 to 46-1:

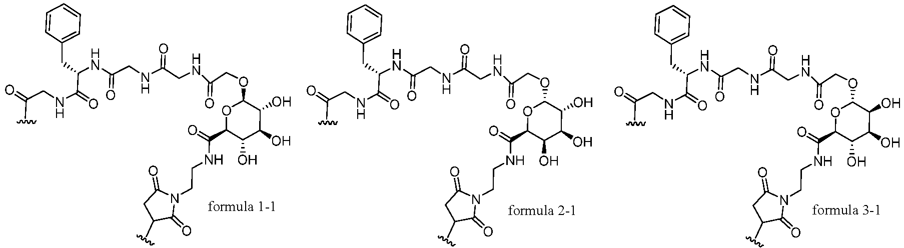

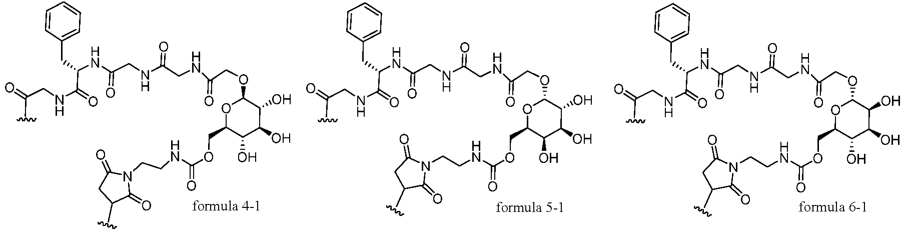

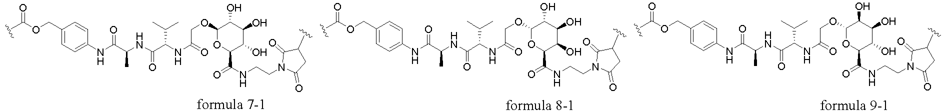

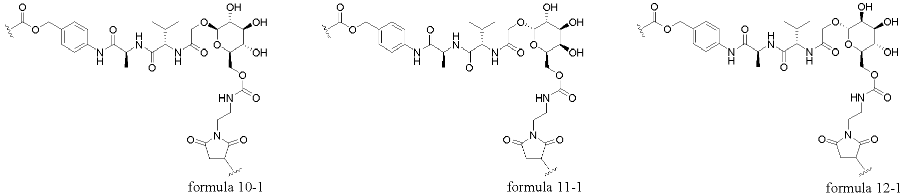

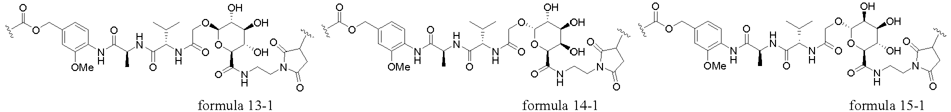

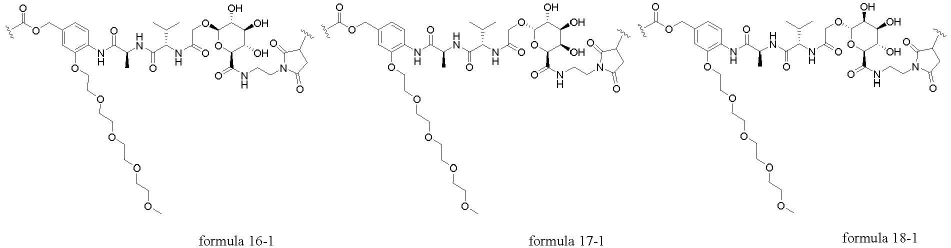

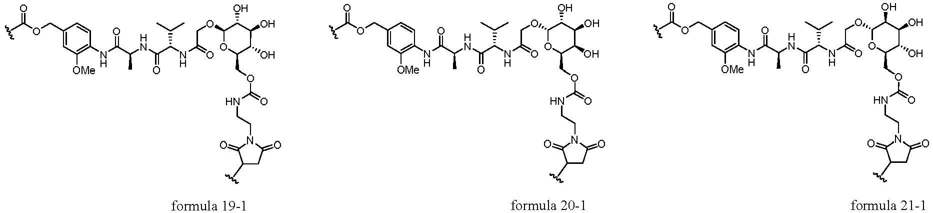

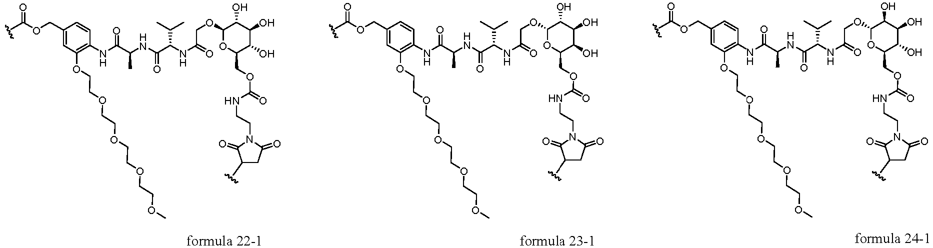

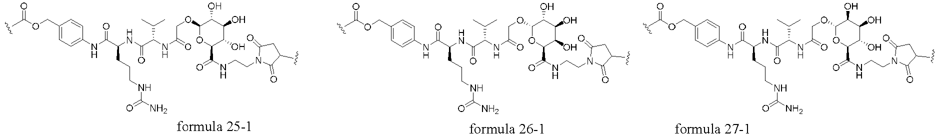

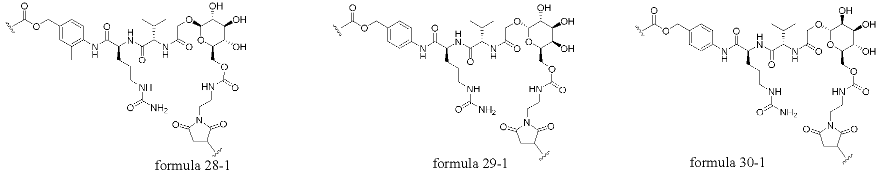

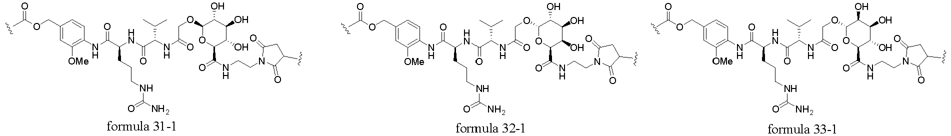

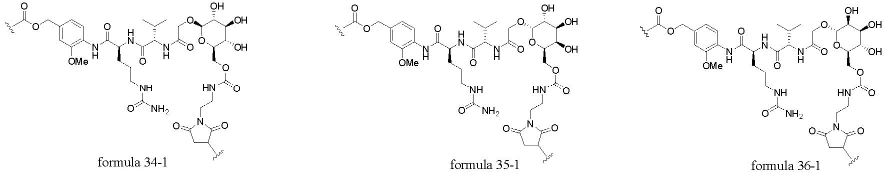

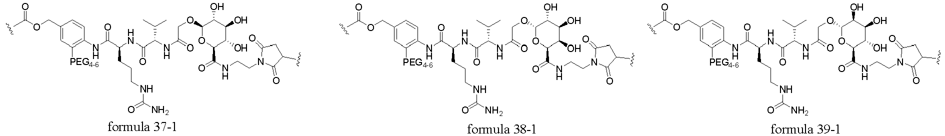

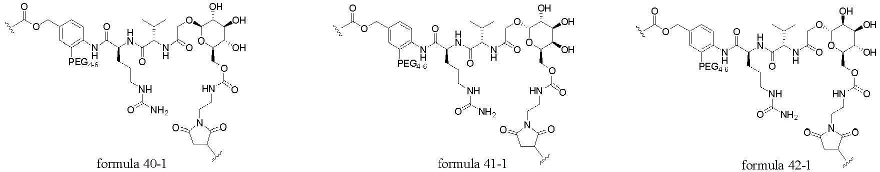

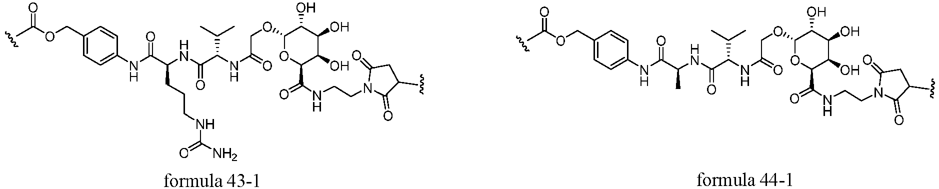

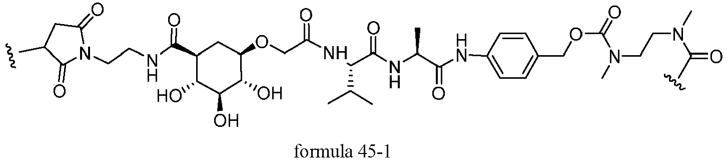

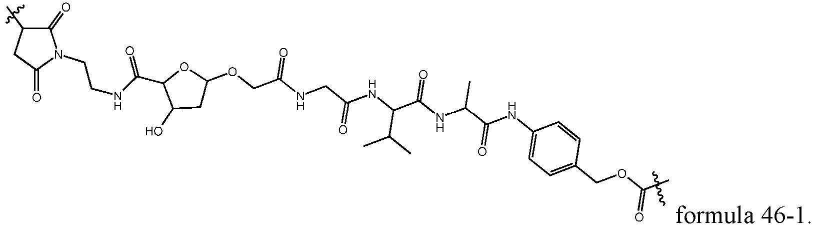

In some embodiments, the targeting moiety is an antibody, such as the various antibodies (Abs) described in the "Antibody-Drug Conjugate" section of the present application. In some embodiments, the drug includes various drug molecules well known in the art, including the various drugs (D) described in the "Linker-Drug Conjugate" section of the present application.

In some embodiments, the present disclosure provides an antibody-drug conjugate comprising the connector according to any one of the embodiments herein. In some embodiments, the present disclosure further provides use of the connector structure according to any one of the embodiments herein, particularly for increasing the drug load of an antibody-drug conjugate and avoiding efficacy reductions caused by hydrophobic aggregation due to local intensive distribution of drugs.

### Pharmaceutical Composition

In a fifth aspect, the present application provides a pharmaceutical composition comprising the antibody-drug conjugate or the pharmaceutically acceptable salt thereof according to any one of the embodiments herein and a pharmaceutically acceptable carrier or auxiliary material.

Herein, the pharmaceutically acceptable carrier or auxiliary material may be any pharmaceutically acceptable carrier or excipient and may vary depending on the dosage form and the mode of administration. Pharmaceutically acceptable carriers are generally safe and non-toxic and may comprise any of the substances known in the pharmaceutical industry for use in formulating pharmaceutical compositions, including fillers, diluents, coagulants, binders, lubricants, glidants, stabilizers, colorants, wetting agents, disintegrants, etc. Suitable pharmaceutically acceptable carriers include sugars such as lactose or sucrose, and mannitol or sorbitol; cellulose formulations and/or calcium phosphates, such as tricalcium phosphate or dicalcium phosphate; starch; silica, talc, and stearic acid or salts thereof, such as magnesium stearate or calcium stearate; and/or polyethylene glycol, etc. In selecting a pharmaceutically acceptable carrier, the mode of administration of the pharmaceutical dosage form is a primary consideration.

The pharmaceutical composition may comprise a therapeutically or prophylactically effective amount of the antibody-drug conjugate. "Effective amount" means that the amount of the ingredient is sufficient to effect the desired response. The specific effective amount will depend upon various factors, such as a particular disease to be treated, the physical conditions of a patient, e.g., weight, age, and sex, the duration of the treatment, the co-administered treatment (if any), and the specific formulation composition used.

The pharmaceutical composition of the present disclosure may be formulated into various suitable dosage forms including, but not limited to, tablets, capsules, injections, etc. and may be administered by any suitable route to achieve the intended purpose. For example, it may be administered parenterally, subcutaneously, intravenously, intramuscularly, intraperitoneally, transdermally, orally, intrathecally, intracranially, intranasally, or topically. The dosage of the drug may depend on the age, health, and weight of the patient, the concurrent treatment, the frequency of treatment, etc. The pharmaceutical composition of the present disclosure may be administered to any subject in need thereof, such as a mammal, especially a human.

### Use and Method

In a sixth aspect, the present application provides use of the linker according to any one of the embodiments of the first aspect herein in the preparation of a linker-drug conjugate or an ADC. In some embodiments, the linker-drug conjugate is the linker-drug conjugate according to any one of the embodiments of the second aspect herein. In some embodiments, the ADC is the ADC according to any one of the embodiments of the third aspect herein.

In a seventh aspect, the present application provides use of the antibody-drug conjugate according to any one of the embodiments of the third aspect herein or the pharmaceutical composition according to any one of the embodiments of the fourth aspect herein in the preparation of a medicament, or the antibody-drug conjugate according to any one of the embodiments of the third aspect herein or the pharmaceutical composition according to any one of the embodiments of the fourth aspect herein for use in the treatment or prevention of a disease. The disease that the medicament can treat or prevent depends on the drug moiety contained in the antibody-drug conjugate. In some embodiments, the drug moiety contained in the antibody-drug conjugate is suitable for treating or preventing a cancer, an autoimmune disease, an inflammatory disease, or an infectious disease; therefore, the disease that the antibody-drug conjugate can be used to treat or prevent includes a cancer, an autoimmune disease, an inflammatory disease, or an infectious disease.

In an eighth aspect, the present application provides a method for treating or preventing a disease, the method comprising a step of administering to a subject in need thereof a therapeutically effective amount or a prophylactically effective amount of the antibody-drug conjugate according to any one of the embodiments of the third aspect of the present application or a pharmaceutical composition thereof. The antibody-drug conjugate of the present disclosure or the pharmaceutical composition thereof may be administered in any suitable manner, including parenteral, subcutaneous, intravenous, intramuscular, intraperitoneal, transdermal, oral, intrathecal, intracranial, intranasal, or topical administration. The dosage of administration may depend on the age, health, and weight of the patient, the concurrent treatment, the frequency of treatment, etc. The disease includes, but is not limited to, diseases that can be treated or prevented by the drug in the antibody-drug conjugate, including cancers, autoimmune diseases, inflammatory diseases, infectious diseases, etc.

### Abbreviations

The following abbreviations are used throughout the specification and examples of the present disclosure:
ADC: antibody-drug conjugate;
PBS solution: phosphate-buffered saline;
EDTA: ethylenediamine tetraacetic acid;
TCEP: tris(2-carboxyethyl)phosphine;
DMSO: dimethyl sulfoxide;
L-His solution: L-histidine solution;
DMF: N,N-dimethylformamide;
DIC: N,N'-diisopropylcarbodiimide;
HOBt: 1-hydroxybenzotriazole;
DIEA: N,N-diisopropylethylamine;
DCM: dichloromethane;
PE: petroleum ether;
4A MS: 4A molecular sieve;
TMSOTf: trimethylsilyl trifluoromethanesulfonate;
THF: tetrahydrofuran;
TBTU: O-benzotriazole-N,N,N',N'-tetramethyluronium tetrafluoroborate.

The present disclosure is further illustrated by the following examples, which should not be construed as limiting the present disclosure. The contents of all references cited throughout the present application are explicitly incorporated herein by reference.

### Examples

### Example 1: Synthesis of Drugs and Linkers

### Synthesis of CPD1 (its structure is formula D)

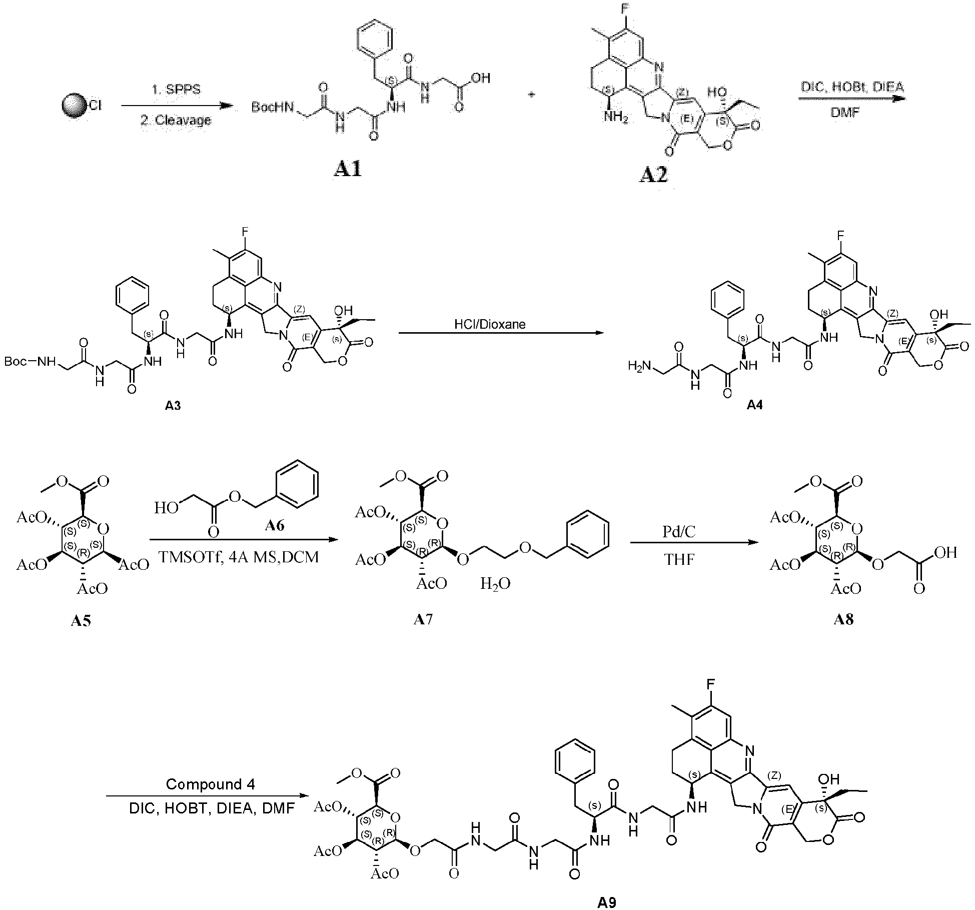

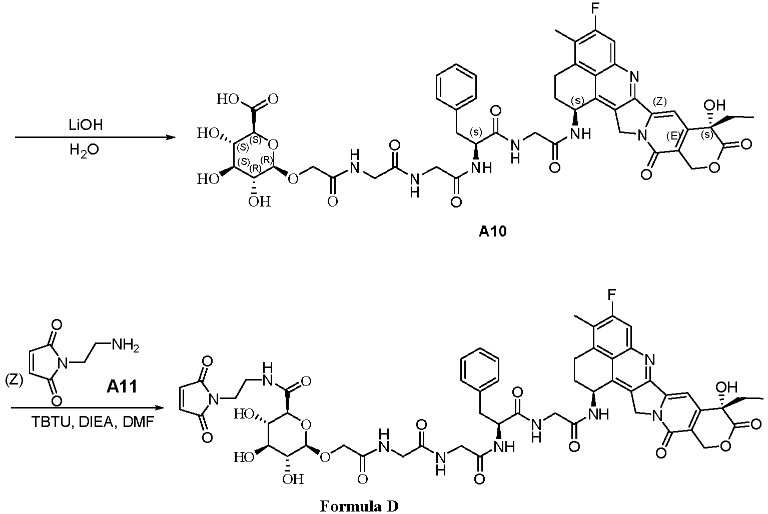

### Synthesis of compound A1, synthesis of a tetrapeptide by solid-phase synthesis

The GGFG tetrapeptide was synthesized using the standard Fmoc mode.

### Synthesis of compound A3

Compound A1 (10.0 g, 22.9 mmol) and commercially available compound A2 (10.9 g, 20.6 mmol) were dissolved in DMF (120 mL), and DIC (7.1 mL, 45.8 mmol), HOBt (3.41 g, 25.2 mmol), and DIEA (7.98 mL, 45.8 mmol) were sequentially added to the resulting solution. The mixture was well mixed by stirring and left to react at 25 °C for three hours. After the reaction finished, precipitation was performed with MTBE. The mixture was filtered to remove the precipitate, and the filtrate was concentrated to dryness by rotary evaporation to give compound A3. Compound A3 was directly used in the synthesis of compound A4 without purification. Compound A3 (21.6 g, 22.7 mmol, 90% purity) was a brown solid; MS: 854.4.

### Synthesis of compound A4

Compound A3 (21.6 g, 22.7 mmol, 90% purity) was dissolved in HCl/dioxane (200 mL) and left to react at 25 °C for 0.5 h. After the reaction finished, the mixture was distilled under reduced pressure to remove the solvent, and the crude product was then dissolved in DCM and PE. The solution was concentrated to dryness under reduced pressure to give compound A4, and compound A4 was directly used in the synthesis of compound A9 without purification. Compound A4 was a yellow solid (17.3 g, 22.9 mmol, 90.7% yield); MS: 754.5.

### Synthesis of compound A7

Commercially available compound A5 (50 g, 132 mmol), compound A6 (33.1 g, 199 mmol, 28.3 mL), 4A MS (50 g), and TMSOTf (26.58 g, 119.5 mmol, 21.6 mL) were dissolved in anhydrous DCM, and the solution was purged with nitrogen three times and left to react at 25 °C for 3 h under a nitrogen atmosphere. After the reaction finished, the mixture was filtered to remove the 4A MS (50 g), and the filtrate was diluted with DCM (500 mL) and washed with saturated sodium bicarbonate (1.50 L). The washing was extracted with DCM (1.50 L × 3). The extract was washed with brine, dried over anhydrous sodium sulfate, and concentrated under reduced pressure. The concentrate was purified by preparative HPLC [Phenomenex Luna C18 (250 × 70 mm, 10 µm), mobile phase [water (TFA)-ACN], B%: 36%-66%, 25 min] to give compound A7 (24.7 g, 51.2 mmol, 38.5% yield) as a white solid.

**¹H NMR:** δ ppm 7.36 (br s, 5 H), 5.22 - 5.32 (m, 2 H), 5.18 (s, 2 H), 5.07 (br t, *J =* 7.82 Hz, 1 H), 4.74 (br d, *J* = 7.25 Hz, 1 H), 4.35 (s, 2 H), 4.04 (br d, *J* = 9.13 Hz, 1 H), 3.74 (s, 3 H), 2.03 (br d, *J* = 4.00 Hz, 9 H).

### Synthesis of compound A8

A 500-mL round-bottom flask was purged with Ar, and Pd/C (1.50 g, 31.9 mmol) was added to the Ar-purged round-bottom flask. Subsequently, THF (10.0 mL) was added to completely soak the Pd/C. Compound A7 (15.0 g, 31.9 mol) was dissolved in THF (150 mL), and the solution was slowly added to the round-bottom flask under an Ar atmosphere. The reaction mixture was purged with H₂ three times and left to react at 25 °C for two hours under a hydrogen atmosphere. After the reaction finished, filtration was performed under a N₂ atmosphere, and the filtrate was concentrated to give compound A8 as a crude product. Compound A8 was used in the synthesis of compound A9 without purification. Compound A8 was a white solid.

**¹H NMR:** δ ppm 8.30 - 9.05 (m, 1 H), 5.21 - 5.34 (m, 2 H), 5.03 - 5.12 (m, 1 H), 4.74 (d, *J* = 7.38 Hz, 1 H), 4.38 (d, *J* = 1.25 Hz, 2 H), 4.10 (d, *J* = 9.51 Hz, 1 H), 3.73 - 3.78 (m, 3 H), 2.01 - 2.09 (m, 9 H).

### Synthesis of compound A9

Compound A8 (3.00 g, 7.65 mmol) and compound A4 (5.76 g, 7.65 mmol) were dissolved in DMF (30.0 mL), and DIC (2.37 mL, 15.3 mmol), HOBt (1.14 g, 8.41 mmol), and DIEA (2.66 mL, 15.3 mmol) were sequentially added to the solution. The resulting mixture was left to react at 25 °C for 3 h. After the reaction finished, the pH was adjusted to 5 using acetic acid, and purification was performed by preparative HPLC [preparative column: YMC Triart C18 250 × 50 mm × 7 µm, mobile phase: [water (TFA)-ACN]; B%: 21%-55%, 30 min]. The resulting solution was lyophilized to give compound A9. Compound A9 was a yellow solid; MS: 1129.1.

### Synthesis of compound A10

An aqueous solution (30.0 mL) of lithium hydroxide (744 mg, 17.7 mmol) was added to a solution of compound A9 (2.00 g, 1.77 mmol), and the mixture was left to react at 0 °C for 0.5 h. After the reaction finished, the pH was adjusted to 5 using acetic acid, and purification was performed by preparative HPLC [preparative column: Phenomenex Luna C18 (250 × 70 mm, 10 µm), mobile phase: [water (TFA)-ACN]; B%: 17%-47%, 20 min]. The resulting solution was lyophilized to give compound A10. Compound A10 was a white solid; MS: 988.4.

### Synthesis of CPD1

Compound A10 (1.18 g, 1.19 mmol) was dissolved in DMF (10.0 mL), and commercially available compound A11 (184 mg, 1.31 mmol), TBTU (767 mg, 2.39 mmol), and DIEA (416 µL, 2.39 mol) were sequentially added to the solution. The reaction mixture was left to react at 25 °C for 1 h. After the reaction finished, the reaction mixture was filtered to remove insoluble substances and purified by preparative HPLC [preparative column: YMC Triart C18 250 × 50 mm × 7 µm, mobile phase: [water (TFA)-ACN]; B%: 13%-39%, 33 min]. The resulting solution was lyophilized to give CPD (820 mg, 738 µmol, 62.0% yield). CPD1 was a yellow solid; MS: 1110.2. The purity was 100%.

¹H NMR (400 MHz, DMSO) δ 8.43 (d, *J* = 8.4 Hz, 1H), 8.31 (d, *J* = 5.5 Hz, 1H), 8.15 - 8.03 (m, 4H), 7.80 (d, *J* = 10.9 Hz, 1H), 7.32 (s, 1H), 7.27 - 7.14 (m, 5H), 6.97 (s, 2H), 5.61 - 5.53 (m, 1H), 5.40 (t, *J* = 11.0 Hz, 2H), 5.22 (d, *J* = 21.6 Hz, 2H), 4.49 - 4.40 (m, 1H), 4.27 (t, *J* = 11.8 Hz, 2H), 4.02 (d, *J* = 15.4 Hz, 1H), 3.83 (dd, *J* = 16.5, 6.0 Hz, 1H), 3.72 (dd, *J* = 21.1, 6.2 Hz, 4H), 3.61 (d, *J* = 5.6 Hz, 1H), 3.54 (dd, *J* = 16.2, 7.7 Hz, 3H), 3.48 (d, *J* = 6.0 Hz, 3H), 3.25 (s, 3H), 3.18 (d, *J* = 9.0 Hz, 4H), 3.12 - 3.07 (m, 1H), 2.99 (dd, *J* = 14.0, 4.5 Hz, 1H), 2.82 - 2.72 (m, 1H), 2.41 (s, 3H), 2.16 (d, *J* = 31.0 Hz, 2H), 1.92 - 1.77 (m, 2H), 0.87 (t, *J* = 7.2 Hz, 3H).

### Synthesis of CPD2

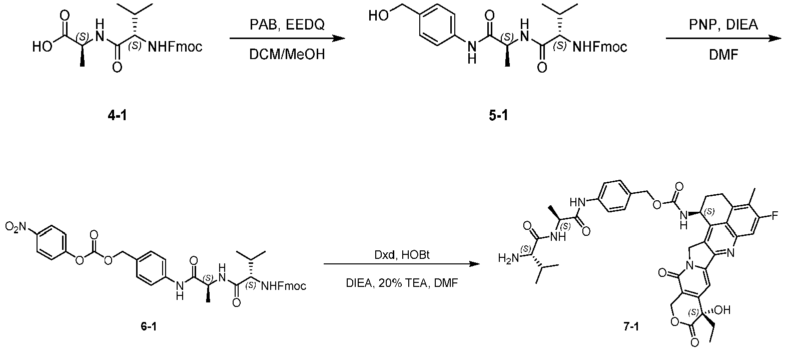

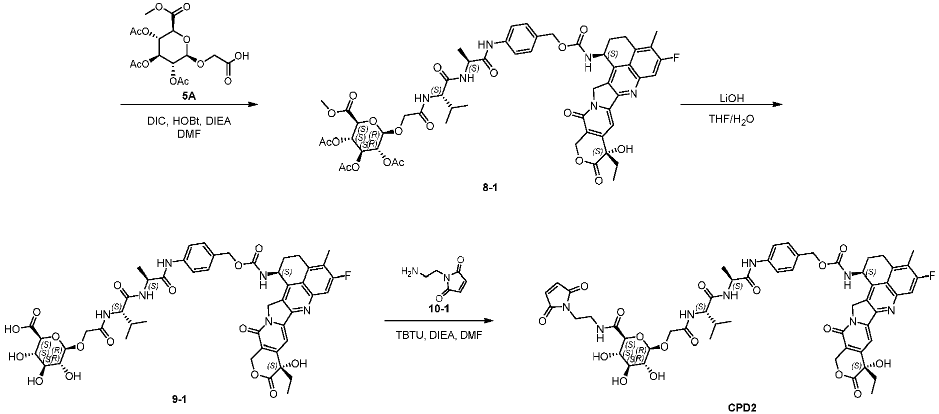

### General steps for preparing compound 5-1

Compound 4-1 (10.0 g, 24.4 mmol) and p-aminobenzyl alcohol (6.00 g, 48.7 mmol) were dissolved in a mixed solution of methyl acetate (90 mL) and methanol (90 mL), and EEDQ (12.1 g, 48.7 mmol) was then added under protection from light. The mixture was stirred at 45 °C for 1.5 h. LC-MS analysis showed that reactant 4-1 had been completely consumed, and one main peak was detected as a product peak. The reaction mixture was distilled under reduced pressure to remove the solvent, and solidification and crystallization were performed with MTBE:ethyl acetate (10:1). After filtration and centrifugal drying, a crude product was obtained. The crude product was directly used in the next step without further purification. The product was a gray solid (11.7 g, 99.3% yield).

MS (ES+) 516.3 [M+H]+.

### General steps for preparing compound 6-1

Compound 5-1 (11.7 g, 22.7 mmol) and PNP (13.8 g, 45.5 mmol) were dissolved in DMF (117 mL), and DIEA (7.92 mL, 45.5 mmol) was added to the mixture. The mixture was stirred at 25 °C for 2 h. LC-MS analysis showed that reactant 5-1 had been completely consumed, and one main peak was detected as a product peak. 1500 mL of ice-cold isopropyl ether was used to cause precipitation in the reaction mixture, and the procedure was repeated twice. The mixture was filtered, and the precipitate was centrifugally dried to give a crude product. The crude product was directly used in the next step without further purification. The product was a brown solid (8.10 g, 52.3% yield).

MS (ES+) 681.4 [M+H]+.

### General steps for preparing compound 7-1

Compound 6-1 (8.10 g, 11.9 mmol) and exatecan (5.06 g, 9.52 mmol) were dissolved in DMF (49 mL), and DIEA (4.15 mL, 23.8 mmol) and HOBt (1.93 g, 14.3 mmol) were then added to the solution. The mixture was stirred at 25 °C for 16 h. LC-MS analysis showed that the reactant had been completely consumed, and one main product peak was detected. Then 20% TEA was added to the reaction mixture, and the mixture was stirred for 5.5 h. TFA was added at 0 °C to acidify the reaction mixture. The mixture was purified by preparative high performance liquid chromatography (TFA conditions). Column: UniSil 10-120 C18 50 × 250 mm; mobile phase: [water (TFA)-acetonitrile]; B%: 20%-50%, 20 min. Compound 7-1 (5.40 g, 60.1% yield) was obtained as a yellow solid.

MS (ES+) 755.3 [M+H]+.

### General steps for preparing compound 8-1

Compound 7-1 (2.70 g, 3.58 mmol) and compound 5A (2.11 g, 5.37 mmol) were dissolved in DMF (27 mL), and HOBt (532 mg, 3.93 mmol), DIC (1.11 mL, 7.15 mmol), and DIEA (1.25 mL, 7.15 mmol) were then added to the solution. The mixture was stirred at 25 °C for 2 h. LC-MS analysis showed that reactant 7-1 had been completely consumed, and one main product peak was detected. CH3COOH was added at 0 °C to acidify the reaction mixture, and then precipitation was performed twice with 1000 mL of ice-cold isopropyl ether. After filtration and centrifugal drying, a crude product was obtained. The crude product was directly used in the next step without further purification. The product was 8-1 as an off-white solid (3.97 g, 98.3% yield).

MS (ES+) 1129.4 [M+H]+.

### General steps for preparing compound 9-1

Compound 8-1 (3.97 g, 3.52 mmol) was dissolved in a mixed solvent of H2O (2 mL) and THF (22 mL), and a solution of LiOH·H2O (1.48 g, 35.2 mmol) in H2O (5 mL) was then added to the solution. The mixture was stirred at 0 °C for 5 min. LC-MS analysis showed that reactant 8-1 had been completely consumed, and one main product peak was detected. CH3COOH was added at 0 °C to adjust the pH of the reaction mixture to 3-4. The reaction mixture was purified by preparative high performance liquid chromatography (TFA conditions). Column: Phenomenex Luna C18 (250 × 70 mm, 10 µm); mobile phase: [water (TFA)-acetonitrile]; B%: 15%-45%, 20 min. Compound 9-1 (1.82 g, 52.4% yield) was obtained as a yellow solid.

MS (ES+) 989.2 [M+H]+.

### General steps for preparing CPD2

Compound 9-1 (1.82 g, 1.84 mmol) and compound 10-1 (387 mg, 2.76 mmol) were dissolved in DMF (18 mL), and TBTU (1.18 g, 3.68 mmol) and DIEA (1.28 mL, 7.36 mmol) were then added. The mixture was stirred at 25 °C for 1 h. LC-MS analysis showed that the reactant had been completely consumed, and one main product peak was detected. The reactant was purified by preparative high performance liquid chromatography (TFA conditions). Column: Phenomenex Luna C18 (250 × 70 mm, 10 µm); mobile phase: [water (TFA)-acetonitrile]; B%: 26%-56%, 23 min. Compound CPD2 (1.15 g, 56.0% yield) was obtained as a yellow solid. MS (ES+) 1111.5 [M+H]+.

### Synthesis of CPD3

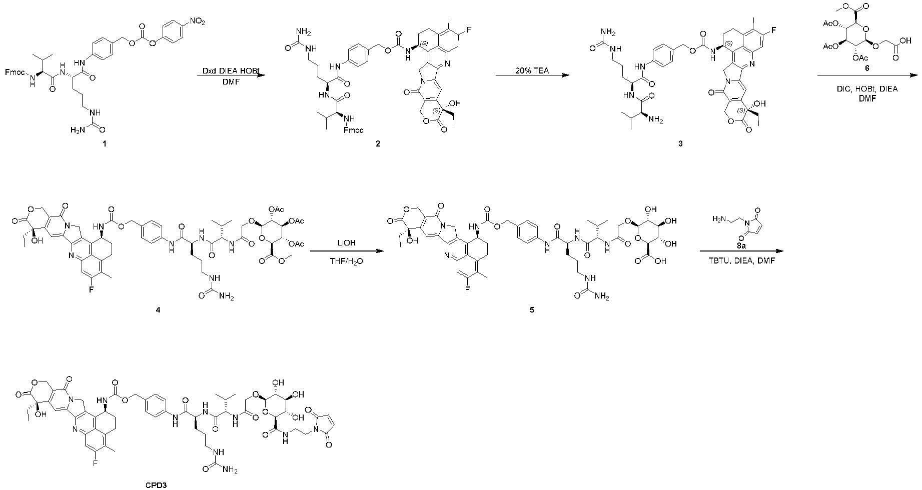

### General steps for preparing compound 2

Compound 1 (2 g, 2.61 mmol) and exatecan (970 mg, 1.83 mmol) were dissolved in DMF (10 mL), and HOBt (422 mg, 3.13 mmol) and DIEA (5.22 mmol, 908 µL) were then added to the solution. The mixture was stirred at 25 °C for 3 h. LC-MS analysis showed that reactant 1 had been completely consumed. The reaction mixture was distilled under reduced pressure to remove the solvent. Crude compound 2 (2.7 g) as a white oil was directly used in the next step without further purification. MS (ES+) 1063.3 [M+H]+.

### General steps for preparing compound 3

Compound 2 (2.70 g, 2.54 mmol) was dissolved in DMF (16 mL), and TEA (28.7 mmol, 4 mL) was then added to the solution. The mixture was stirred at 25 °C for 10 h. LC-MS analysis showed that compound 2 had been completely consumed. The reaction mixture was distilled under reduced pressure to remove the solvent, and a crude product was obtained. The crude product was purified by preparative high performance liquid chromatography (TFA conditions) to give compound 3 as a yellow solid (1.3 g, 60.8% yield); MS (ES+) 841.2 [M+H]+.

### General steps for preparing compound 4

Compound 3 (1.2 g, 1.43 mmol) was dissolved in DMF (10 mL), and DIEA (2.85 mmol, 497 µL) was then added to the solution. The mixture was stirred at 25 °C for 10 min. Subsequently, compound 6 (559 mg, 1.43 mmol), HOBt (212 mg, 1.57 mmol), and DIC (2.85 mmol, 442 µL) were added to the mixture. The mixture was stirred at 25 °C for 3 h. LC-MS analysis showed that compound 3 had been completely consumed, and one main product peak was detected. The reaction mixture was distilled under reduced pressure to remove the solvent, and a crude product was obtained. The crude product was purified by preparative high performance liquid chromatography (TFA conditions) to give compound 4 as a yellow solid (1.3 g, 74.9% yield). MS (ES+) 1215.3 [M+H]+.

### General steps for preparing compound 5

Compound 4 (500 mg, 411 µmol) was dissolved in THF (100 µL), and LiOH.H2O (172 mg, 4.11 mmol) was then added to the solution. The mixture was stirred at 0 °C for 5 min. LC-MS analysis showed that compound 4 had been completely consumed, and one main product peak was detected. The reaction mixture was distilled under reduced pressure to remove the solvent, and a crude product was obtained. The crude product was compound 5 as a green solid (158 mg, 35.7% yield). Note: Reactions totaling 500 mg were carried out in parallel, yielding 300 mg of compound 5. MS (ES+) 1075.2 [M+H]+.

### General steps for preparing CPD3

Compound 5 (300 mg, 278 µmol) and compound 8a (30.8 mg, 418 µmol) were dissolved in DMF (600 µL), and TBTU (94.3 mg, 556 µmol) and DIEA (1.11 mmol, 102 µL) were then added to the solution. The mixture was stirred at 25 °C for 1 h. LC-MS analysis showed that compound 5 had been completely consumed, and one main product peak was detected. The reaction mixture was distilled under reduced pressure to remove the solvent, and a crude product was obtained. The crude product was purified by preparative high performance liquid chromatography (TFA conditions) to give compound CPD3 as a yellow solid (106 mg, 31.8% yield). MS (ES+) 1197.2 [M+H]+.

### Synthesis of CPD4

### General steps for preparing compound 5A-1

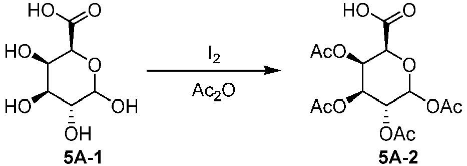

Compound 5A-1 (5 g, 25.8 mmol) was dissolved in Ac2O (80 mL), and I2 (392 mg, 1.55 mmol) was slowly added to the solution, with the reaction mixture kept at 0 °C. After the addition, the mixture was stirred at that temperature for 2 h. LC-MS analysis showed that the reactant had been completely consumed, and the spectrum showed several newly formed peaks, with about 80% being products. With the reaction mixture kept at 0 °C, 50 mL of dry MeOH was added dropwise. Then the mixture was left to stand at 25 °C for 18 h. The reaction mixture was diluted with 150 mL of 1 M Na2SO3 and extracted with 150 mL of DCM (50 mL × 3). The organic phase was dried over Na2SO4, filtered, and distilled under reduced pressure to give a crude product. The crude product was slurried with 2-acetonylpropane at 25 °C for 30 min to give compound 5A-2 as a white solid (5 g, 53.6% yield). MS (ES+) 380.2 [M+H2O]+.

### General steps for preparing compound 5A-3

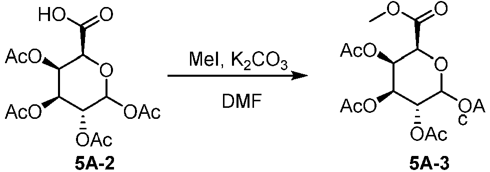

Compound 5A-2 (5 g, 13.8 mmol), iodomethane (3.13 g, 22.1 mmol), and K2CO3 (5.72 g, 41.4 mmol) were dissolved in DMF (2 mL), and the solution was purged with nitrogen three times and then stirred at 25 °C for 16 h under a N2 atmosphere. LC-MS analysis showed that the reactant had been completely consumed, and one main product peak was detected. 10 mL of water and 130 mL of ethyl acetate (10 mL × 3) were added to the reaction mixture. The organic phase was separated, washed with 20 mL of 1 M NaCl (10 mL × 2), dried over Na2SO4, filtered, and distilled under reduced pressure to give a crude product. The crude product was slurried with 2-acetonylpropane at 25 °C for 30 min to give compound 5A-3 as a yellow oil (4.4 g, 84.7% yield).

MS (ES+) 394.3 [M+Na]+.

### General steps for preparing compound 5A-3-1

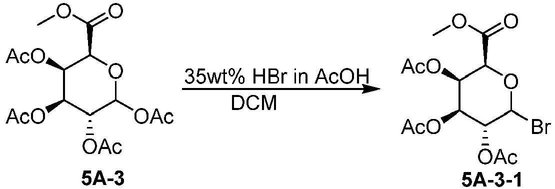

Compound 5A-3 (1 g, 2.66 mmol) was dissolved in DCM (10 mL), and 35% HBr in AcOH (2 mL) was added to the solution. The mixture was stirred at 25 °C for 16 h. LC-MS analysis showed that the reactant had been completely consumed and showed several newly formed peaks, with the main peak being the product. 10 mL of H2O was added to the reaction mixture at 25 °C to quench the reaction, and the mixture was then diluted with 10 mL of EtOAc and extracted with 30 mL of EtOAc (10 mL × 3). The organic phase was washed with 30 mL of 1 M NaCl (10 mL × 3), dried over Na2SO4, filtered, and distilled under reduced pressure to give a crude product. The crude product was directly used in the next step without further purification. The crude product was compound 5A-3-1 as a yellow oil (1.00 g, 94.8% yield).

MS (ES+) 342.9.

### General steps for preparing compound 5A-4

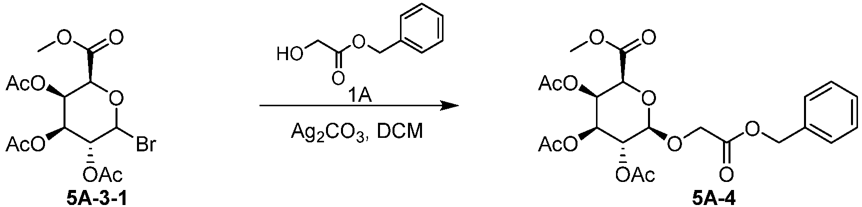

Compound 5A-3-1 (2.00 g, 5.04 mmol) was dissolved in DCM (10 mL), and Ag2CO3 (1.39 g, 5.04 mmol) and benzyl 2-hydroxyacetate (1.26 g, 7.55 mmol) were added to the solution. The mixture was stirred at 25 °C for 16 h. LC-MS analysis showed that reactant 1 had been completely consumed, and a main peak was detected as a product peak. 10 mL of H2O was added to the reaction mixture at 25 °C to quench the reaction, and the mixture was then diluted with 5 mL of EtOAc and extracted with 15 mL of EtOAc (5 mL × 3). The combined organic phases were washed with 15 mL of NaCl (5 mL × 3), dried over Na2SO4, filtered, and distilled under reduced pressure to give a crude product. The crude product was purified by silica gel column chromatography (SiO2, petroleum ether/ethyl acetate = 1/3). The product was compound 5A-4 as a yellow oil (800 mg, 1.66 mmol, 32.93% yield). MS (ES+) 500.2 [M+H2O]+.

### General steps for preparing compound 5A

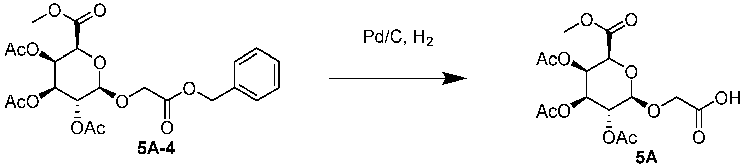

First, a 50-mL round-bottom flask was purged with Ar three times. Then, 100 mg of Pd/C (100 mg, 1.04 mmol, 10% purity) (dry) was carefully added. Subsequently, THF (1 mL) was added to completely soak the Pd/C. Then a solution of compound 5A-4 (500 mg, 1.04 mmol) in THF (4 mL) was slowly added dropwise under an Ar atmosphere. The resulting mixture was purged with H2 three times and then stirred at 25 °C for 2 h under H2. LC-MS monitoring showed that the reaction had finished. The reaction mixture was carefully filtered under reduced pressure under a N2 atmosphere. Upon completion of filtration, water was added to the filter membrane to keep the Pd/C wet. The organic phase was distilled under reduced pressure to give a crude product. The crude product was directly used in the next step without further purification. The crude product was compound 5A as a yellow solid (400 mg, 98.4% yield). MS (ES+) 410.1 [M+H2O]+.

### General steps for preparing compound 5

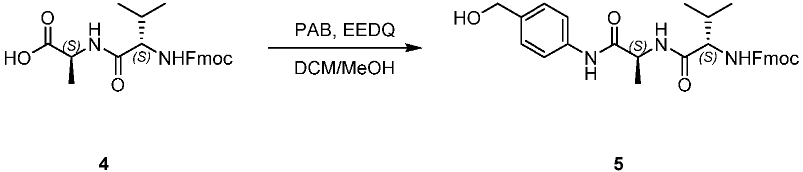

Compound 4 (1.00 g, 2.44 mmol) and p-aminobenzyl alcohol (600 mg, 4.87 mmol) were dissolved in a mixed solvent of DCM (5 mL) and MeOH (5 mL), and EEDQ (1.20 g, 4.87 mmol) was added to the solution under protection from light. The mixture was stirred at 45 °C for 1.5 h. LC-MS monitoring showed that the reactant had been completely consumed, and a main peak was detected as a product peak. The reaction mixture was distilled under reduced pressure to remove the solvent, and a crude product was obtained. The crude product was slurried with MTBE:EtOAc (10:1). The slurry was filtered, and the solid was dried. The solid was directly used in the next step without further purification. Compound 5 (1.26 g, 100% yield) was a white solid.

MS (ES+) 516.0 [M+H]+.

### General steps for preparing compound 6

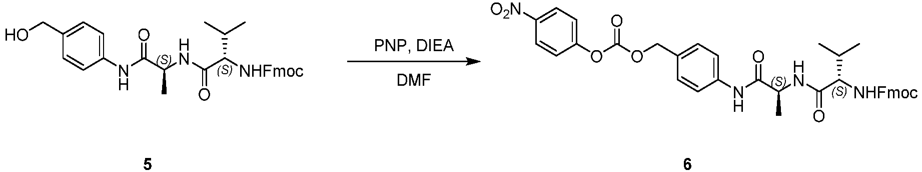

Compound 5 (1.26 g, 2.44 mmol) and PNP (1.49 g, 4.89 mmol) were dissolved in DMF (12.6 mL), and DIEA (851 µL, 4.89 mmol) was added to the solution. The mixture was stirred at 25 °C for 1.5 h. LC-MS monitoring showed that compound 4 had been completely consumed, and a main peak was detected as a product peak. Ice-cold isopropyl ether (500 mL) was used to cause precipitation in the reaction mixture. The mixture was filtered, and the solid was dried to give a crude product. The crude product was directly used in the next step without further purification. Compound 6 (1.23 g, 73.9% yield) was a light pink solid.

MS (ES+) 681.1 [M+H]+.

### General steps for preparing compound 7

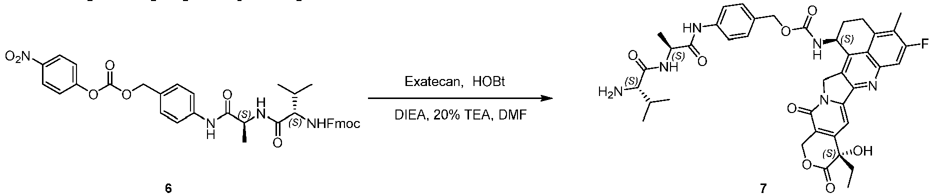

Compound 6 (1.23 g, 1.81 mmol) and exatecan (866 mg, 1.63 mmol) were dissolved in DMF (12.3 mL), and DIEA (631 µL, 3.62 mmol) and HOBt (293 mg, 2.17 mmol) were added to the solution. The mixture was stirred at 25 °C for 16 h. LC-MS monitoring showed that the reactant had been completely consumed, and a main peak was detected as a product peak. Then 20% TEA was added to the reaction mixture, and the mixture was stirred for another 4 h. The reaction mixture was purified by preparative HPLC (TFA conditions). Column: UniSil 10-120 C18 50 × 250 mm; mobile phase: [water (TFA)-propionitrile]; B%: 15%-45%, 23 min. Compound 7 (1.69 g, 95.56% yield) was a yellow solid. MS (ES+) 755.2 [M+H]+.

### General steps for preparing compound 8

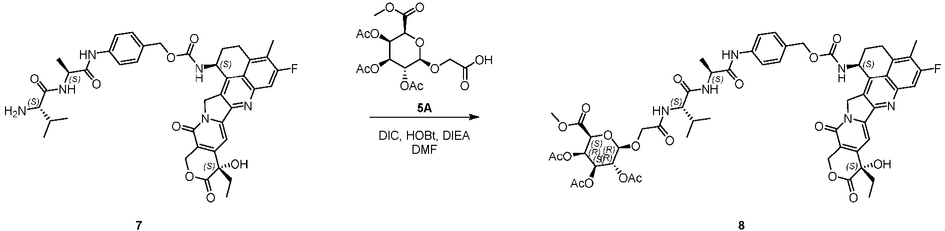

Compound 7 (117 mg, 155 µmol) and compound 5A (60.8 mg, 155 µmol) were dissolved in DMF (1 mL), and HOBt (23.0 mg, 171 µmol), DIC (48.0 µL, 310 µmol), and DIEA (54.0 µL, 310 µmol) were added to the solution. The mixture was stirred at 25 °C for 3 h. LC-MS monitoring showed that the reaction had been complete. The reaction mixture was slurried with isopropyl ether twice, filtered, and dried to a solid state. The crude product was directly used in the next step without further purification. Compound 8 (149 mg, 85.1% yield) was a green solid. MS (ES+) 1129.0 [M+H]+.

### General steps for preparing compound 9

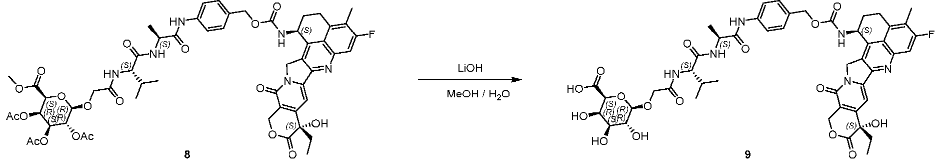

Compound 8 (149 mg, 132 µmol) was dissolved in a mixed solvent of MeOH (5.96 mL) and water, and LiOH·H2O (55.4 mg, 1.32 mmol) was then added. The mixture was stirred at 0 °C for 4 min, and H2O (1.49 mL) was then added. The mixture was stirred for 30 min. LC-MS monitoring showed that the reaction had been complete, and the main peak was a product peak. TFA was added at 0 °C to acidify the reaction mixture, and the mixture was distilled under reduced pressure to remove the solvent. The crude product was purified by preparative high performance liquid chromatography (TFA conditions). A Welch Ultimate C18 column (150 × 25 mm × 5 µm) and mobile phase [water (TFA)-ACN] were used, B% was gradually increased from 22% to 52%, and gradient elution was performed over 10 min. Compound 9 (47.0 mg, 36.0% yield) was obtained as a yellow solid. MS (ES+) 989.3 [M+H]+.

### General steps for preparing CPD4

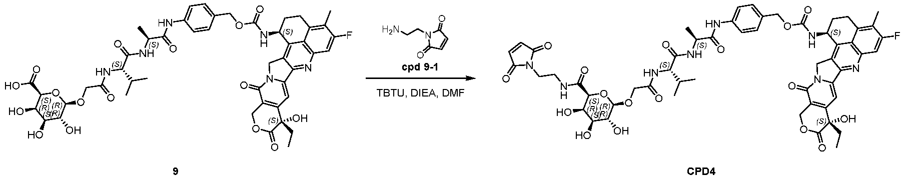

Compound 9 (47.0 mg, 47.5 µmol) and compound 9-1 (9.99 mg, 71.3 µmol) were dissolved in DMF (0.5 mL), and TBTU (30.5 mg, 95.1 µmol) and DIEA (33.2 µL, 190.10 µmol) were then added. The mixture was stirred at 25 °C for 1 h. LC-MS analysis showed that the reaction had been complete, and the main peak was a product peak. TFA was added at 0 °C to acidify the reaction mixture. The crude product was purified by preparative high performance liquid chromatography (TFA conditions). A C18-1 column (150 × 30 mm × 5 µm) and mobile phase [water (TFA)-ACN] were used, B% was gradually increased from 23% to 46%, and gradient elution was performed over 21 min. Compound CPD4 (29.0 mg, 54.9% yield) was obtained as a yellow solid. MS (ES+) 1111.2 [M+H]+.

### Synthesis of CPD5

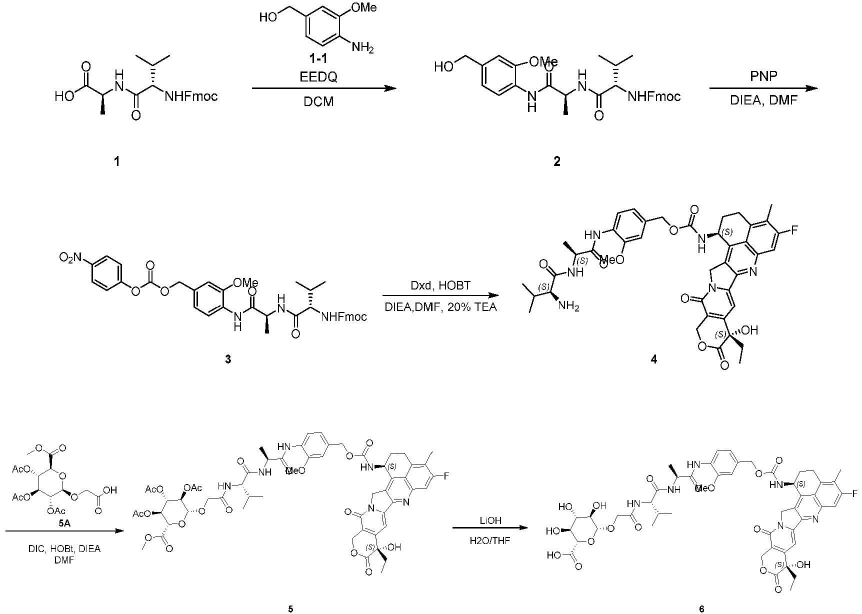

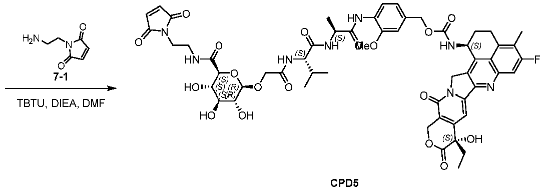

### General steps for preparing compound 2

Compound 1 (2.00 g, 4.87 mmol) and compound 1-1 (1.49 g, 9.75 mmol) were dissolved in a mixed solvent of DCM (16 mL) and MeOH (16 mL), and EEDQ (2.41 g, 9.75 mmol) was added under protection from light. The mixture was stirred at 25 °C for 2 h. LC-MS analysis showed that the reaction had been complete, and the main peak was a product peak. The reaction mixture was distilled under reduced pressure to remove the solvent. The crude product was slurried with MTBE:EtOAc (10:1). The slurry was filtered, and the solid was dried. The solid was directly used in the next step without further purification. Compound 2 (1.54 g, 57.9% yield) was obtained as a brown solid. MS (ES+) 546.2 [M+H]+.

### General steps for preparing compound 3

Compound 2 (1.54 g, 2.82 mmol) and PNP (3.43 g, 11.3 mmol) were dissolved in DMF (15.5 mL), and DIEA (1.47 mL, 8.47 mmol) was then added. The mixture was stirred at 25 °C for 2 h. LC-MS monitoring showed that the reaction had been complete. Ice-cold isopropyl ether (500 mL × 3) was used to cause precipitation in the reaction mixture. The mixture was filtered, and the solid was dried. The solid was used in the next step without further purification. Compound 3 (1.14 g, 56.6% yield) was obtained as a brown solid. MS (ES+) 711.3 [M+H]+.

### General steps for preparing compound 4

Compound 3 (500 mg, 704 µmol) and exatecan (337 mg, 633 µmol) were dissolved in DMF (5 mL), and HOBt (114 mg, 844 µmol) and DIEA (245 µL, 1.41 mmol) were then added. The mixture was stirred at 25 °C for 1.5 h. LC-MS monitoring showed that the reaction had been complete. Then TEA (1.25 mL, 8.98 mmol) was added to the reaction mixture, and the mixture was stirred at 25 °C for 16 h. After the reaction finished, TFA was added to the reaction mixture at 0 °C to acidify it. The crude product was purified by preparative HPLC (TFA conditions). Column: Phenomenex Luna C18 200 × 40 mm × 10 µm; mobile phase: [water (TFA)-acetonitrile]; B%: 18%-48%, 10 min. Compound 4 (331 mg, 59.9% yield) was obtained as a yellow solid. MS (ES+) 785.3 [M+H]+.

### General steps for preparing compound 5

Compound 4 (331 mg, 422 µmol) and compound 5A (331 mg, 843 µmol) were dissolved in DMF (3.5 mL), and HOBt (125 mg, 928 µmol), DIC (261 µL, 1.69 mmol) and DIEA (367 µL, 2.11 mmol) were then added. The mixture was stirred at 25 °C for 6 h. LC-MS monitoring showed that the reaction had been complete. 50 mL of isopropyl ether (50 mL × 2) was used to cause precipitation in the reaction mixture. The turbid mixture was filtered, and drying was performed. The solid was used in the next step without further purification. Compound 5 (555 mg, crude) was obtained as a gray solid. MS (ES+) 1159.1 [M+H]+.

### General steps for preparing compound 6

Compound 5 (155 mg, 134 µmol) was dissolved in a mixed solvent of H2O (1 mL) and THF (0.5 mL), and a solution of LiOH·H2O (56.11 mg, 1.34 mmol) in H2O (200 µL) was then added. The mixture was stirred at 0 °C for 10 min. LC-MS analysis showed that the reactant had been completely consumed, and a main peak was detected as a product peak. CH3COOH was added at 0 °C to acidify the reaction mixture. The crude product was purified by preparative HPLC (TFA conditions). Column: Welch Xtimate C18 150 × 25 mm × 5 µm; mobile phase: [water (TFA)-acetonitrile]; B%: 20%-50%, 40 min. Compound 6 (50.0 mg, 36.7% yield) was obtained as a yellow solid. MS (ES+) 1019.4 [M+H]+.

### General steps for preparing CPD5

Compound 6 (50.0 mg, 49.1 µmol) and compound 7-1 (10.3 mg, 73.6 µmol) were dissolved in DMF (0.5 mL), and TBTU (31.5 mg, 98.1 µmol) and DIEA (34.2 µL, 196 µmol) were then added. The mixture was stirred at 25 °C for 1 h. LC-MS analysis showed that the reaction had been complete. The reaction mixture was purified by preparative HPLC (TFA conditions). Column: Welch Xtimate C18 150 × 25 mm × 5 µm; mobile phase: [water (TFA)-acetonitrile]; B%: 22%-52%, 10 min. Compound Target 1 (27.0 mg, 48.2% yield) was obtained as a yellow solid. MS (ES+) 1141.1 [M+H]+.

### Synthesis of CPD6

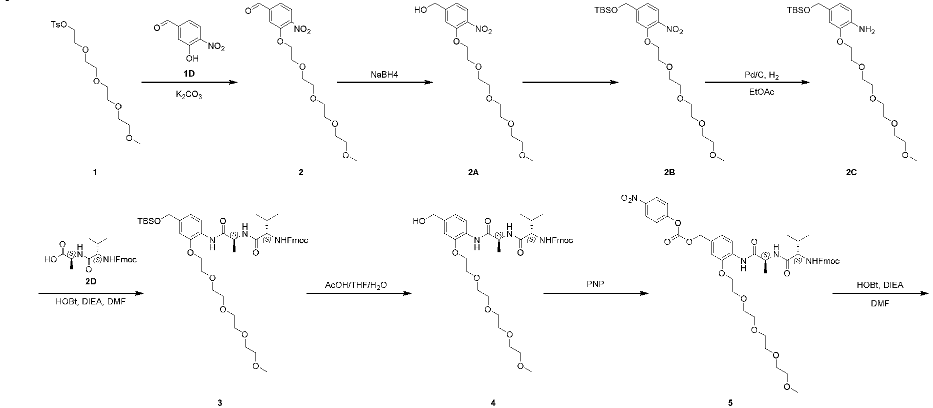

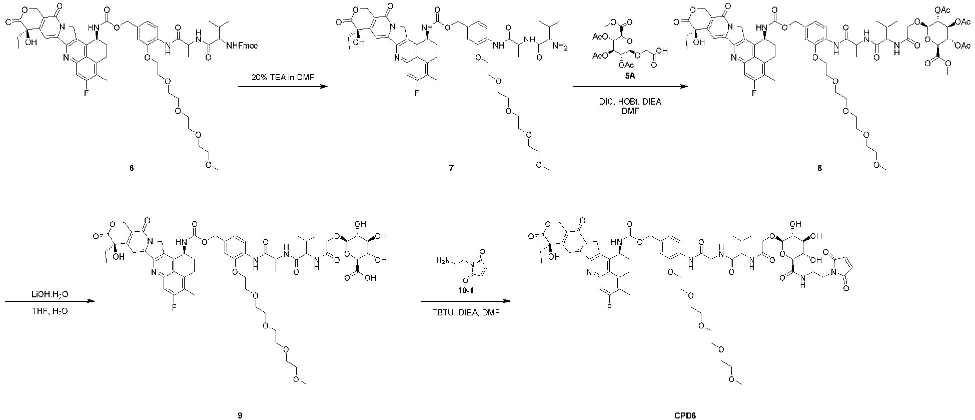

### General steps for preparing compound 2

Compound 1 (2.93 g, 8.08 mmol) and compound 1D (1.35 g, 8.08 mmol) were dissolved in DMF (40 mL), and K2CO3 (2.23 g, 16.2 mmol) was then added. The mixture was stirred at 80 °C for 3 h. LC-MS monitoring showed that the reaction had been complete, and the main peak was a product peak. The reaction mixture was filtered to remove K2CO3. The filtrate was purified by preparative HPLC (TFA conditions). Compound 2 (2.30 g, 79.6% yield) was obtained as a yellow liquid. MS (ES+) 358.1 [M+H]+.

### General steps for preparing compound 2A

Compound 2 (1.93 g, 5.40 mmol) was dissolved in MeOH (40 mL), and NaBH4 (306 mg, 8.10 mmol) was then added at 0 °C. The mixture was stirred at 0 °C for 30 min. LC-MS monitoring showed that compound 2 had been completely consumed, and the main peak was a product peak. 1 N HCl (200 mL) was added to the reaction mixture at 0 °C to quench the reaction. Subsequently, the mixture was diluted with DCM (100 mL) and extracted (200 mL × 2). The organic phases were combined, washed with brine (200 mL), dried over Na2SO4, filtered, and then distilled under reduced pressure to give the product. Compound 2A (crude, 1.89 g) was obtained as a white oil. MS (ES+) 360.1 [M+H]+.

### General steps for preparing compound 2B

Compound 2A (3.00 g, 8.35 mmol) was dissolved in DMF (30 mL), and 1H-imidazole (1.70 g, 25.0 mmol) and tert-butyldimethylchlorosilane (2.52 g, 16.7 mmol) were then added. The mixture was stirred at 25 °C for 2 h. LC-MS monitoring showed that compound 2A had been completely consumed, and the main peak was a product peak. The reaction mixture was poured into ice water (250 mL). The mixture was extracted with ethyl acetate (200 mL × 3). The organic phases were combined, washed with brine (200 mL), dried over Na2SO4, and distilled under reduced pressure to remove the solvent. The crude product was directly used in the next step without purification. Compound 2B (3.50 g, 79.4% yield) was obtained as a yellow oil. MS (ES+) 474.3 [M+H]+.

### General steps for preparing compound 2C

A 250-mL round-bottom flask was purged with argon three times, and 300 mg of dry Pd/C was added. Then ethyl acetate (20 mL) was slowly added to completely soak the Pd/C. Subsequently, a solution of compound 2B (3.50 g, 7.39 mmol) in ethyl acetate (20 mL) was slowly added under an argon atmosphere. The resulting mixture was purged with hydrogen three times and then stirred at 25 °C for 3 h under H2 (15 psi). The reaction was monitored by LC-MS. The reaction mixture was carefully filtered under reduced pressure under a nitrogen atmosphere. Then water was immediately added to the filter cake. The organic phase was concentrated under reduced pressure to give a crude product. The crude product was used in the next step without further purification. Compound 2C (3.18 g, 86.6% yield) was obtained as a yellow liquid. MS (ES+) 444.3 [M+H]+.

### General steps for preparing compound 3

Compound 2C (2.90 g, 6.54 mmol) and compound 2D (3.22 g, 7.84 mmol) were dissolved in DMF (30 mL), and EEDQ (3.23 g, 13.0 mmol) was added. The mixture was stirred at 25 °C for 2 h. LC-MS analysis showed that compound 2C had been completely consumed, and the main peak was a product peak. The reaction mixture was purified by preparative HPLC (TFA conditions). Compound 3 (2.8 g) was obtained as a white solid. MS (ES+) 858.6 [M+Na]+.

### General steps for preparing compound 4

Compound 3 (370 mg, 442.53 µmol) was dissolved in a mixed solvent of THF (1 mL) and water (1 mL), and AcOH (2 mL) was added. The mixture was stirred at 25 °C for 1 h. LC-MS analysis showed that compound 3 had been completely consumed, and a main peak with the expected m/z was detected. The reaction mixture was cooled using an ice bath and diluted with a saturated NaHCO3 solution (10 mL). The mixture was extracted with ethyl acetate (10 mL × 3). The combined organic phases were washed with water (20 mL) and brine (10 mL) and dried over sodium sulfate, and evaporation was then performed under reduced pressure. Compound 4 (290 mg, crude) was obtained as a white solid. MS (ES+) 704.4 [M-H₂O]+.

### General steps for preparing compound 5

Compound 4 (290 mg, 401 µmol) and bis(4-nitrophenyl) carbonate (244 mg, 803 µmol) were dissolved in DMF (4 mL), and DIEA (103 mg, 803 µmol) was added. The mixture was stirred at 25 °C for 2 h. LC-MS analysis showed that compound 4 had been completely consumed, and a main peak with the product m/z was detected. The mixture was diluted with 1 N hydrochloric acid (20 mL) and then extracted with DCM (20 mL × 3). The organic phases were combined, then dried over sodium sulfate, filtered, and distilled under reduced pressure to give a crude product. The crude product was purified by preparative HPLC (TFA conditions). Compound 5 (135 mg, 37.9% yield) was obtained as a white solid. MS (ES+) 887.5 [M+H]+.

### General steps for preparing compound 6

Compound 5 (135 mg, 152 µmol) and exatecan mesylate (89.0 mg, 167 µmol) were dissolved in DMF (2 mL), and HOBt (24.6 mg, 182 µmol) and DIEA (39.3 mg, 304 µmol) were added. The mixture was stirred at 25 °C for 2 h. LC-MS monitoring showed that compound 5 had been completely consumed, and a main peak with the product m/z was detected. The reaction mixture was added dropwise to MTBE (30 mL × 2) for precipitation, and a crude product was obtained. Compound 6 (190 mg, crude) was obtained as a yellow solid. MS (ES+) 1184.7 [M+H]+.

### General steps for preparing compound 7

Compound 6 (190 mg, 160 µmol) was dissolved in DMF (2 mL), and TEA (0.5 mL) was added. The mixture was stirred at 25 °C for 4 h. LC-MS monitoring showed that compound 6 had completely reacted, and a main peak with the product m/z was detected. The reaction mixture was purified by preparative HPLC (TFA conditions). Compound 7 (100 mg, 64.8% yield) was obtained as a yellow solid. MS (ES+) 961.6 [M+H]+.

### General steps for preparing compound 8

Compound 7 (100 mg, 104 µmol) and compound 5A (40.8 mg, 104 µmol) were dissolved in DMF (2 mL), and DIEA (26.9 mg, 208 µmol), HOBt (21.1 mg, 156 µmol), and DIC (26.4 mg, 208 µmol) were added. The mixture was stirred at 25 °C for 2 h. LC-MS monitoring showed that compound 7 had completely reacted, and the main peak was a product peak. The reaction mixture was added to MTBE (30 mL × 2) for precipitation. After filtration and drying, a crude product was obtained. The crude product was directly used in the next step without purification. Compound 8 (136 mg, crude) was obtained as a yellow solid. MS (ES+) 1335.8 [M+H]+.

### General steps for preparing compound 9

Compound 8 (136 mg, 102 µmol) was dissolved in a mixed solvent of THF (1 mL) and H2O (1 mL), and LiOH.H2O (42.7 mg, 1.02 mmol) was added. The mixture was stirred at 0 °C for 0.5 h. LC-MS monitoring showed that compound 8 had completely reacted, and the main peak was a product peak. Acetic acid was added to the reaction mixture to adjust the pH to 5. The reaction mixture was purified by preparative HPLC (TFA conditions). Compound 9 (65.0 mg, 53.4% yield) was obtained as a white solid. MS (ES+) 1195.7 [M+H]+.

### General steps for preparing CPD6

Compound 9 (65.0 mg, 54.4 µmol) was dissolved in DMF (1 mL), and compound 10-1 (9.15 mg, 65.2 µmol) was added. Then TBTU (34.9 mg, 108 µmol) and DIEA (28.1 mg, 217 µmol) were added to the reaction mixture. The mixture was stirred at 25 °C for 1 h. LC-MS monitoring showed that compound 9 had completely reacted, and the main peak was a product peak. The reaction mixture was directly purified by preparative HPLC (TFA conditions). Compound CPD6 (35 mg, 48.8% yield) was obtained as a yellow solid. MS (ES+) 1317.3 [M+H]+.

### Synthesis of CPT004

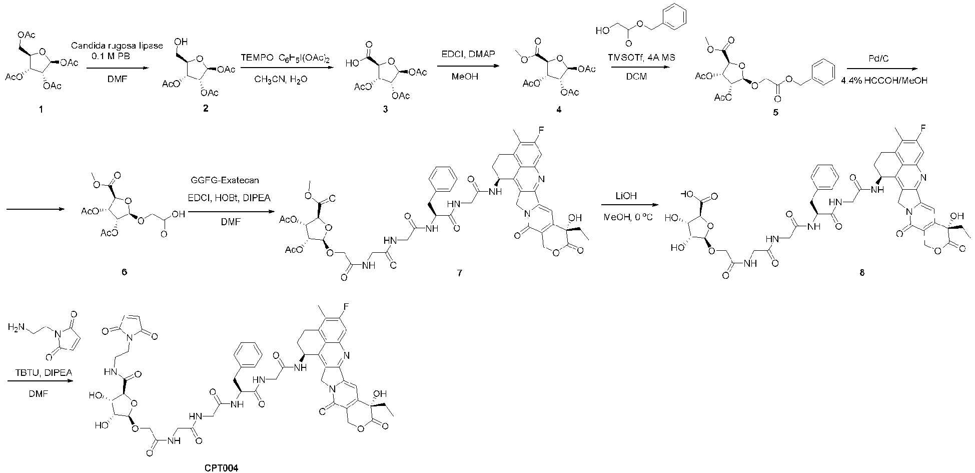

### General steps for preparing compound 2

Compound 1 (7.0 g, 21.99 mmol) was dissolved in DMF (50 mL), and the solution was diluted with a 0.1 M, pH 7.0 phosphate-buffered saline (450 mL). Then lipase C (5.93 g) was added, and the mixture was stirred at 25 °C for 24 h. The reaction mixture was filtered, and the filtrate was extracted with 1500 mL of ethyl acetate (3 × 500 mL). The organic phase was washed with 500 mL of saturated brine, dried over anhydrous sodium sulfate, filtered, and distilled under reduced pressure to remove the solvent, and a crude product was obtained. The crude product was purified on a silica gel column to give compound 2 (3.0 g, 40% yield). Compound 2 was a colorless oil.

### General steps for preparing compound 3

Compound 2 was dissolved in a mixed solvent of acetonitrile and water (30 mL, v/v = 1/1), and TEMPO (424 mg, 2.72 mmol) and BAIB (7.0 g, 21.72 mmol) were added to the solution. The mixture was stirred at 25 °C for 16 h, and the solvent was removed by evaporation under reduced pressure. The crude product was diluted with water and DCM and extracted with DCM (3 × 50 mL). The organic phase was washed with saturated brine (50 mL) and dried over anhydrous sodium sulfate, and the solvent was removed by evaporation under reduced pressure to give a crude product. The crude product (4.0 g) was directly used in the next step without purification.

### General steps for preparing compound 4

Compound 3 (4.0 g, 13.78 mmol) was dissolved in methanol (50 mL), and EDCI (5.28 g, 27.56 mmol) and DMAP (0.34 mg, 2.76 mmol) were added to the solution. The mixture was stirred at 25 °C for 2 h and concentrated by rotary evaporation to remove the methanol. The crude product was diluted with DCM (50 mL) and washed with water (50 mL × 3) and saturated brine (2 × 50 mL). The organic phase was dried over anhydrous sodium sulfate, filtered, and concentrated by rotary evaporation to give a crude product. The crude product was purified on a silica gel column to give compound 4 (1.3 g, 31.0% yield). Compound 4 was a colorless oil.

### General steps for preparing compound 5

4A molecular sieve (0.3 g) was added to a three-necked flask, heated at high temperature for 5 min, and cooled, and anhydrous DCM (200 mL) was then added. Compound 4 (0.3 g, 0.99 mmol), compound 6 (0.2 g, 1.18 mmol), and TMSOTf (0.26 g, 1.18 mmol) were sequentially added to the mixture. The mixture was purged with nitrogen three times and stirred at 25 °C for 3 h under a nitrogen atmosphere. The reaction was monitored by TLC (developing solvent: PE/EA = 3/1). After the reaction was complete, the mixture was filtered to remove the 4A molecular sieve, and the filtrate was diluted with 50 mL of DCM and washed with saturated sodium bicarbonate (30 mL) and saturated saline (30 mL × 3). The organic phase was dried over anhydrous sodium sulfate and concentrated by rotary evaporation to remove the solvent, and a crude product was obtained. The crude product was purified on a silica gel column to give compound 5 (0.2 g, 49.4% yield). Compound 5 is a white solid. MS (ES+) 428.6 [M+H2O+H]+.

### General steps for preparing compound 6

Compound 5 (0.2 g, 2.07 mmol) was dissolved in 4.4% formic acid in methanol (20 mL), and Pd/C (0.2 g, wet) was added to the solution. The mixture was left to react at 25 °C for 5 h, and TLC (developing solvent: PE/EA = 2/1) monitoring showed that compound 5 had completely reacted. The mixture was filtered through diatomaceous earth to give a filtrate. The filtrate was concentrated by rotary evaporation to remove the solvent, and a crude product was obtained. The crude product (0.15 mg, 96.1% yield) was directly used in the next step without purification.

### General steps for preparing compound 7

Compound 6 (350 mg, 0.46 mmol) and GGFG-exatecan (150 mg, 0.46 mmol) were dissolved in DMF (5 mL), and HOBt (94 mg, 0.70 mmol), EDCI (133 mg, 0.70 mmol), and DIEA (171 µL, 0.93 mmol) were added. The mixture was stirred at 25 °C for 2 h, and HPLC analysis showed that compound 6 had completely reacted. The reaction mixture was concentrated by rotary evaporation to remove the solvent, and the residue was purified by preparative HPLC to give compound 7 (115 mg, 23.5% yield). Compound 7 was a yellow solid.

### General steps for preparing compound 8

Compound 7 (115 mg, 0.109 mmol) was dissolved in a mixed solvent of THF/MeOH/H2O (5 mL), and a solution of LiOH (13 mg, 0.54 mmol) in H2O (0.5 mL) was added at 0 °C. The mixture was left to react at 0 °C for 0.5 h, and HPLC monitoring showed that compound 7 had completely reacted. The reaction mixture was concentrated by rotary evaporation to remove the solvent, and the residue was purified by preparative HPLC. Compound 8 (50.0 mg, 47.9% yield) was a white solid.

### General steps for preparing CPT004

Compound 8 (50.0 mg, 52.20 µmol) and 1-(2-aminoethyl)-1H-pyrrole-2,5-dione hydrochloride (18.44 mg, 104.39 µmol) were dissolved in DMF (2.0 mL), and TBTU (25.14 mg, 78.29 µmol) and DIEA (19.27 µL, 104.39 µmol) were added. The mixture was stirred at 25 °C for 1 h, and HPLC monitoring showed that the reaction had been complete. The reaction mixture was diluted with MeOH (2 mL) and purified by preparative HPLC to give the product. CPT004 (14 mg, 24.8% yield) was a yellow solid. HRMS (ES+) 1079.854 [M+H]+.

¹H NMR (400 MHz, DMSO-d6) δ 8.42 (d, J = 8.6 Hz, 1H), 8.31 (s, 1H), 8.16 (t, J = 5.6 Hz, 2H), 8.09 (d, J = 8.0 Hz, 1H), 8.04 (t, J = 5.5 Hz, 1H), 7.80 (d, J = 11.0 Hz, 1H), 7.32 (s, 1H), 7.27 - 7.13 (m, 5H), 6.94 (s, 2H), 5.65 - 5.51 (m, 1H), 5.48 - 5.35 (m, 2H), 5.31 - 5.18 (m, 2H), 4.89 (d, J = 2.0 Hz, 1H), 4.48 - 4.40 (m, 1H), 4.18 - 4.02 (m, 4H), 3.83 (dd, J = 4.4, 2.1 Hz, 1H), 3.73 (t, J = 6.2 Hz, 6H), 3.29 - 3.16 (m, 6H), 2.98 (dd, J = 13.7, 4.7 Hz, 1H), 2.77 (dd, J = 13.5, 9.5 Hz, 1H), 2.41 (s, 3H), 2.20 (s, 1H), 2.12 (s, 1H), 1.86 (tt, J = 14.0, 7.1 Hz, 2H), 0.87 (t, J = 7.3 Hz, 3H).

### Synthesis of CPT005

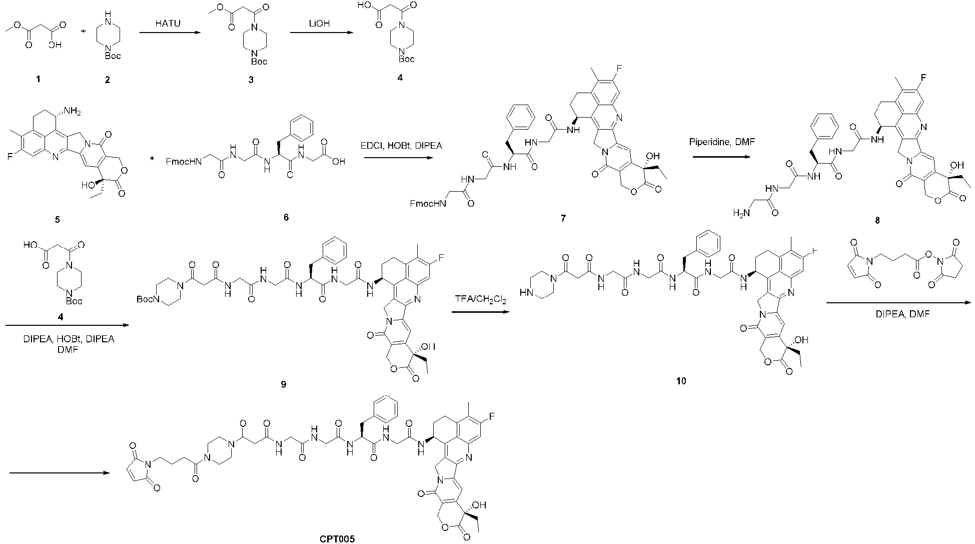

### General steps for preparing compound 3

Compound 1 (2.0 g, 16.94 mmol) and compound 2 (2.84 g, 15.24 mmol) were dissolved in DMF (20 mL), and DIEA (2.85 mmol, 9.4 mL) and HATU (9.66 g, 25.40 mmol) were added. The mixture was stirred at 25 °C for 3 h. TLC monitoring showed that compound 2 had completely reacted. The reaction mixture was diluted with H2O (50 mL) and extracted with ethyl acetate (50 mL × 3). The organic phases were combined, washed with a saturated aqueous sodium chloride solution (50 mL), dried over anhydrous Na2SO4, filtered, and concentrated by rotary evaporation to remove the solvent, and a crude product was obtained. The crude product was purified by silica gel column chromatography. Compound 3 (4.0 g, 82.5% yield) was obtained as a yellow solid.

MS (ES+) 309.2 [M+Na]+.

### General steps for preparing compound 4

Compound 3 (4.0 g, 13.97 mmol) was dissolved in a mixed solvent of THF/MeOH/H2O (50 mL, 3/1/1), and LiOH (1.0 g, 41.91 mmol) was then added. The mixture was stirred at 25 °C for 3 h. TLC monitoring showed that compound 3 had completely reacted. The reaction mixture was diluted with H2O (50 mL) and extracted with ethyl acetate (50 mL × 3). The organic phases were combined, washed with a saturated aqueous sodium chloride solution (20 mL), dried over Na2SO4, filtered, and concentrated by rotary evaporation to remove the solvent, and a crude product was obtained. The crude product (2.0 g, 52.6% yield) was directly used in the next step without further purification. MS (ES+) 295.1 [M+Na]+.

### General steps for preparing compound 7

Compound 5 (1.0 g, 2.30 mmol) and compound 6 (1.28 g, 2.30 mmol) were dissolved in DMF (20 mL), and HOBt (0.46 g, 3.44 mmol), DIEA (1.27 mL, 6.89 mmol), and EDCI (0.66 g, 3.44 mmol) were added. The mixture was left to react at 25 °C for 2 h. LC-MS monitoring showed that reactant 5 had completely reacted, and the main peak was a product peak. The reaction mixture was diluted with H2O (50 mL) and extracted with ethyl acetate (20 mL × 3). The organic phases were combined, washed with a saturated aqueous sodium chloride solution (50 mL), dried over Na2SO4, filtered, and concentrated by rotary evaporation to remove the solvent, and a crude product was obtained. The crude product was purified by silica gel column chromatography. Compound 7 (2.0 g, 89.2% yield) was obtained as a yellow solid.

### General steps for preparing compound 8

Compound 7 (1.0 g, 1.02 mmol) was dissolved in DMF (10 mL), and TEA (2 mL) was added. The mixture was stirred at 25 °C for 12 h. HPLC monitoring showed that compound 7 had completely reacted. The reaction mixture was poured into ice water (50 mL) for precipitation, and the turbid mixture was filtered to give a solid. The solid was washed with ethyl acetate and dried to give compound 8 (0.5 g, 64.7% yield). Compound 8 was a yellow solid. MS (ES+) 754.4 [1/2 M+H]+.

### General steps for preparing compound 9

Compound 8 (0.4 g, 0.53 mmol) and compound 4 (0.22 g, 0.80 mmol) were dissolved in DMF (10 mL), and HOBt (0.11 g, 0.80 mmol), EDCI (0.15 g, 0.80 mmol), and DIEA (0.19 mL, 1.06 mmol) were added. The mixture was stirred at 25 °C for 2 h. TLC monitoring showed that reactant 8 had completely reacted. The reaction mixture was diluted with H2O (50 mL) and extracted with ethyl acetate (20 mL × 3). The organic phases were combined, washed with a saturated aqueous sodium chloride solution (50 mL), dried over Na2SO4, filtered, and concentrated by rotary evaporation to give a crude product. The crude product was purified by silica gel column chromatography to give the product. Compound 9 (0.17 g, 31.8% yield) was obtained as a yellow solid.

### General steps for preparing compound 10

Compound 9 (0.150 g, 0.15 mmol) was dissolved in DMF (3 mL), and TFA (13.39 mmol, 1 mL) was added. The mixture was stirred at 25 °C for 5 h. HPLC monitoring showed that compound 9 had completely reacted. The solvent was removed by rotary evaporation to give a crude product. The crude product was purified by preparative high performance liquid chromatography. Compound 10 (0.1 g, 74% yield) was a yellow solid. MS (ES+) 454.8 [1/2 M+H]+.

### General steps for preparing CPT005

Compound 10 (100 mg, 0.11 mmol) and GMBS (46.3 mg, 0.17 mmol) were dissolved in DMF (2.2 mL), and DIEA (40.7 mL, 0.22 mmol) was added. The mixture was stirred at 0 °C for 1 h. HPLC monitoring showed that compound 10 had completely reacted. The solvent was removed by rotary evaporation to give a crude product. The crude product was purified by preparative high performance liquid chromatography. CPT005 (30 mg, 25.4% yield) was obtained as a yellow solid. MS (ES+) 537.3 [1/2 M+H]+.

¹H NMR (400 MHz, DMSO) δ 8.42 (s, 2H), 8.32 (s, 1H), 8.18 (s, 1H), 8.06 (s, 1H), 7.81 (d, J = 11.1 Hz, 1H), 7.32 (s, 1H), 7.20 (dd, J = 18.0, 10.1 Hz, 5H), 6.99 (s, 2H), 5.57 (s, 1H), 5.42 (s, 2H), 5.25 (s, 2H), 4.43 (s, 1H), 3.71 (s, 6H), 3.42 (d, J = 18.6 Hz, 15H), 3.18 (s, 2H), 2.98 (d, J = 10.3 Hz, 1H), 2.78 (d, J = 10.6 Hz, 1H), 2.41 (s, 3H), 2.32 (d, J = 7.2 Hz, 2H), 2.20 (s, 1H), 2.08 (s, 1H), 1.91 - 1.79 (m, 2H), 1.77 - 1.68 (m, 2H), 0.87 (t, J = 7.2 Hz, 3H).

### Synthesis of CPT006

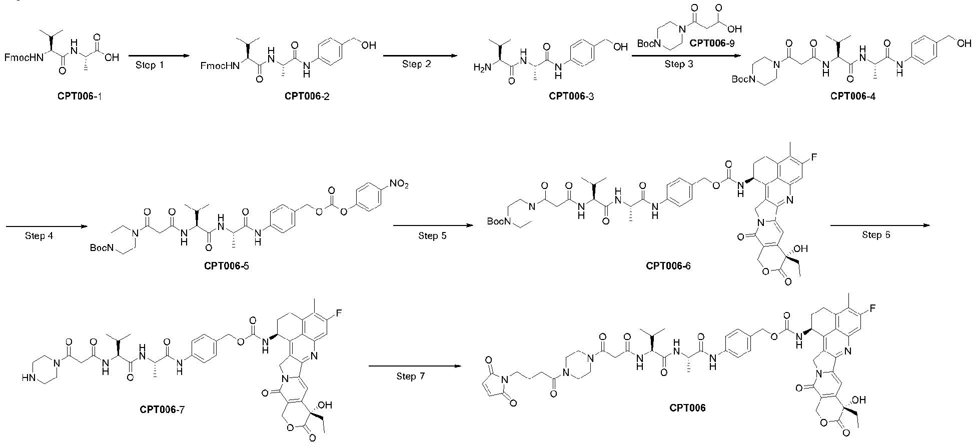

### General steps for synthesizing CPT006-2

Compound CPT006-1 (100.0 mg, 243.9 µmol, 1.0 eq.), 2-ethoxy-1-ethoxycarbonyl-1,2-dihydroquinoline (EEDQ, 60.0 mg, 246.7 µmol, 1.0 eq.), and p-aminobenzyl alcohol (30.0 mg, 243.6 µmol, 1.0 eq.) were sequentially added to a 50-mL flask, and a mixed solvent of methanol/dichloromethane (1:4) was added. The reaction mixture was stirred at 25 °C for 6 h, and the reaction was monitored by high performance liquid chromatography until it was complete. The solvent was removed by rotary evaporation, and the residue was slurried with 20 mL of petroleum ether at room temperature for 2 h. The slurry was filtered to give CPT006-2 as a pale yellow solid. LC-MS: 538.2 [M+Na].

### General steps for synthesizing CPT006-3

Compound CPT006-2 (100.0 mg, 194.2 µmol, 1.0 eq.) was dissolved in a mixed solution of 3 mL of dichloromethane and 1 mL of N,N-dimethylformamide, and 40.0 µL of piperidine was added. The mixture was stirred at 25 °C for 1 h. The reaction was monitored by thin-layer chromatography (TLC). After the reaction finished, the solvent was removed by rotary evaporation to give a yellow solid, namely crude CPT006-3. The crude product was directly used in the next step without purification. LC-MS: 294.3 [M+H].

### General steps for synthesizing CPT006-4

The crude compound CPT006-3 was dissolved in 5 mL of dichloromethane, and CPT006-9 (53.0 mg, 194.9 µmol) and 2-ethoxy-1-ethoxycarbonyl-1,2-dihydroquinoline (EEDQ, 48.2 mg, 194.9 µmol, 1.0 eq.) were added. The reaction was monitored by thin-layer chromatography (TLC). After the reaction finished, the solvent was removed by rotary evaporation to give a yellow semisolid, namely crude CPT006-4. The crude product was purified on a silica gel column (petroleum ether/ethyl acetate (10:1)) to give the target product as a colorless oil (60 mg).

Structural characterization: LC-MS: 570.3 [M+Na], 474.3 [M-tBuO], 448.3 [M-Boc].

### General steps for synthesizing CPT006-5

Compound CPT006-4 (120.0 mg, 219.3 µmol, 1.0 eq.), bis(p-nitrophenyl) carbonate (132.0 mg, 434.2 µmol, 2.0 eq.), and N,N-diisopropylethylamine (72 µL, 434.2 µmol, 2.0 eq.) were dissolved in 10 mL of N,N-dimethylformamide, and the solution was stirred overnight at 25 °C. 20 mL of purified water was added for dilution. After 1 h of stirring, extraction was performed with ethyl acetate (50 mL × 3). The organic phases were combined, then washed with a half-saturated aqueous Na2CO3 solution (50 mL × 2), dried over anhydrous sodium sulfate, filtered, and then concentrated to dryness by rotary evaporation to give CPT006-5 as a pale yellow solid (90 mg). The crude product was directly used in the next step without further purification.

### General steps for synthesizing CPT006-6

Compound CPT006-5 (20 mg, 30.0 µmol, 1.2 eq.) and exatecan mesylate (11.5 mg, 26.4 µmol, 1.0 eq.) were dissolved in 2 mL of N,N-dimethylformamide, and N,N-diisopropylethylamine (8.3 µL, 50.0 µmol, 2.0 eq.) was added. The reaction was monitored by HPLC until it was complete. The reaction solvent was removed under reduced pressure to give a crude product, and the crude product was directly purified by preparative high performance liquid chromatography followed by lyophilization to give the trifluoroacetate of compound CPT006-6 (13 mg).
Mobile phase A: acetonitrile (0.1% trifluoroacetic acid); mobile phase B: water (0.1% trifluoroacetic acid).
Structural characterization: LC-MS: 1009.4 [M+H].

### General steps for synthesizing CPT006-7

Compound CPT006-6 (13.0 mg, 12.9 µmol, 1.0 eq.) was dissolved in 2 mL of 20% trifluoroacetic acid in dichloromethane. After 2 h of reaction, the reaction was monitored by thin-layer chromatography (TLC). After the reaction finished, the solvent was removed by rotary evaporation to give crude CPT006-7 as a pale yellow solid (15 mg).

Structural characterization: LC-MS: 455.2 [M+2H]/2, 909.4 [M+H].

### General steps for synthesizing CPT006

Compound CPT006-7 (15.0 mg, 14.7 µmol, 1.0 eq.) and 4-maleimidobutyric acid-N-hydroxysuccinimide ester (10.0 mg, 35.7 µmol, 2.5 eq.) were dissolved in 2 mL of N,N-dimethylformamide. N,N-Diisopropylethylamine (4.8 µL, 29.4 µmol, 2.0 eq.) was added with stirring. The mixture was left to react at 25 °C for 16 h, and the reaction was monitored by high performance liquid chromatography until the starting material was completely consumed. The reaction solvent was removed under reduced pressure, and the concentrate was directly purified by high performance liquid chromatography followed by lyophilization to give pale yellow compound CPT006 (5 mg).
Mobile phase A: acetonitrile (0.1% trifluoroacetic acid); mobile phase B: water (0.1% trifluoroacetic acid).
Structural characterization: LC-MS: 1074.4 [M+H].

¹H NMR (400 MHz, DMSO) δ 9.78 (s, 1H), 8.35 (dd, J = 5.0, 1.9 Hz,1H), 8.21 (d, J = 8.0 Hz, 1H), 8.07 (dd, J = 8.8, 1.6 Hz, 1H), 7.79 (d, J = 10.9 Hz, 1H), 7.61 (d, J = 8.3 Hz, 2H), 7.37 (d, J = 8.3 Hz, 2H), 7.32 (s, 1H), 6.99 (s, 2H), 6.53 (m, 1H), 5.45 (s, 2H), 5.29 (s, 3H), 5.08 (s, 2H), 4.36 (dd, J = 10.5, 4.1 Hz, 2H), 4.20 (dd, J = 11.2, 3.6 Hz, 2H), 2.38 (s, 2H), 2.32 (dd, J = 11.8, 4.3 Hz, 2H), 2.18 (m, 4H), 2.00 (dd, J = 14.7, 7.1 Hz, 4H), 1.88 (m, 3H), 1.73 (m, 2H), 1.33 (d, J = 7.1 Hz, 2H), 1.24 (s, 6H), 0.89 (m, 9H).

### Synthesis of CPT008

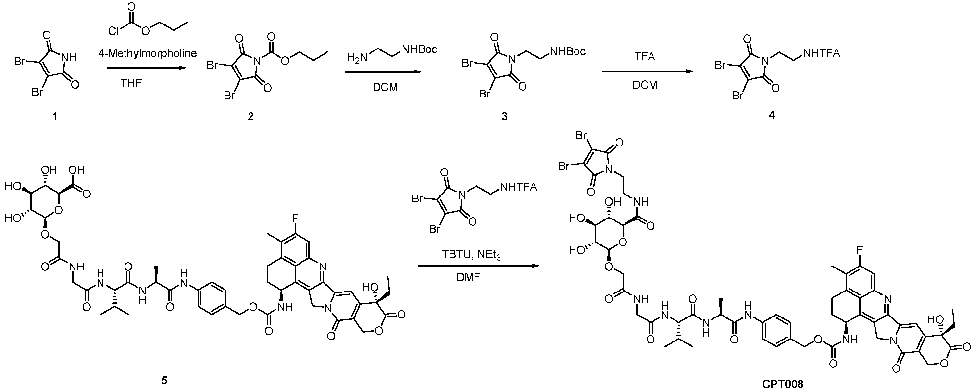

### Synthesis of compound 2

1,4-Dibromomaleimide and N-methylmorpholine were dissolved in tetrahydrofuran, and propyl chloroformate was then added. The reaction was stirred at room temperature for 20 min. After the stirring, an appropriate amount of dichloromethane was added. The organic phase was washed with water, dried over anhydrous sodium sulfate, and filtered, and the solvent was evaporated under reduced pressure. The crude product was directly used in the next step.

### Synthesis of compound 3

Compound **2** and N-tert-butylcarbonyl-ethylenediamine were each dissolved in a dichloromethane solution. The N-tert-butylcarbonyl-ethylenediamine solution was slowly added using an addition funnel to the solution of compound **2** in dichloromethane. The mixture was stirred for 2 h. After the reaction finished, the reaction mixture was washed with a saturated aqueous ammonium chloride solution and then washed with a saturated aqueous sodium chloride solution. The organic phase was dried over anhydrous sodium sulfate and filtered, and the solvent was evaporated under reduced pressure. The residue was further purified by silica gel chromatography. After the solvent was removed by evaporation under reduced pressure, a purified product was obtained.

### Synthesis of compound 4

Compound **3** was dissolved in a mixture of dichloromethane/trifluoroacetic acid (TFA) (1: 1), and the mixture was stirred at room temperature for 1 h. The solvent was evaporated under reduced pressure to give a crude product.

### Synthesis of compound CPT008

Compound 4 and compound 5 were dissolved in an N,N-dimethylformamide solution, and triethylamine and 2-(1H-benzotriazole-1-yl)-1,1,3,3-tetramethyluronium tetrafluoroborate were then sequentially added. The reaction was stirred at room temperature for 30 min. After the stirring, methanol was added for dilution, and purification was then performed by preparative liquid phase chromatography. The solvent was removed by evaporation under reduced pressure to give a purified product.

### Synthesis of CPT009

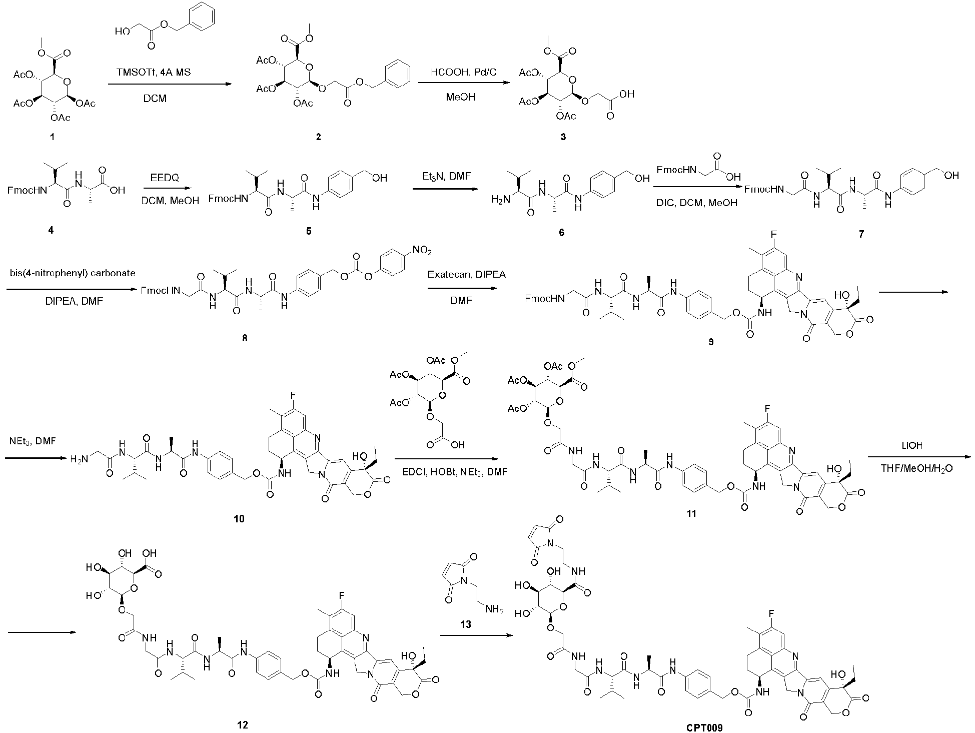

### General steps for preparing compound 2

Compound 1 (10 g, 26.57 mmol), compound 6 (6.62 g, 39.86 mmol), 4A MS (10 g), and trimethylsilyl trifluoromethanesulfonate (5.91 g, 26.57 mmol) were mixed and dissolved in anhydrous dichloromethane (200 mL), and the mixture was purged with nitrogen three times and stirred at 25 °C for 3 h under a N2 atmosphere. TLC monitoring showed that the reaction had been complete. The reaction mixture was filtered to remove the 4A MS (50 g). The filtrate was diluted with dichloromethane (200 mL) and washed with a saturated sodium bicarbonate solution (100 mL), and the aqueous phase was extracted with dichloromethane (150 mL × 3). The combined organic phases were washed with saturated brine (150 mL), dried over Na2SO4, and concentrated by rotary evaporation to give a crude product. The crude product was purified by silica gel column chromatography. Compound 2 (3.0 g, 23.4% yield) was obtained as a white solid.

### General steps for preparing compound 3

Compound 2 (1.0 g, 2.07 mmol) was dissolved in a 4.4% formic acid-methanol solution (20 mL), and Pd/C (0.1 g) was then added. The mixture was stirred at 25 °C for 5 h. TLC monitoring showed that compound 2 had completely reacted. The reaction mixture was filtered through silica filter paper. The filtrate was concentrated by rotary evaporation to give a crude product (0.8 g, 98.4% yield), and the crude product was used in the next step without further purification.

### General steps for preparing compound 5

Compound 4 (5.00 g, 12.18 mmol) and PAB (3.0 g, 24.36 mmol) were dissolved in a mixed solvent of dichloromethane (100 mL) and methanol (10 mL), and EEDQ (6.02 g, 24.36 mmol) was then added. The mixture was stirred at 25 °C for 1.5 h. TLC monitoring showed that compound 3 had completely reacted. The reaction mixture was a suspension and was filtered to give a solid. The solid was washed with dichloromethane (10 mL) and then dried under reduced pressure to give compound 5 (5.0 g, 79.6% yield) as a white solid.

### General steps for preparing compound 6

Compound 5 (3.0 g, 5.82 mmol) was dissolved in DMF (10 mL), and TEA (11.64 mmol, 2 mL) was added. The mixture was stirred at 25 °C for 12 h. HPLC monitoring showed that compound 5 had completely reacted. The reaction mixture was diluted with H2O (50 mL) and extracted with ethyl acetate (20 mL × 3). The combined organic phases were washed with a saturated sodium chloride solution (50 mL), dried over Na2SO4, filtered, and concentrated by rotary evaporation to give a crude product. The crude product (2.7 g) was used in the next step without further purification.

### General steps for preparing compound 7

Compound 6 (2.4 g, 8.18 mmol) and Fmoc-Gly-OH (2.68 g, 9.00 mmol) were dissolved in DMF (20 mL), and DIC (2.06 g, 16.36 mmol) was then added. The mixture was stirred at 25 °C for 2 h. TLC monitoring showed that reactant 6 had completely reacted. The reaction mixture was poured into ice water (50 mL) for precipitation, and the suspension was filtered to give a solid. The filter cake was washed with ethyl acetate and dried under reduced pressure to give compound 7 (2.0 g, 42.7% yield) as a white solid. MS (ES+) 595.3 [M+Na]+.

### General steps for preparing compound 8

Compound 7 (2.0 g, 3.49 mmol) and PNP (2.12 g, 6.98 mmol) were dissolved in DMF (20 mL), and DIEA (1.3 mL, 6.98 mmol) was then added. The mixture was stirred at 25 °C for 1.5 h. TLC monitoring showed that compound 7 had completely reacted. The reaction mixture was diluted with H2O (50 mL) and extracted with ethyl acetate (20 mL × 3). The combined organic phases were washed with a saturated sodium chloride solution (50 mL), dried over Na2SO4, filtered, and concentrated by rotary evaporation to give a crude product. The crude product was purified by silica gel chromatography. Compound 8 (1.5 g, 58.2% yield) was obtained as a yellow solid.

### General steps for preparing compound 9

Compound 8 (400 mg, 0.54 mmol) and exatecan mesylate (236.09 mg, 0.54 mmol) were dissolved in DMF (10 mL), and HOBt (80.59 mg, 0.60 mmol) and DIEA (200 µL, 1.08 mmol) were then added. The mixture was stirred at 25 °C for 16 h. TLC monitoring showed that the exatecan had completely reacted. The reaction mixture was diluted with H2O (50 mL) and extracted with EA (20 mL × 3). The combined organic phases were washed with a saturated sodium chloride solution (50 mL), dried over Na2SO4, filtered, and concentrated by rotary evaporation to give a crude product. The crude product was purified by silica gel chromatography. Compound 9 (0.38 g, 67.8% yield) was obtained as a yellow solid.

### General steps for preparing compound 10

Compound 9 (380 mg, 0.47 mmol) was dissolved in DMF (5 mL), and TEA (1.06 mL, 7.35 mmol) was then added. The mixture was stirred at 25 °C for 12 h. HPLC monitoring showed that compound 9 had completely reacted. The reaction mixture was poured into ice water (50 mL) for precipitation, and the mixture was filtered to give a solid. The solid was washed with ethyl acetate and dried under reduced pressure to give compound 10 (250 mg, 83.8% yield) as a white solid. MS (ES+) 406.8 [1/2 M+H]+.

### General steps for preparing compound 11

Compound 10 (250 mg, 0.31 mmol) and compound 3 (145 mg, 0.37 mmol) were dissolved in DMF (5 mL), and HOBt (62 mg, 0.46 mmol), EDCI (88 mg, 0.46 mmol), and DIEA (113 µL, 0.61 mmol) were then added. The mixture was stirred at 25 °C for 2 h. HPLC monitoring showed that reactant 10 had completely reacted. The reaction mixture was concentrated by rotary evaporation to remove the solvent, and a crude product was obtained. The crude product was purified by preparative HPLC. Compound 11 (70 mg, 19.1% yield) was obtained as a yellow solid. MS (ES+) 1186.4 [M+H]+.

### General steps for preparing compound 12

Compound 11 (70.0 mg, 0.059 mmol) was dissolved in a mixed solvent of THF/MeOH/H2O (5 mL), and a solution of LiOH (14.1 mg, 0.59 mmol) in H2O (0.5 mL) was then added. The mixture was stirred at 0 °C for 0.5 h. HPLC monitoring showed that compound 11 had completely reacted. The reaction mixture was concentrated by rotary evaporation to remove the solvent, and a crude product was obtained. The crude product was purified by preparative HPLC. Compound 12 (40.0 mg, 64.8% yield) was obtained as a white solid. MS (ES+) 1046.4 [M+H]+.

### General steps for preparing CPT009

Compound 12 (13.0 mg, 12.43 µmol) was dissolved in DMF (2.0 mL), and compound 13 (2.61 mg, 18.64 µmol) was then added. Subsequently, EDCI (3.57 mg, 18.64 µmol), HOBt (2.52 mg, 18.64 µmol), and DIEA (4.59 µL, 24.86 µmol) were added to the reaction mixture. The mixture was stirred at 25 °C for 1 h. HPLC monitoring showed that the reaction had been complete. The reaction mixture was diluted with MeOH (2 mL) and directly purified by preparative HPLC followed by lyophilization to give CPT009 (5 mg, 34.4% yield) as a yellow solid. MS (ES+) 1076.4 [M+H]+.

### Synthesis of CPT010

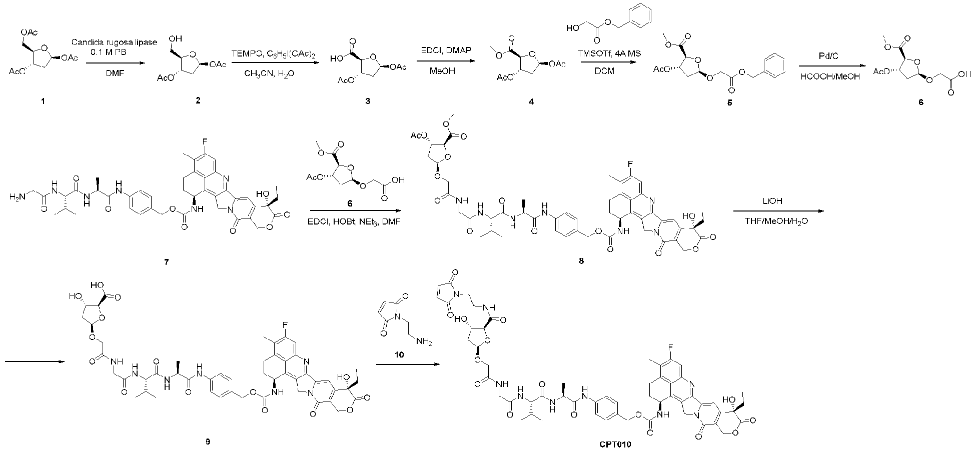

### Synthesis method for compound 2:

A solution of compound 1 in N,N-dimethylformamide was diluted with a 0.1 M, pH 7.0 sodium phosphate buffer, and C. rugosa lipase was added to the mixed solution. The mixture was stirred at 25 °C for 24 h. The mixture was filtered, and the filtrate was extracted with ethyl acetate. The organic phase was washed with brine and dried over sodium sulfate, and evaporation was performed under reduced pressure. The crude product was purified by silica gel column chromatography to give compound 2.

### Synthesis method for compound 3

TEMPO and BAIB were added to a solution of compound 2 in acetonitrile/water solution. The mixture was stirred at 25 °C for 18 h. The solvent was removed by evaporation *in vacuo.* The residue was extracted with DCM. The organic layer was washed with saturated brine, dried over sodium sulfate, and concentrated under reduced pressure to give a residue. The crude product was used in the next step.

### Synthesis method for compound 4

EDCI and DMAP were added to a solution of compound 3 in methanol. The mixture was stirred at room temperature for 2 h. Methanol was evaporated, and the residue was diluted with dichloromethane, washed with water, and then washed with brine. The organic extract was dried over anhydrous sodium sulfate, filtered, and concentrated under reduced pressure to give a residue. The crude product was purified by silica gel column chromatography to give compound 4 as a colorless oil.

### Preparation method for compound 5

Compound 4, benzyl 2-hydroxyacetate, 4A MS, and trimethylsilyl trifluoromethanesulfonate were degassed in anhydrous DCM, purged with N2 three times, and then stirred at 25 °C for 3 h under a N2 atmosphere. The reaction was monitored by TLC (eluent: PE/EA = 3/1). The reaction mixture was filtered to remove the 4A MS, and the residue was diluted with DCM and washed with Sat. NaHCO3. The organic phase was dried over Na2SO4, concentrated, and purified by silica gel chromatography.

### Preparation method for compound 6

Compound 5 was dissolved in a 4.4% formic acid-methanol solution, and Pd/C was added. The mixture was stirred at 25 °C for 5 h. TLC analysis (eluent: PE/EA = 2/1) showed that compound 5 had been completely consumed. The reaction mixture was filtered through a layer of diatomaceous earth. The filtrate was concentrated under reduced pressure. The crude product was used in the next step without further purification.

### Preparation method for compound 8

HOBt, EDCI, and DIEA were added to a solution of compound 6 and compound 7 in DMF. The mixture was stirred at 25 °C for 2 h. HPLC analysis showed that reactant 6 had been completely consumed. The reaction mixture was concentrated under reduced pressure to remove the solvent and purified by pre-high performance liquid chromatography. Compound 8 was a yellow solid.

### Preparation method for compound 9

Compound 8 was dissolved in a THF/MeOH/H2O solution, and LiOH in H2O was then added. The mixture was stirred at 0 °C for 0.5 h, and HPLC analysis showed that compound 8 had been completely consumed. The reaction mixture was concentrated under reduced pressure to remove the solvent and purified by pre-high performance liquid chromatography. Compound 9 was a white solid.

### Preparation method for CPT010

TBTU and DIEA were added to a solution of compound 9 and 1-(2-aminoethyl)-1h-pyrrole-2,5-dione hydrochloride in DMF. The mixture was stirred at 25 °C for 1 h. High performance liquid chromatography analysis showed that the reaction had been complete. The reaction mixture was diluted with MeOH and directly purified by pre-high performance liquid chromatography followed by lyophilization to give CPT010 as a yellow solid.

### Synthesis of MMAE derivative 1

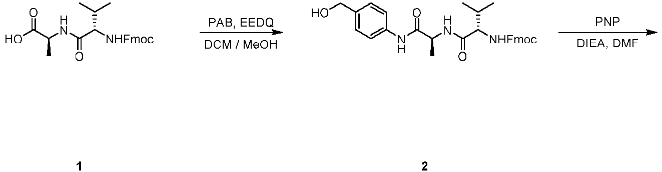

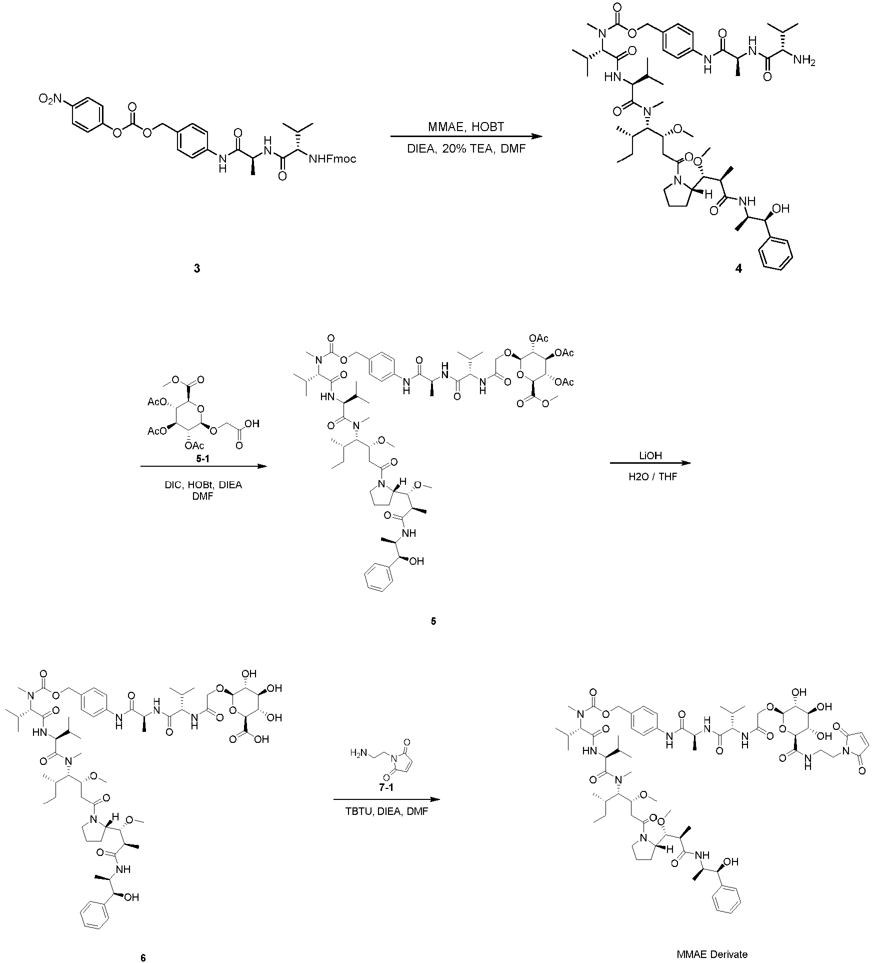

### General steps for preparing compound 2

Compound 1 (10.0 g, 24.4 mmol) and PAB (6.00 g, 48.7 mmol) were dissolved in a mixed solvent of DCM (80 mL) and MeOH (80 mL), and EEDQ (12.0 g, 4.87 mmol) was then added under protection from light. The mixture was stirred at 45 °C for 1.5 h. LC-MS monitoring showed that reactant 1 had completely reacted, and the main peak was a product peak. The reaction mixture was concentrated by rotary evaporation to remove the solvent, and a crude product was obtained. The crude product was slurried with MTBE:EtOAc (10:1). After filtration and centrifugal drying, a product was obtained. The product was used in the next step without further purification. Compound 2 (11.4 g, 90.7% yield) was obtained as a white solid. MS (ES+) 516.3 [M+H]+.

### General steps for preparing compound 3

Compound 2 (11.4 g, 22.1 mmol) and PNP (13.4 g, 44.2 mmol) were dissolved in DMF (114 mL), and DIEA (7.69 mL, 44.2 mmol) was then added. The mixture was stirred at 25 °C for 1.5 h. LC-MS monitoring showed that reactant 2 had completely reacted, and the main peak was a product peak. Ice-cold isopropyl ether (1500 mL) was used to cause precipitation in the reaction mixture. After filtration and centrifugal drying, a solid was obtained. The solid was used in the next step without further purification. Compound 3 (13.9 g, 92.3% yield) was obtained as a yellow solid. MS (ES+) 681.4 [M+H]+.

### General steps for preparing compound 4

Compound 3 (1.00 g, 1.47 mmol) and MMAE (1.06 g, 1.47 mmol) were dissolved in DMF (10 mL), and HOBt (218 mg, 1.62 mmol) and DIEA (512 µL, 2.94 mmol) were then added. The mixture was stirred at 25 °C for 16 h. LC-MS monitoring showed that the reactant had been completely consumed, and the main peak was a product peak. Then 20% triethylamine was added to the reaction mixture, and the mixture was stirred at 25 °C for another 2 h. The reaction mixture was purified by preparative HPLC (TFA conditions). Column: Phenomenex Luna C18 250 × 50 mm × 10 µm; mobile phase: [water (TFA)-acetonitrile]; B%: 30%-60%, 20 min. Compound 4 (910 mg, 59.7% yield) was obtained as a white solid. MS (ES+) 1037.6 [M+H]+.

### General steps for preparing compound 5

Compound 4 (400 mg, 386 µmol) and compound 5-1 (151 mg, 386 µmol) were dissolved in DMF (4 mL), and HOBt (115 mg, 848 µmol), DIEA (269 µL, 1.54 mmol), and DIC (239 µL, 1.54 mmol) were then added. The mixture was stirred at 25 °C for 6 h. LC-MS monitoring showed that compound 4 had completely reacted, and the main peak was a product peak. 100 mL of isopropyl ether (100 mL × 3) was used to cause precipitation in the reaction mixture. After filtration and centrifugal drying, a solid was obtained. The solid was used in the next step without further purification. Compound 5 (429 mg, 78.8% yield) was obtained as a white solid. MS (ES+) 1411.5 [M+H]+.

### General steps for preparing compound 6

Compound 5 (200 mg, 142 µmol) was dissolved in a mixed solvent of THF (1 mL) and H2O (1 mL), and LiOH·H2O (59.5 mg, 1.42 mmol) was then added. The mixture was stirred at 0 °C for 5 min. LC-MS monitoring showed that compound 5 had been completely consumed, and the main peak was a product peak. CH3COOH was added at 0 °C to acidify the reaction mixture. The mixture was purified by preparative HPLC (TFA conditions). Column: Welch Ultimate C18 150 × 25 mm × 5 µm; mobile phase: [water (TFA)-acetonitrile]; B%: 31%-61%, 10 min. Compound 6 (100 mg, 55.5% yield) was obtained as a white solid. MS (ES+) 1271.5 [M+H]+.

### General steps for preparing MMAE derivative 1

Compound 6 (50.0 mg, 39.3 µmol) and compound 7-1 (16.5 mg, 118 µmol) were dissolved in DMF (0.5 mL), and DIEA (27.4 µL, 157 µmol) and TBTU (25.3 mg, 78.7 µmol) were then added. The mixture was stirred at 25 °C for 1 h. LC-MS monitoring showed that compound 6 had completely reacted, and the main peak was a product peak. TFA was added at 0 °C to acidify the reaction mixture. The mixture was purified by preparative HPLC (TFA conditions). Column: Welch Ultimate C18 150 × 25 mm × 5 µm; mobile phase: [water (TFA)-acetonitrile]; B%: 30%-60%, 10 min. MMAE derivative 1 (28.0 mg, 51.09% yield) was obtained as a white solid. MS (ES+) 1393.7 [M+H]+.

### Synthesis of MMAE derivative 2

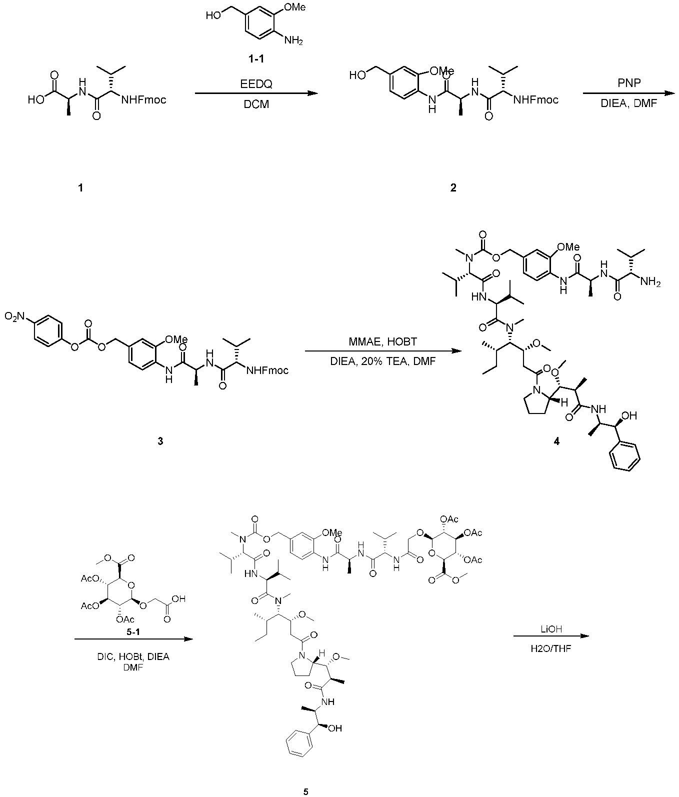

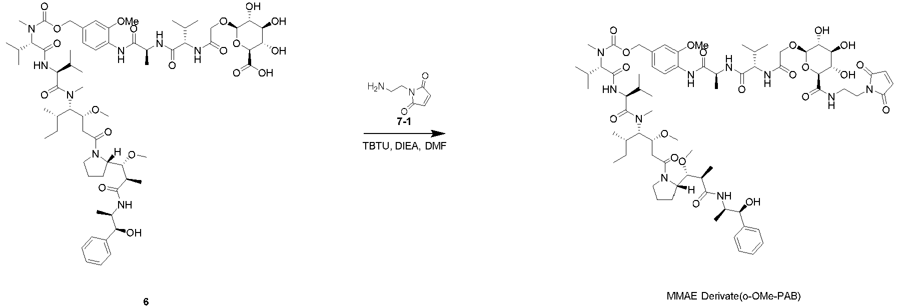

### General steps for preparing compound 2

Compound 1 (1.00 g, 2.44 mmol) and compound 1-1 (746 mg, 4.87 mmol) were dissolved in a mixed solvent of DCM (8 mL) and MeOH (8 mL), and EEDQ (1.20 g, 4.87 mmol) was then added under protection from light. The mixture was stirred at 25 °C for 2 h. LC-MS monitoring showed that compound 1 had completely reacted, and the main peak was a product peak. The solvent was removed by rotary evaporation to give a crude product. The crude product was slurried with MTBE:EtOAc (10:1). The slurry was filtered, and the solid was dried by rotary evaporation. The product was used in the next step without further purification. Compound 2 (611 mg, 46% yield) was obtained as a brown solid. MS (ES+) 546.4 [M+H]+.

### General steps for preparing compound 3

Compound 2 (0.611 g, 1.12 mmol) and PNP (1.36 g, 4.48 mmol) were dissolved in DMF (6.2 mL), and DIEA (585 µL, 3.36 mmol) was then added. The reaction mixture was stirred at 25 °C for 4.5 h. LC-MS monitoring showed that compound 2 had completely reacted, and the main peak was a product peak. 150 mL of ice-cold isopropyl ether (150 mL × 3) was added to cause precipitation in the reaction mixture. The mixture was filtered, and the solid was dried by rotary evaporation. The product was used in the next step without further purification. Compound 3 (0.272 g, 34.2% yield) was obtained as a brown solid. MS (ES+) 711.4 [M+H]+.

### General steps for preparing compound 4

Compound 3 (0.272 g, 383 µmol) and MMAE (275 mg, 382.71 µmol) were dissolved in a solution of DMF (3 mL), and HOBt (56.9 mg, 421 µmol) and DIEA (133 µL, 765 µmol) were then added. The mixture was stirred at 25 °C for 16 h. LC-MS monitoring showed that the reactant had completely reacted, and the main peak was a product peak. Then 20% TEA was added to the reaction mixture, and the mixture was stirred at 25 °C for another 2 h. The reaction mixture was purified by preparative HPLC (TFA conditions). Column: Phenomenex Luna C18 200 × 40 mm × 10 µm; mobile phase: [water (TFA)-acetonitrile]; B%: 30%-60%, 10 min. Compound 4 (0.212 g, 51.9% yield) was obtained as a brown solid. MS (ES+) 1067.5 [M+H]+.

### General steps for preparing compound 5

Compound 4 (0.212 g, 199 µmol) and compound 5-1 (156 mg, 397 µmol) were dissolved in a solution of DMF (2.2 mL), and HOBt (59.0 mg, 437 µmol), DIEA (138 µL, 794 µmol), and DIC (123 µL, 794 µmol) were then added. The mixture was stirred at 25 °C for 2 h. LC-MS monitoring showed that compound 4 had completely reacted, and the main peak was a product peak. CH3COOH was added to acidify the reaction mixture. The mixture was purified by preparative HPLC (TFA conditions). Column: Phenomenex Luna C18 200 × 40 mm × 10 µm; mobile phase: [water (TFA)-acetonitrile]; B%: 43%-73%, 10 min. Compound 5 (0.118 g, 41.2% yield) was obtained as a yellow solid. MS (ES+) 1441.6 [M+H]+.

### General steps for preparing compound 6

Compound 5 (100 g, 69.4 µmol) was dissolved in a mixed solvent of H2O (0.8 mL) and THF (0.2 mL), and LiOH·H2O (29.1 mg, 694 µmol) was then added. The mixture was stirred at 0 °C for 5 min. LC-MS monitoring showed that compound 5 had completely reacted, and the main peak was a product peak. CH3COOH was added to acidify the reaction mixture. The mixture was purified by preparative HPLC (TFA conditions). Column: Welch Ultimate C18 150 × 25 mm × 5 µm; mobile phase: [water (TFA)-acetonitrile]; B%: 30%-60%, 10 min. Compound 6 (45.0 mg, 49.9% yield) was obtained as a white solid. MS (ES+) 1301.6 [M+H]+.

### General steps for synthesizing MMAE derivative 2

Compound 6 (45.0 mg, 34.6 µmol) and compound 7-1 (7.27 mg, 51.9 µmol) were dissolved in a solution of DMF (0.5 mL), and TBTU (22.2 mg, 69.2 µmol) and DIEA (24.1 µL, 138 µmol) were then added. The mixture was stirred at 25 °C for 1 h. LC-MS monitoring showed that compound 6 had completely reacted, and the main peak was a product peak. Trifluoroacetic acid (TFA) was added to acidify the reaction mixture. The mixture was purified by preparative HPLC (TFA conditions). Column: Welch Xtimate C18 150 × 25 mm × 5 µm; mobile phase: [water (TFA)-acetonitrile]; B%: 30%-60%, 10 min. MMAE derivative 2 (o-OMe-PAB) (32.0 mg, 65.0% yield) was obtained as a white solid. MS (ES+) 1424.6 [M+H]+.

### Synthesis of Comp.5

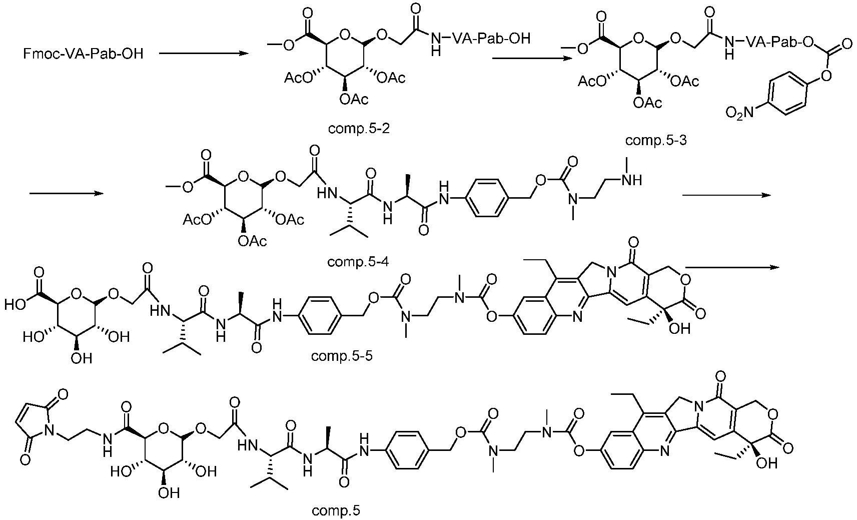

### General steps for synthesizing Comp.5-2:

Fmoc-VA-Pab-OH (1.0 g, 1.9 mmol, 1.0 eq) was dissolved in 20% triethylamine in dichloromethane (50 mL, 20% V/V triethylamine). The mixture was stirred at 25 °C for 16 h, and TLC monitoring showed that no starting material remained. The solvent was removed by rotary evaporation to give a crude product. The crude product was a white solid. The crude product was directly used in the next step without purification. The crude product was dissolved in 50 mL of dichloromethane, and comp.5-1 (0.91 g, 2.3 mmol, 1.2 eq) was added. After thorough stirring, N,N'-diisopropylcarbodiimide (DIC, 0.29 g, 2.3 mmol, 1.2 eq) was added dropwise. The mixture was stirred at 25 °C for 5 h, and TLC analysis showed that no NH2-VA-Pab-OH remained. 50 mL of water was added to stop the reaction. After liquid separation, the organic phase was washed with 50 mL of water twice, dried over anhydrous sodium sulfate, and filtered. The solvent was completely evaporated to give a crude product, and the crude product was purified by silica gel column chromatography (dichloromethane/methanol = 10:1) followed by rotary evaporation to give the product comp.5-2 as an off-white solid (800 mg).

### General steps for synthesizing Comp.5-3:

Comp.5-2 (800 mg, 1.2 mmol, 1.0 eq) was dissolved in 50 mL of N,N-dimethylformamide, and bis(p-nitrophenyl) carbonate (800 mg, 2.6 mmol, 2.2 eq) was added. After the mixture was stirred to uniformly dissolve the compounds, triethylamine (400 µL, 2.9 mmol, 2.4 eq) was added dropwise. After the mixture was stirred at 25 °C for 2 h, TLC analysis showed that no comp.5-2 remained. 100 mL of water and 100 mL of ethyl acetate were added, and the mixture was stirred well and then separated. The aqueous phase was extracted with 50 mL of ethyl acetate twice, and the organic phases were combined. The organic phase was washed with 50 mL of a 3% (m/m) aqueous sodium carbonate solution twice, washed with 50 mL of saturated brine once, dried over anhydrous sodium sulfate, and concentrated to give a crude product, and the crude product was purified by silica gel column chromatography (dichloromethane/methanol = 10:1) followed by rotary evaporation to give the product comp.5-3 as a pale yellow solid (620 mg).

### General steps for synthesizing Comp.5-4:

Comp.5-3 (400 mg, 0.48 mmol, 1.0 eq) was dissolved in 4 mL of N,N-dimethylformamide, and 4 mL of a solution of N,N'-dimethylethylenediamine (600 mg, 4.8 mmol, 10.0 eq) in N,N-dimethylformamide was added dropwise. The mixture was stirred at 25 °C for half an hour, and TLC monitoring showed that the reaction had finished. The solvent was removed by rotary evaporation to give a crude product, and the crude product was purified by silica gel column chromatography (dichloromethane/methanol = 10:1) followed by rotary evaporation to give the product comp.5-4 as a pale yellow solid (210 mg).

### General steps for synthesizing Comp.5-5:

SN38 (5.0 mg, 12.7 µmol, 1.0 eq) and bis(p-nitrophenyl) carbonate (3.8 mg, 12.7 µmol, 1.0 eq) were dissolved in 5 mL of dichloromethane. After three hours of stirring, the solvent was completely evaporated, and 2 mL of N,N-dimethylformamide was added to make an SN38-PNP solution.

Comp.5-4 (9 mg, 11.5 µmol, 1.0 eq) was dissolved in 2 mL of tetrahydrofuran, and 200 µL of an aqueous lithium hydroxide solution (0.3 mol/mL, 60 µmol, 5.2 eq) was added at -5 °C. The mixture was left to react at that temperature for half an hour, and TLC monitoring showed that no starting material remained. Dilute hydrochloric acid was added to adjust the pH to 3-4, and the solvent was removed by rotary evaporation. 2 mL of N,N-dimethylformamide was added, and the mixture was stirred until the residue dissolved. The SN38-PNP solution was added, and the mixture was stirred at 25 °C for four hours. The reaction mixture was purified by preparative liquid chromatography to give comp.5-5 as a yellow solid (10.4 mg).
Mobile phase A: acetonitrile (0.1% trifluoroacetic acid); mobile phase B: water (0.1% trifluoroacetic acid).
Structural characterization: LC-MS: 1059.4 [M].

### General steps for synthesizing Comp.5:

Comp.5-5 (10.4 mg, 9.8 µmol, 1.0 eq) was dissolved in 2 mL of N,N-dimethylformamide, and the solution was stirred at 25 °C. N-(2-Aminoethyl)maleimide hydrochloride (4.0 mg, 22.6 µmol, 2.3 eq), benzotriazole-N,N,N',N'-tetramethyluronium hexafluorophosphate (4.5 mg, 12.0 µmol, 1.2 eq), and diisopropyl-ethylamine (5 µL) were sequentially added. The mixture was stirred at 25 °C for 1.5 h, and HPLC monitoring showed that no starting material comp.5-5 remained. The reaction mixture was purified by preparative liquid chromatography to give comp.5 as a yellow solid (5.7 mg).
Mobile phase A: acetonitrile (0.1% trifluoroacetic acid); mobile phase B: water (0.1% trifluoroacetic acid).
Structural characterization: LC-MS: 1081.4 [M].

### Synthesis of Comp6

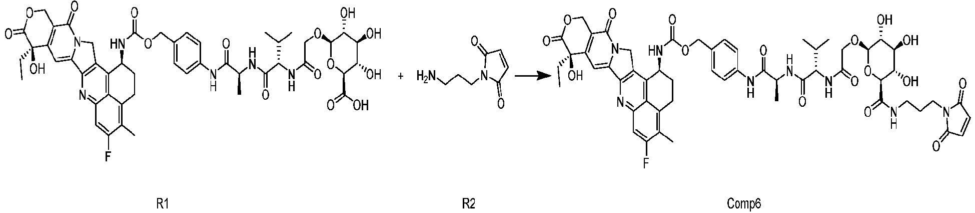

Compound R1 (19 mg, 19.2 µmol) was dissolved in DMF (3.0 mL), and commercially available compound R2 (5.5 mg, 28.8 µmol), TBTU (12.3 mg, 38.4 µmol), and DIEA (13.4 µL, 76.8 µmol) were sequentially added to the solution. The reaction mixture was left to react at 25 °C for 1 h, and HPLC monitoring showed that R1 had completely reacted. The reaction mixture was filtered to remove insoluble substances and purified by preparative HPLC, and the resulting solution was lyophilized to give Comp6. Comp6 was a yellow solid; MS: 1125.1.

### Synthesis of Comp7

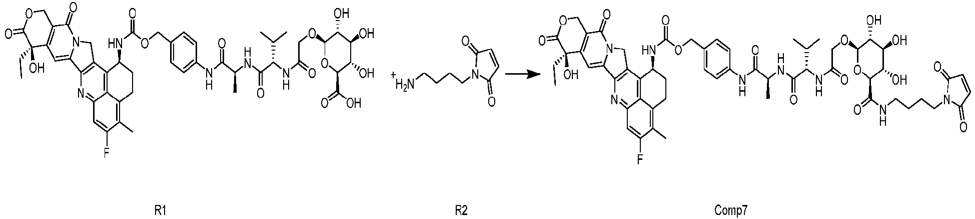

Compound R1 (19 mg, 19.2 µmol) was dissolved in DMF (3.5 mL), and commercially available compound R2 (5.9 mg, 28.8 µmol), TBTU (12.3 mg, 38.4 µmol), and DIEA (13.4 µL, 76.8 µmol) were sequentially added to the solution. The reaction mixture was left to react at 25 °C for 1 h, and HPLC monitoring showed that R1 had completely reacted. The reaction mixture was filtered to remove insoluble substances and purified by preparative HPLC, and the resulting solution was lyophilized to give Comp7. Comp7 was a yellow solid; MS: 1139.2.

### Synthesis of Comp8

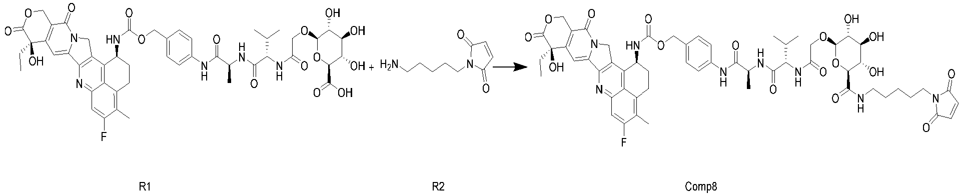

Compound R1 (19 mg, 19.2 µmol) was dissolved in DMF (4.0 mL), and commercially available compound R2 (8.5 mg, 28.8 µmol), TBTU (12.3 mg, 38.4 µmol), and DIEA (13.4 µL, 76.8 µmol) were sequentially added to the solution. The reaction mixture was left to react at 25 °C for 1 h, and HPLC monitoring showed that R1 had completely reacted. The reaction mixture was filtered to remove insoluble substances and purified by preparative HPLC, and the resulting solution was lyophilized to give Comp8. Comp8 was a yellow solid; MS: 1153.2.

### Synthesis of Comp9

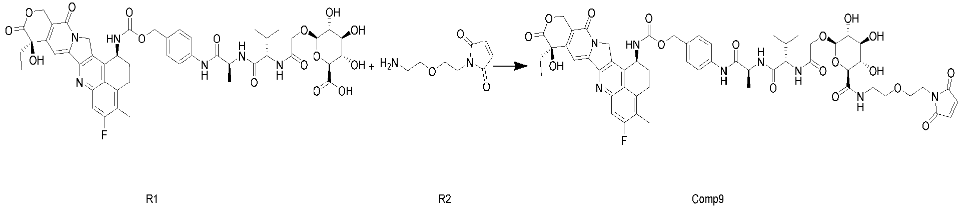

Compound R1 (19 mg, 19.2 µmol) was dissolved in DMF (3.0 mL), and commercially available compound R2 (8.5 mg, 28.8 µmol), TBTU (12.3 mg, 38.4 µmol), and DIEA (13.4 µL, 76.8 µmol) were sequentially added to the solution. The reaction mixture was left to react at 25 °C for 1 h, and HPLC monitoring showed that R1 had completely reacted. The reaction mixture was filtered to remove insoluble substances and purified by preparative HPLC, and the resulting solution was lyophilized to give Comp9. Comp9 was a yellow solid; MS: 1155.2.

### Example 2: Preparation and Effect Evaluation of ADCs

### CPD-ADC preparation

The anti-TROP2 antibody hRS7 (at a concentration of 13.6 mg/mL) was exchanged into a 40 mM PB/2.0 mM EDTA (pH 7.0) buffer using a tangential flow ultrafiltration system, and 1.8-2.2 equivalents of TCEP were added. The mixture was stirred at 25 °C for 2-4 h to partially break the interchain disulfide bonds of the antibody. The linker-drug conjugates CPD1, CPD2, CPD3, and GGFG-Dxd, prepared in Example 1, were each dissolved in DMSO and slowly added to the reduced antibody solution at 5-8 equivalents, and additional DMSO was added to adjust the final concentration to 5-20% (v/v). The mixture was stirred at 25 °C for 1-4 h. After the reaction was complete, the sample was filtered through a 0.22 µm membrane. Unconjugated small molecules were removed by purification using a tangential flow ultrafiltration system, with a 20 mM L-His solution (pH 5.5) as the buffer. The final products were stored in a freezer at -80 °C. The DAR values were determined using hydrophobic interaction chromatography, and the results are shown in Table 1.

**Table 1: DAR value results**

| ADC No. | Antibody | Linker-drug conjugate | DAR |
|---|---|---|---|
| ADC01 | Datopotamab | GGFG-Dxd | 3.73 |
| ADC02 | hRS7 | GGFG-Dxd | 3.69 |
| ADC03 | hRS7 | CPD1 (compound of formula D) | 3.82 |
| ADC04 | hRS7 | CPD2 (compound of formula B) | 3.92 |
| ADC05 | hRS7 | CPD3 (compound of formula E) | 3.99 |
| ADC06 | Anti-KLH hIgG1 | GGFG-Dxd | 3.82 |
| ADC07 | Anti-KLH hIgG1 | CPD1 (compound of formula D) | 3.86 |

The structure of GGFG-Dxd is shown below: 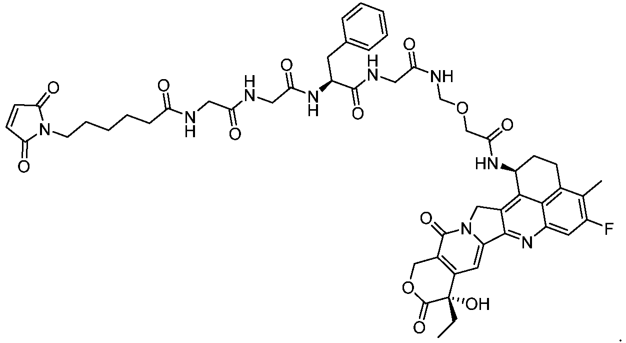
hRS7-light chain (SEQ ID NO: 1) 
DIQLTQSPSSLSASVGDRVSITCKASQDVSIAVAWYQQKPGKAPKLLIYSASYRYTGVPDR
FSGSGSGTDFTLTISSLQPEDFAVYYCQQHYITPLTFGAGTKVEIKRTVAAPSVFIFPPSDEQ
LKSGTASVVCLLNNFYPREAKVQWKVDNALQSGNSQESVTEQDSKDSTYSLSSTLTLSK
ADYEKHKVYACEVTHQGLSSPVTKSFNRGEC.
hRS7-heavy chain (SEQ ID NO: 2) 
QVQLQQSGSELKKPGASVKVSCKASGYTFTNYGMNWVKQAPGQGLKWMGWINTYTGE
PTYTDDFKGRFAFSLDTSVSTAYLQISSLKADDTAVYFCARGGFGSSYWYFDVWGQGSL
VTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTL
 
PPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPGK.
Datopotamab-light chain (SEQ ID NO: 3) 
DIQMTQSPSSLSASVGDRVTITCKASQDVSTAVAWYQQKPGKAPKLLIYSASYRYTGVPS
RFSGSGSGTDFTLTISSLQPEDFAVYYCQQHYITPLTFGQGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC.
Datopotamab-heavy chain (SEQ ID NO: 4)

QVQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQGTL
VTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPGK.

### Effect Example 1: In Vitro Cell Viability Assay

A431 cells highly expressing TROP2 were selected as the cell strain for this *in vitro* viability assay to investigate the dose-response relationships of the ADCs' cell-killing effects. The plating density for each type of cells was initially set to 10³-10⁵ cells/well, and after 1-7 days, cytotoxic activity assays were performed. For the sample concentrations, after the antibody conjugate drugs prepared in Example 2 were added, the final concentration was set to 50 µg/mL as the starting concentration, and a series of 8 concentrations ranging from 50 µg/mL to 0.00064 µg/mL were designed (2- to 10-fold dilution). Killing (or inhibition) was measured using CellTiter-Glo@Luminescent Cell Viability Assay chemiluminescence staining on days 1-7, and IC₅₀ values were calculated. The results of the *in vitro* cell viability assay are shown in Table 2.

**Table 2: The results of the in vitro cell viability assay**

| ADC No. | A431 IC₅₀/µg/mL |
|---|---|
| ADC01 | 0.110 |
| ADC02 | 0.057 |
| ADC03 | 0.074 |
| ADC04 | 0.048 |
| ADC05 | 0.024 |

### Effect Example 2

### In vivo evaluation 1

Female Balb/c nude mice, 6-8 weeks old, were subcutaneously injected in the dorsal neck region with 5 × 10⁶ human squamous lung carcinoma cells (EBC-1) suspended in 100 µL of PBS. Once the mean tumor volume reached approximately 100 mm³ (on day 11 post-inoculation), 48 nude mice were randomly divided into 8 groups of 6 according to tumor size, and each mouse was given one dose via tail vein injection:
group 1 was a blank control group;
group 2 was ADC07 (10 mg/kg);
group 3 was ADC01 (2 mg/kg);
group 4 was ADC01 (10 mg/kg);
group 5 was ADC02 (10 mg/kg);
group 6 was ADC03 (2 mg/kg);
group 7 was ADC03 (10 mg/kg);
group 8 was ADC04 (10 mg/kg).

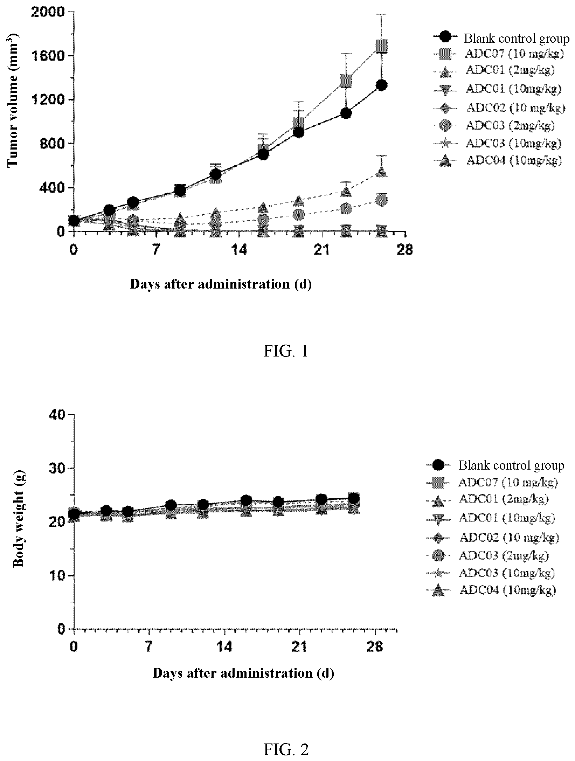

The body weight and tumor volume of the experimental animals were measured twice weekly, and the survival status of the animals was monitored during the experiment. The experimental results (FIGs. 1 and 2) show that ADC01, ADC02, ADC03, and ADC04 exhibited anti-tumor activity compared to the control group. Among these, ADC03 and ADC04 showed relatively good *in vivo* anti-tumor activity; in addition, no mortality or weight loss was observed in any of the experimental mice, indicating that ADC03 and ADC04 have very good safety profiles.

### In vivo evaluation 2

Female Balb/c nude mice, 6-8 weeks old, were subcutaneously injected in the dorsal neck region with 5 × 10⁶ human lung adenocarcinoma cells (Calu-3) suspended in 100 µL of PBS. Once the mean tumor volume reached approximately 100 mm³ (on day 4 post-inoculation), 48 nude mice were randomly divided into 8 groups of 6 according to tumor size, and each mouse was given one dose via tail vein injection:
group 1 was a blank control group;
group 2 was ADC07 (8 mg/kg);
group 3 was ADC01 (2 mg/kg);
group 4 was ADC01 (8 mg/kg);
group 5 was ADC02 (8 mg/kg);
group 6 was ADC03 (2 mg/kg);
group 7 was ADC03 (8 mg/kg);
group 8 was ADC04 (8 mg/kg).

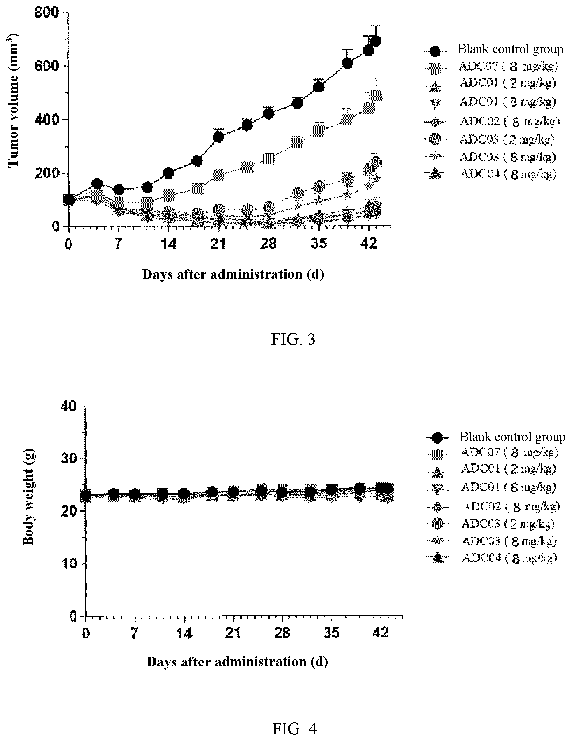

The body weight and tumor volume of the experimental animals were measured twice weekly, and the survival status of the animals was monitored during the experiment. The experimental results (FIGs. 3 and 4) show that, compared to the control group, ADC01, ADC02, ADC03, ADC04, and ADC07 all exhibited anti-tumor activity. Among these, ADC03 and ADC04 showed relatively good *in vivo* anti-tumor activity; in addition, no mortality or weight loss was observed in any of the experimental mice, indicating that ADC03 and ADC04 have very good safety profiles.

### Example 3: Preparation and Effect Evaluation of Drug-Conjugated Anti-Trop2 Antibody ADCs

Drug source: Deruxtecan (connector-cytotoxin, CAS No. 1599440-13-7) and CL2A-SN-38 were purchased from Shanghai Haoyuan Chemexpress Co., Ltd.

The anti-Trop2 antibody used was sacituzumab (hRS7 for short, prepared in-house by the applicant).

The anti-Trop2 antibody was conjugated to the connector-cytotoxins to prepare conjugates using the following method:
Step 1: TCEP (tris(2-carboxyethyl)phosphine, 2.1 eq.) was added to the antibody in a pH 6.5, 50 mM PB/2 mM EDTA buffer, and the mixture was stirred at 25 °C for 4 h to give a reduced antibody solution. Step 2: DMA (N,N-dimethylacetamide) and an appropriate amount of cytotoxin CPD2 or the like (e.g., 6.0 eq. or 5.5 eq.) were added to the reduced antibody solution (the total DMA proportion was 10%), and the mixture was left to react at 25 °C for 1 h. The reaction mixture was exchanged into a buffer solution (e.g., 20 mM L-Histidine/pH 5.5, or 50 mM MES/pH 6.0, or 20 mM L-Histidine/pH 5.5). After filtration, the antibody-drug conjugate (ADC) of the present disclosure was obtained. The ADC was stored at -60 °C to -90 °C. m = 0-8. Free small molecules were detected using reverse-phase high performance liquid chromatography. The DAR was determined using hydrophobic interaction chromatography. The purity was measured using size exclusion chromatography.

### In vitro killing experiment of anti-Trop2 antibody ADCs

After BxPC-3 (ATCC#CRL-1555) cells, which express Trop2, were co-cultured for 6 days with serially diluted DXD (deruxtecan, #HY-13631E, MCE)-conjugated sacituzumab (hereinafter referred to as hRS7-DXD for short, synthesized in-house by the applicant); DXD-conjugated control IgG1 protein (antiKLH-DXD); hRS7-CPD1, hRS7-CPD2, hRS7-CPD3, hRS7-CPD4, hRS7-CPD5, hRS7-CPD6, hRS7-CPT004, hRS7-CPT005, hRS7-CPT006, and hRS7-CPT009, obtained after conjugation to the linker-drug conjugates of the present disclosure; hRS7-SN38, obtained after conjugation to comp.5-linked SN38; hRS7-CL2A-SN38, obtained after conjugation to CL2A-SN38 (MCE), and conjugated control IgG1 protein (antiKLH-CPD1), the number of viable cells was determined using cell counting lite (Vazyme #DD1102-01), and proliferation inhibition rates (proliferation inhibition rate (%) = 100 × (number of viable cells in untreated group - number of viable cells in treated group) / (number of viable cells in untreated group - number of viable cells in solution control group)%) were calculated to determine the *in vitro* killing activity of the candidate molecules.

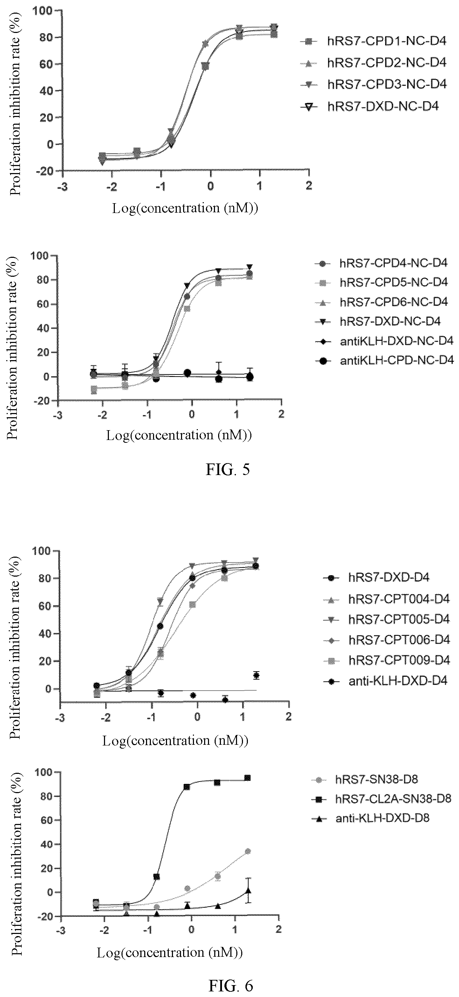

The results are shown in FIGs. 5 and 6 and Tables 3 and 4.

**Table 3: The ability of the candidate molecules and the negative control ADCs to inhibit BxPC-3 cell proliferation (EC₅₀)**

| Sample | EC₅₀(nM) | Relative activity (%) |
|---|---|---|
| hRS7-CPD1-NC-D4 | 0.4681 | 102.9 |
| hRS7-CPD2-NC-D4 | 0.3271 | 147.3 |
| hRS7-CPD3-NC-D4 | 0.3091 | 155.8 |
| hRS7-CPD4-NC-D4 | 0.4243 | 89.6 |
| hRS7-CPD5-NC-D4 | 0.4779 | 79.6 |
| hRS7-CPD6-NC-D4 | 0.3412 | 111.4 |
| hRS7-DXD-NC-D4 | 0.3802 | 100 |
| antiKLH-DXD-NC-D4 | NA | NA |
| antiKLH-CPD-NC-D4 | NA | NA |

| | | |
|---|---|---|
| Note: NA indicates that no killing was observed and calculation could not be performed. | | |

As shown in FIG. 5 and Table 3, compared to anti-KLH-CPD1, hRS7-CPD1/hRS7-CPD2/hRS7-CPD3/hRS7-CPD4/hRS7-CPD5/hRS7-CPD6 exhibited specific proliferation-inhibiting effects on BxPC-3 cells, and hRS7-DXD also exhibited a specific proliferation-inhibiting effect on BxPC-3; the CPD1, CPD2, CPD3, CPD4, CPD5, and CPD6 conjugates were comparable in proliferation-inhibiting activity to the DXD conjugate.

**Table 4: The ability of the candidate molecules and the negative control ADCs to inhibit BxPC-3 cell proliferation (EC₅₀)**

| Sample | EC50(nM) | Relative activity (%) |
|---|---|---|
| hRS7-DXD-D4 | 0.1562 | 100 |
| hRS7-CPT004-D4 | 0.1436 | 108.8 |
| hRS7-CPT005-D4 | 0.1009 | 154.8 |
| hRS7-CPT006-D4 | 0.2587 | 60.4 |
| hRS7-CPT009-D4 | 0.3526 | 50.7 |
| anti-KLH-DXD-D4 | NA | NA |
| hRS7-SN38-D8 | 6.462 | NA |
| hRS7-CL2A-SN38-D8 | 0.2585 | 100 |
| anti-KLH-DXD-D8 | NA | NA |

| | | |
|---|---|---|
| Note: NA indicates that no killing was observed and calculation could not be performed. | | |

As shown in Table 4 and FIG. 6, hRS7-CPT004, hRS7-CPT005, hRS7-CPT006, and hRS7-CPT009 exhibited significant proliferation-inhibiting effects on BxPC-3 cells and were comparable in activity to hRS7-DXD. hRS7-SN38 also exhibited a proliferation-inhibiting effect on BxPC-3 cells.

### Example 4: Preparation and Effect Evaluation of Comp6, etc.-Conjugated Anti-Trop2 Antibody ADCs

Drug source: Comp6, comp7, comp8, and comp9 were prepared in-house by the applicant.

The anti-Trop2 antibody used was sacituzumab (hRS7 for short, prepared in-house by the applicant).

### ADC preparation

The anti-Trop2 antibody was conjugated to the connector-cytotoxins to prepare conjugates using the following method:
Step 1: TCEP (tris(2-carboxyethyl)phosphine, 2.4 eq.) was added to the antibody in a pH 6.5, 50 mM PBS/2 mM EDTA buffer, and the mixture was stirred at 25 °C for 3.5 h to give a reduced antibody solution.
Step 2: DMA (N,N-dimethylacetamide) and comp6, comp7, comp8, or comp9 (7.0 eq.) were added to the reduced antibody solution (the total DMA proportion was 20%), and the mixture was left to react at 25 °C for 1 h. The reaction mixture was exchanged into a buffer solution (20 mM Histidine/pH 5.5). After filtration, the antibody-drug conjugate (ADC) of the present disclosure was obtained. The ADC was stored at -60 °C to -90 °C. m = 0-8. Free small molecules were detected using reverse-phase high performance liquid chromatography. The DAR was determined using PLRP chromatography. The purity was measured using size exclusion chromatography.

The DAR values, determined by PLRP chromatography, of the ADCs of comp6, comp7, comp8, and comp9 were 3.49, 3.56, 3.52, and 3.54, respectively.

### In vitro killing experiment

After BxPC-3 (ATCC#CRL-1555) cells, which express Trop2, were co-cultured for 6 days with serially diluted DXD (deruxtecan, #HY-13631E, MCE)-conjugated sacituzumab ADC (hRS7-DXD for short); DXD-conjugated control IgG1 protein (antiKLH-DXD for short); and comp6, 7, 8, or 9-conjugated sacituzumab ADC, designated hRS7-C6, hRS7-C7, hRS7-C8, or hRS7-C9, respectively, the number of viable cells was determined using cell counting lite (Vazyme #DD1102-01), and proliferation inhibition rates (proliferation inhibition rate (%) = 100 × (number of viable cells in untreated group - number of viable cells in treated group) / (number of viable cells in untreated group - number of viable cells in solution control group)%) were calculated to determine the *in vitro* killing activity of the candidate molecules.

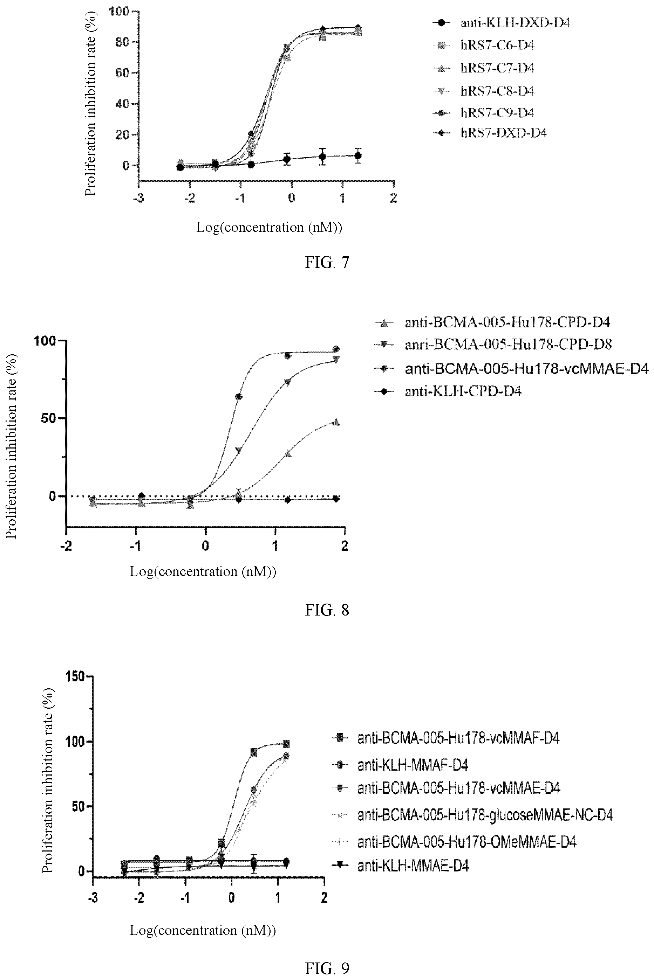

As shown in Table 5 and FIG. 7, hRS7-C6, hRS7-C7, hRS7-C8, and hRS7-C9 exhibited significant proliferation-inhibiting effects on BxPC-3 cells and were comparable in activity to hRS7-DXD.

**Table 5: The ability of the candidate molecules and the negative control ADCs to inhibit BxPC-3 cell proliferation (EC₅₀)**

| Sample | EC₅₀(nM) | Relative activity (%) |
|---|---|---|
| hRS7-DXD-D4 | 0.3137/0.4071 | 100 |
| hRS7-C6-D4 | 0.3932 | 80 |
| hRS7-C7-D4 | 0.3046 | 103 |
| hRS7-C8-D4 | 0.3231 | 97 |
| hRS7-C9-D4 | 0.3769 | 108 |
| anti-KLH-DXD-D4 | NA | NA |

| | | |
|---|---|---|
| Note: NA indicates that no killing was observed and calculation could not be performed. | | |

### Example 5: Anti-BCMA Antibody ADCs

An anti-BCMA antibody was conjugated to the connector-cytotoxin vc-MMAE, CPD, or the like to prepare a conjugate.

Drug sources: vc-MMAE, vc-MMAF, and deruxtecan (DXD) were purchased from Shanghai Haoyuan Chemexpress Co., Ltd.; Linker-MMAE derivative 1, Linker-MMAE derivative 2, and CPD (i.e., CPD1) were prepared by WuXi AppTec (Shanghai) Co., Ltd. on commission.

The anti-BCMA antibody used was anti-BCMA-005-Hu178, prepared in-house by the applicant, and its heavy and light chain amino acid sequences are shown below:
anti-BCMA-005-Hu178 heavy chain (SEQ ID NO: 5): 
EVQLVESGGGLVKPGGSLRLSCAASGFTFSNFDMAWVRQAPGKGLEWVASIVTRGDDT
YYRDSVRGRFTISRDNAKNTQYLQMNSLRAEDTAVYYCARHDYGGPSGWFGYWGQGT
LVTVSSASTKGPSVFPLAPSSKSTSGGTAALGCLVKDYFPEPVTVSWNSGALTSGVHTFP
 
AVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGK.
anti-BCMA-005-Hu178 light chain (SEQ ID NO: 6): 
DIQMTQSPSSLSASVGDRVTITCRASQGFSNNLNWYQQKPGKTPKPLIYYTSNLHSGVPSR
FSGSGSGTDYSLTISSLQPEDFATYYCQQDASFPPTFGQGTKLEIKRtvaapsvfifppsdeqlksgtas
vvcllnnfypreakvqwkvdnalqsgnsqesvteqdskdstyslsstltlskadyekhkvyacevthqglsspvtksfnrgec.
 The control IgG1 protein was prepared in-house by the applicant.

### ADC preparation

Step 1: TCEP (tris(2-carboxyethyl)phosphine, 2.4 eq.) was added to the antibody in a pH 7.0, 40 mM PB buffer, and the mixture was stirred at 37 °C for 4.0 h to give a reduced antibody solution. Step 2: DMA (N,N-dimethylacetamide) and vc-MMAE, vc-MMAF, deruxtecan (DXD), Linker-MMAE derivative 1, Linker-MMAE derivative 2, CPD (i.e., CPD1), or the like (8.0 eq.) were added to the reduced antibody solution (the total DMA proportion was 10%), and the mixture was left to react at 25 °C for 1 h. The reaction mixture was exchanged into a buffer solution (20 mM L-His/pH 5.5). After filtration, the antibody-drug conjugate (ADC) of the present disclosure was obtained. The ADC was stored at -60 °C to -90 °C. m = 0-8. Free small molecules were detected using reverse-phase high performance liquid chromatography. The DAR was determined using HIC chromatography. The purity was measured using size exclusion chromatography.

Results: The HIC-DAR value of conjugating vc-MMAE to the antibody was 3.78; the HIC-DAR value of conjugating vc-MMAF was 3.86; the HIC-DAR value of conjugating Linker-MMAE derivative 1 was 3.73; the HIC-DAR value of conjugating Linker-MMAE derivative 2 was 3.79; the HIC-DAR value of anti-BCMA-CPD 1-D4 was 4.24; the HIC-DAR value of anti-BCMA-CPD1-D8 was 7.73; the HIC-DAR value of conjugating the connector-cytotoxin DXD to the anti-KLH antibody was 7.34.

### In vitro killing experiment

After MM.1S cells, which express BCMA, were co-cultured for 4 days with serially diluted vcMMAE (MCE)-conjugated anti-BCMA mAb (anti-BCMA-vcMMAE for short); vcMMAF-conjugated anti-BCMA mAb (anti-BCMA-vcMMAF for short); Linker-MMAE derivative 1, Linker-MMAE derivative 2, or CPD-conjugated anti-BCMA (referred to as anti-BCMA-Linker-MMAE derivative 1, anti-BCMA-Linker-MMAE derivative 2, anti-BCMA-CPD-D4, or anti-BCMA-CPD-D8, respectively, for short); CPD-conjugated control IgG1 protein (anti-KLH-CPD-D4 for short); and MMAF or MMAE-conjugated control IgG1 protein (referred to as anti-KLH-MMAF-D4 or anti-KLH-MMAE-D4, respectively, for short), the number of viable cells was determined using cell counting lite (Vazyme #DD1102-01), and proliferation inhibition rates (proliferation inhibition rate %, proliferation inhibition rate = 100 × (number of viable cells in untreated group - number of viable cells in treated group) / (number of viable cells in untreated group - number of viable cells in solution control group)%) were calculated to determine the *in vitro* killing activity of the candidate molecules.

The results are shown in Tables 6 and 7 and FIGs. 8 and 9.

**Table 6: The ability of the candidate molecules and the negative control ADCs to inhibit MM.1S cell proliferation (EC₅₀)**

| Sample | EC₅₀(nM) |
|---|---|
| anti-BCMA-005-Hu178-CPD-D4 | 12.25 |
| anri-BCMA-005-Hu178-CPD-D8 | 4.558 |
| anti-KLH-CPD-D4 | ~ 39.56 |
| anti-BCMA-005-Hu178-vcMMAE-D4 | 2.303 |

**Table 7: The ability of the candidate molecules and the negative control ADCs to inhibit MM.1 S cell proliferation (EC50)**

| Sample | EC₅₀(nM) | Relative activity (%) |
|---|---|---|
| anti-KLH-MMAF-D4 | ~ 0.003792 | NA |
| anti-BCMA-005-Hu178-vcMMAF-D4 | 1.122 | 166 |
| anti-KLH-MMAE-D4 | 0.01344 | NA |
| anti-BCMA-005-Hu178-vcMMAE-D4 | 1.866 | 100 |
| anti-BCMA-005-Hu178-glucoseMMAE-NC-D4 | 2.149 | 86.8 |
| anti-BCMA-005-Hu178-OMeMMAE-D4 | 2.419 | 77.1 |

| | | |
|---|---|---|
| Note: NA indicates that no killing was observed and calculation could not be performed. | | |

As shown in Table 6, Table 7, FIG. 8, and FIG. 9, anti-BCMA-vcMMAE, anti-BCMA-vcMMAF, anti-BCMA-Linker-MMAE derivative 1, anti-BCMA-Linker-MMAE derivative 2, anti-BCMA-CPD-D4, and anti-BCMA-CPD-D8 exhibited significant proliferation-inhibiting effects on MM.1S cells. The anti-BCMA-Linker-MMAE derivative 1 was anti-BCMA-005-Hu178-glucoseMMAE-NC-D4; the anti-BCMA-Linker-MMAE derivative 2 was anti-BCMA-005-Hu178-OMeMMAE-D4; the anti-BCMA-vcMMAE was anti-BCMA-005-Hu178-vcMMAE; the anti-BCMA-vcMMAF was anti-BCMA-005-Hu178-vcMMAF.

## Claims

1. A linker, having a structure represented by formula A: 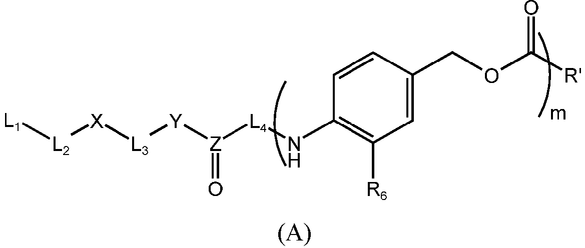 wherein:
L₁ is a functional group capable of reacting with mercapto;
L₂ and Y are each independently selected from -(CH₂)ₚ-, -(OCH₂CH₂)ₚ-, and -(CH₂CH₂O)ₚ-;
L₃ is a hydrophilic group;
L₄ is an amino acid group;
R' is -OR or -NR"-C₁-C₆ alkylene-NR"R"';
R" and R‴ are each independently H and C₁-C₆ alkyl;
R is selected from H and phenyl optionally substituted with one or more nitro groups;
R₆ is selected from H, C₁-C₆ alkoxy, -(OCH₂CH₂)ₚO-C₁-C₆ alkyl, and -(CH₂CH₂O)ₚ-C₁-C₆ alkyl;
X is selected from -NH-, -NH(CH₂)ₙC(=O)-, -C(=O)(CH₂)ₙNH-, and -(CH₂)ₙC(=O)-;
Z is selected from C and S;
m is selected from 0 and 1;
n is selected from 0, 1, and 2;
p is selected from an integer from 1 to 10.

2. The linker according to claim 1, having a structure represented by formula I: 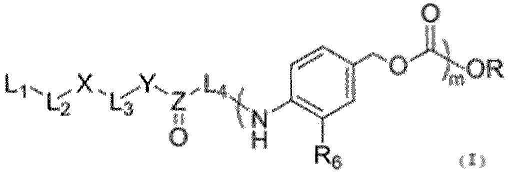 wherein:
L₁ is a functional group capable of reacting with mercapto;
L₂ and Y are each independently selected from -(CH₂)ₚ-, -(OCH₂CH₂)ₚ-, and -(CH₂CH₂O)ₚ-;
L₃ is a hydrophilic group;
L₄ is an amino acid group;
R is selected from H and phenyl optionally substituted with one or more nitro groups;
R₆ is selected from H, C₁-C₆ alkoxy, -(OCH₂CH₂)ₚO-C₁-C₆ alkyl, and -(CH₂CH₂O)ₚ-C₁-C₆ alkyl;
X is selected from -NH-, -NH(CH₂)ₙC(=O)-, -C(=O)(CH₂)ₙNH-, and -(CH₂)ₙC(=O)-;
Z is selected from C and S;
m is selected from 0 and 1;
n is selected from 0, 1, and 2;
p is selected from an integer from 1 to 10.

3. The linker according to claim 1 or 2, wherein L₁ is selected from maleimide, halogen, halogen-substituted functional groups (e.g., halogen-substituted aldehyde groups or halogen-substituted - S(O)₂-), aldehyde groups, alkenyl, alkynyl, alkanones, sulfonyl, silane, isocyanate groups, and norbornenyl.

4. The linker according to claim 3, wherein L₁ is selected from: 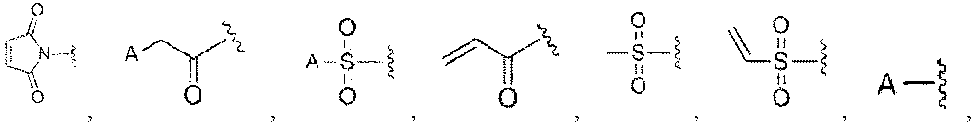 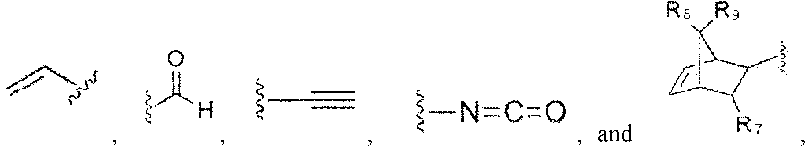 and derivatives thereof;
wherein:
R₇, R₈, and R₉ are each independently selected from H, halogen, C₁-C₆ alkyl, C₂-C₆ alkenyl, C₂-C₆ alkynyl, aryl, heteroaryl, C₃-C₉ cycloalkyl, C₃-C₉ heterocyclyl, -(OCH₂CH₂)ₚOCH₃, -NO₂, -CN,-SCN, -OR₁₀, -SR₁₀, -N(R₁₀)₂, -C(=O)R₁₀, -C(=O)OR₁₀, -C(=O)N(R₁₀)₂, -C(=S)OR₁₀,-C(=S)N(R₁₀)₂, -C(=S)SR₁₀, -NR₁₀(C=O)R₁₀, -NR₁₀(C=S)N(R₁₀)₂, -O(C=O)N(R₁₀)₂, -S(=O)₂R₁₀, and -S(=O)₂OR₁₀, wherein each R₁₀ is independently selected from H, C₁-C₆ alkyl, C₂-C₆ alkenyl, and C₂-C₆ alkynyl; p is an integer from 1 to 6;
A is halogen;
the wavy line indicates a position where L₁ is connected to L₂;
preferably, L₁ is:

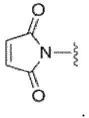

5. The linker according to any one of claims 1-4, wherein
L₂ is -(CH₂)ₚ-, and p is an integer from 1 to 4; preferably, L₂ is -CH₂CH₂- or -CH₂CH₂CH₂-; and/or
X is -NH- or -NH(CH₂)ₙC(=O)-; preferably, X is -NH- or -NHC(=O)-; and/or
Y is -(CH₂)ₚ-, and p is 1, 2, or 3; preferably, Y is methylene; and/or
Z is C.

6. The linker according to any one of claims 1-5, wherein L₃ is selected from divalent hydrophilic groups with two monovalent radical centers that result from removal of two hydrogen atoms from a monosaccharide, a disaccharide, and a five- or six-membered saturated heterocycle containing 1-2 nitrogen atoms and a derivative thereof;
preferably, the monosaccharide is selected from a triose, a tetrose, a pentose, a hexose, and a heptose;
preferably, the disaccharide is selected from maltose, sucrose, and lactose;
preferably, the five- or six-membered saturated heterocycle containing 1-2 nitrogen atoms or the derivative thereof is selected from piperazinyl, piperidinyl, and pyrrolidinyl.

7. The linker according to any one of claims 1-6, wherein L₃ is selected from divalent hydrophilic groups with two monovalent radical centers that result from removal of two hydrogen atoms from glucose, galactose, mannose, glucuronic acid, galactonic acid, mannuronic acid, N-acetylglucosamine, N-acetylgalactosamine, N-acetylmannosamine, and N-acetylmuramic acid.

8. The linker according to any one of claims 1-6, wherein L₃ is selected from: 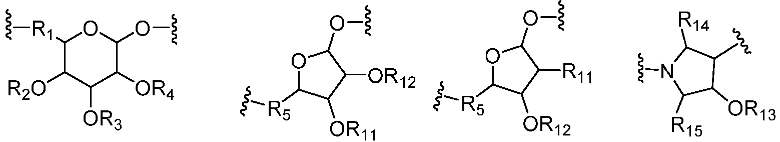 and 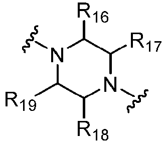 wherein in the formulas,
R₁ is selected from -O(CH₂)ₚ- and -C(=O)-, and p is an integer from 1 to 6, preferably 1, 2, 3, or 4;
R₅ is C₁-C₃ alkylene unsubstituted or substituted with one or more substituents selected from -OR₂₀ and -C(=O)OR₂₀-;
R₂, R₃, R₄, R₁₁, R₁₂, and R₁₃ are independently selected from H, C₁-C₆ alkyl, -(CH₂CH₂O)ₜ-C₁-C₆ alkyl, a sulfonic acid group, and a phosphoric acid group;
R₁₄ and R₁₅ are independently selected from H, -OR₂₀, and -CH₂R₂₀;
R₁₆, R₁₇, R₁₈, and R₁₉ are independently selected from H and -OR₂₀;
R₂₀ is selected from H, C₁-C₆ alkyl, -(CH₂CH₂O)ₜ-C₁-C₆ alkyl, a sulfonic acid group, and a phosphoric acid group;
t is an integer from 1 to 20;
the wavy lines indicate positions where L₃ is connected to X and Y.

9. The linker according to any one of claims 1-8, wherein
L₄ is selected from the following amino acid residues: valine, citrulline, alanine, glycine, phenylalanine, asparagine, glutamic acid, lysine, serine, threonine, cysteine, and tyrosine; the amino acid groups are optionally substituted with one or more -OR₂₀, wherein R₂₀ is selected from H, C₁-C₆ alkyl, -(CH₂CH₂O)ₜ-C₁-C₆ alkyl, a sulfonic acid group, and a phosphoric acid group, wherein t is an integer from 1 to 20; or
L₄ is selected from the following peptide fragments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine, and lysine-phenylalanine, preferably from the following peptide fragments: valine-citrulline (VC), valine-alanine (VA), and glycine-glycine-phenylalanine-glycine (GGFG); each of the peptide fragments is optionally substituted with one or more substituents selected from -OR₂₀ and -(OCH₂CH₂)ₚO-C₁-C₆ alkyl, wherein R₂₀ is selected from H, C₁-C₆ alkyl, -(CH₂CH₂O)ₜ-C₁-C₆ alkyl, a sulfonic acid group, and a phosphoric acid group, t is an integer from 1 to 20, and p is an integer from 1 to 6.

10. The linker according to claim 9, wherein L₄ is selected from:

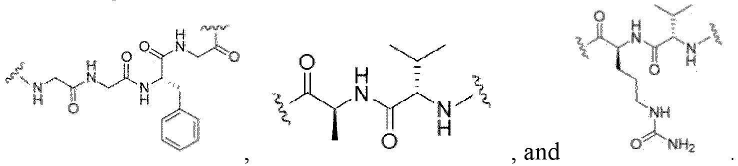

11. The linker according to any one of claims 1-10, wherein
R₆ is H, C₁-C₃ alkoxy, or -(OCH₂CH₂)ₚO-C₁-C₃ alkyl, wherein p is an integer from 1 to 6; preferably, R₆ is H, methoxy, or -(OCH₂CH₂)ₚOCH₃, wherein p is an integer from 1 to 6 or from 1 to 4; and/or
R is selected from H and phenyl optionally substituted with 1-3 nitro groups and is preferably H or 4-nitrophenyl.

12. The linker according to any one of claims 1-11, wherein the linker is selected from linkers represented by formulas II to XIV; in each of the formulas, R", R‴, R, L₂, X, L₃, R₂-R₄, R₆, R₁₁-R₁₃, and R₁₅ are as described in any one of claims 1-11.

13. The linker according to any one of claims 1-12, wherein the linker is selected from linkers represented by formulas 1 to 46.

14. A linker-drug conjugate, having a structural formula represented by formula XIII': 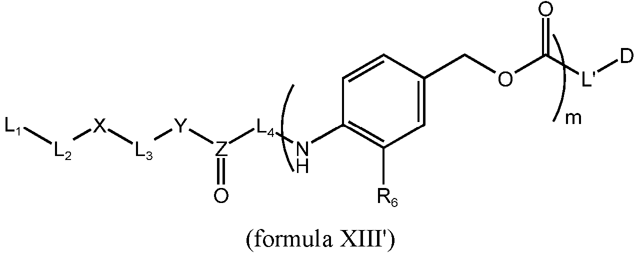 wherein in the formula, L₁, L₂, L₃, L₄, X, Y, Z, R₆, and m are as described in any one of claims 1-11; L' is a bond, -NR"-C₁-C₆ alkylene-NR"-CO-*, or -NH-CH₂-O-; D is a drug moiety; each R" is independently H and C₁-C₆ alkyl; * indicates a position where L' is connected to D; preferably, the structure other than moiety D in formula XIII' is as shown in claim 12 or 13.

15. The linker-drug conjugate according to claim 14, having a structure represented by formula XIII: 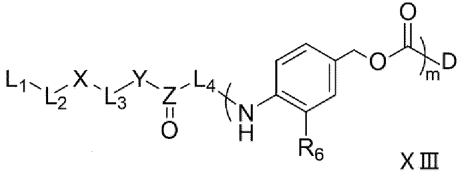 wherein L₁, L₂, L₃, L₄, X, Y, Z, R₆, and m are as described in any one of claims 1-11; D is a drug moiety; preferably, the structure other than moiety D in formula XIII is as defined in claim 12 or 13.

16. The linker-drug conjugate according to claim 14 or 15, wherein the drug moiety D is selected from drug moieties represented by formulas D-1 to D-9; in each of the formulas, the wavy line indicates a position where D is connected to the remainder of the linker-drug conjugate.

17. The linker-drug conjugate according to any one of claims 14-16, wherein the linker-drug conjugate is selected from CPD1, CPD2, CPD3, CPD4, CPD5, CPD6, CPT004, CPT005, CPT006, CPT008, CPT009, CPT010, MMAE Derivate, MMAE Derivate (o-OMe-PAB), Comp.5, Comp6, Comp7, Comp8, and Comp9.

18. An antibody-drug conjugate or a pharmaceutical composition thereof, wherein the antibody-drug conjugate has a structure represented by formula XIV:
Ab-(L-D)ₛ (formula XIV)
wherein L is the linker according to any one of claims 1-13; D is a drug; s represents an average number of L-D units conjugated to Ab, and s ranges from 1 to 8; preferably, s is 2-6; Ab is a targeting moiety; preferably, L-D is the linker-drug conjugate according to any one of claims 14-17.

19. The antibody-drug conjugate or the pharmaceutical composition thereof according to claim 18, wherein the antibody-drug conjugate has a structure represented by the following formula: 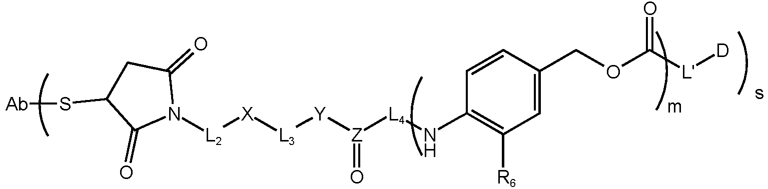 wherein L₂, X, L₃, Y, Z, L₄, R₆, and m are as described in any one of claims 1-11; L' is as described in claim 14; s represents an average number of L-D units conjugated to Ab, and s ranges from 1 to 8; preferably, s is 2-6; Ab is a targeting moiety; S represents a sulfur atom in a cysteine residue in the antibody.

20. The antibody-drug conjugate or the pharmaceutical composition thereof according to claim 18, wherein the antibody-drug conjugate has a structure of: 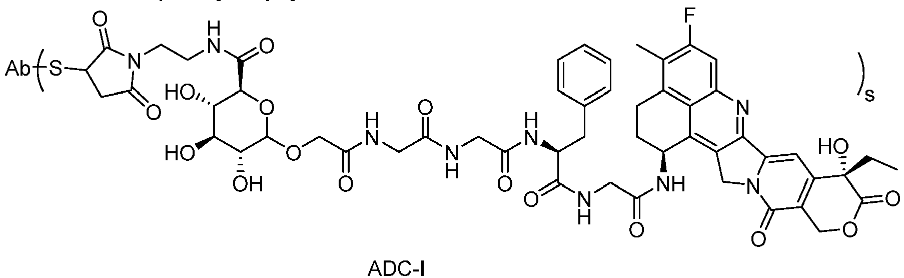 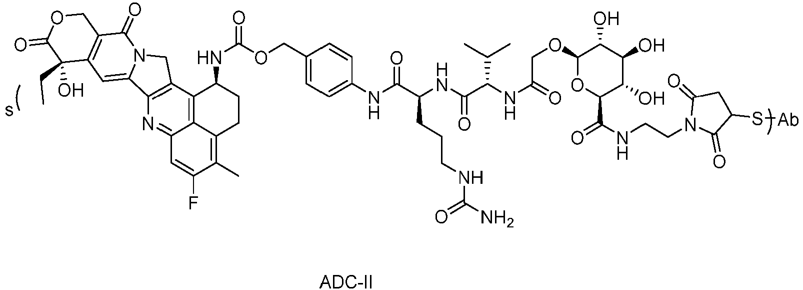 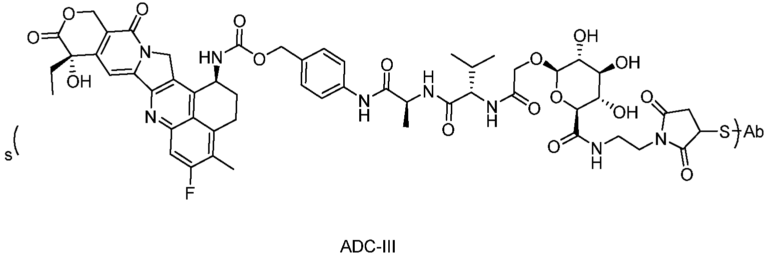 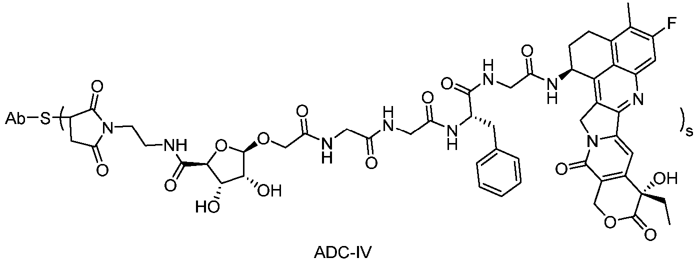 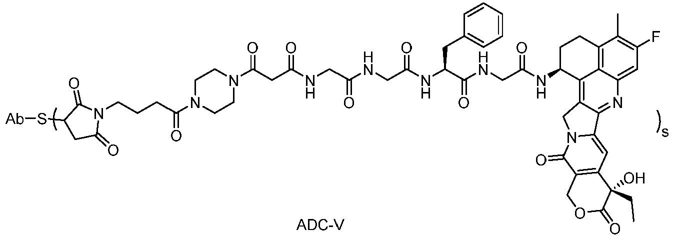 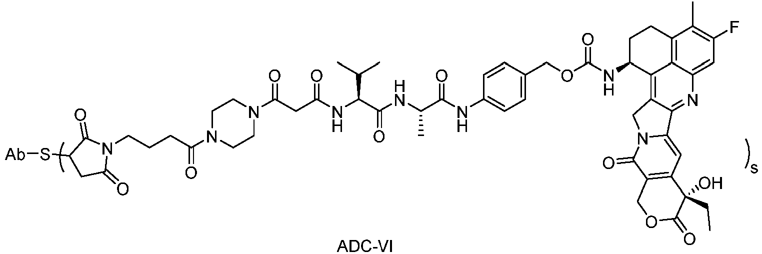 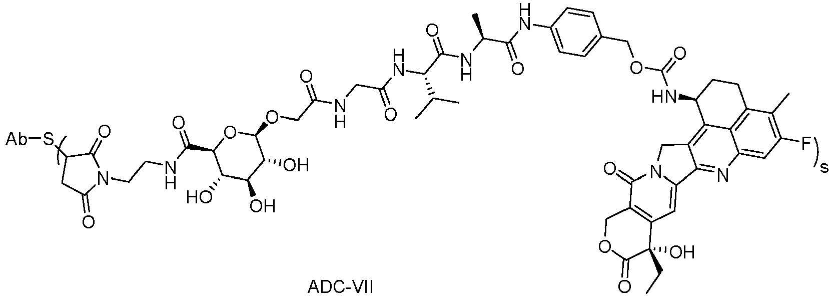 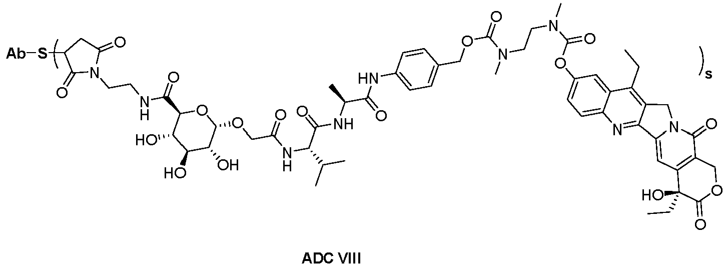 wherein in the formulas, s is 1-8.

21. A connector having a structure represented by the following formula, wherein the connector is used for connecting a drug to a targeting moiety to form a targeting moiety-drug conjugate:
-L₁-L₂-X-L₃-Y-Z(=O)-L₄-(L₅)ₘ-L₆-
wherein in the formula,
L₁ is selected from -(maleimido-3-yl-N)-, -C(=O)-, -(CH₂)ₙC(=O)-, -S(=O)-, -(CH₂)ₙS(=O)-, -CH₂-(CH₂)ₙ-, and -CH=CH-;
L₂ and Y are each independently selected from -(CH₂)ₚ-, -(OCH₂CH₂)ₚ-, and -(CH₂CH₂O)ₚ-;
L₃ is a hydrophilic group;
L₄ is an amino acid group;
L₅ is: 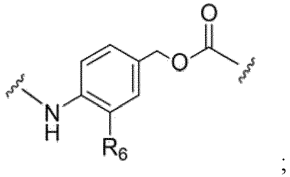
R₆ is selected from H, C₁-C₆ alkoxy, -(OCH₂CH₂)ₚO-C₁-C₆ alkyl, and -(CH₂CH₂O)ₚ-C₁-C₆ alkyl;
L₆ is a bond, -NR"-C₁-C₆ alkylene-NR"-, or -NR"-C₁-C₆ alkylene-NR"-CO-;
each R" is independently H or C₁-C₆ alkyl; preferably, each R" is independently H, methyl, or ethyl; more preferably, each R" is methyl;
X is selected from -NH-, -NH(CH₂)ₙC(=O)-, -C(=O)(CH₂)ₙNH-, and -(CH₂)ₙC(=O)-;
Z is selected from C and S;
n is selected from 0, 1, and 2;
p is selected from an integer from 1 to 10;
m is selected from 0 and 1;
wherein the L₁ is used for connecting to the targeting moiety, and the other end of the connector is used for connecting to the drug;
wherein when m is 0, the drug is connected to the carbonyl end (C(=O)) of L₄; when m is 1, the drug is connected to the carbonyl end (C(=O)) of L₅, and L₄ is connected to the amino end (NH) of L₅;
wherein the -(maleimido-3-yl-N)- is a structure represented by the following formula and is connected to the targeting moiety via locant 3,

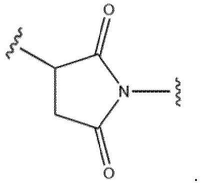

22. The connector according to claim 21, wherein
L₁ is -(maleimido-3-yl-N)-; and/or
L₂ is -(CH₂)ₚ-, and p is an integer from 1 to 4; preferably, L₂ is -CH₂CH₂- or -CH₂CH₂CH₂-; and/or
X is -NH- or -NH(CH₂)ₙC(=O)-; preferably, X is -NH- or -NHC(=O)-; and/or
Y is -(CH₂)ₚ-, and p is 1, 2, or 3; preferably, Y is methylene; and/or
Z is C.

23. The connector according to claim 21 or 22, wherein L₃ is selected from divalent hydrophilic groups with two monovalent radical centers that result from removal of two hydrogen atoms from a monosaccharide, a disaccharide, and a five- or six-membered saturated heterocycle containing 1-2 nitrogen atoms and a derivative thereof; preferably, the monosaccharide is selected from at least one of a triose, a tetrose, a pentose, a hexose, and a heptose; the disaccharide is selected from at least one of maltose, sucrose, and lactose; the five- or six-membered saturated heterocycle containing 1-2 nitrogen atoms is selected from piperazinyl, piperidinyl, and pyrrolidinyl.

24. The connector according to any one of claims 21-23, wherein L₃ is selected from divalent hydrophilic groups with two monovalent radical centers that result from removal of two hydrogen atoms from glucose, galactose, mannose, glucuronic acid, galactonic acid, mannuronic acid, N-acetylglucosamine, N-acetylgalactosamine, N-acetylmannosamine, and N-acetylmuramic acid.

25. The connector according to any one of claims 21-24, wherein L₃ is selected from: 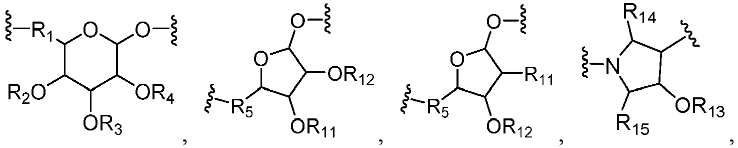 and 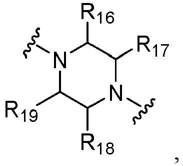 wherein in the formulas,
R₁ is selected from -O(CH₂)ₚ- and -C(=O)-, and p is an integer from 1 to 6, preferably 1, 2, 3, or 4;
R₅ is C₁-C₃ alkylene unsubstituted or substituted with one or more substituents selected from -OR₂₀ and -C(=O)OR₂₀-;
R₂, R₃, R₄, R₁₁, R₁₂, and R₁₃ are independently selected from H, C₁-C₆ alkyl, -(CH₂CH₂O)ₜ-C₁-C₆ alkyl, a sulfonic acid group, and a phosphoric acid group;
R₁₄ and R₁₅ are independently selected from H, -OR₂₀, and -CH₂R₂₀;
R₁₆, R₁₇, R₁₈, and R₁₉ are independently selected from H and -OR₂₀;
R₂₀ is selected from H, C₁-C₆ alkyl, -(CH₂CH₂O)ₜ-C₁-C₆ alkyl, a sulfonic acid group, and a phosphoric acid group;
t is an integer from 1 to 20;
the wavy lines indicate positions where L₃ is connected to X and Y.

26. The connector according to any one of claims 21-25, wherein
L₄ is selected from the following amino acid residues: valine, citrulline, alanine, glycine, phenylalanine, asparagine, glutamic acid, lysine, serine, threonine, cysteine, and tyrosine; the amino acid groups are optionally substituted with one or more -OR₂₀, wherein R₂₀ is selected from H, C₁-C₆ alkyl, -(CH₂CH₂O)ₜ-C₁-C₆ alkyl, a sulfonic acid group, and a phosphoric acid group, wherein t is an integer from 1 to 20; or
L₄ is selected from the following peptide fragments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine, and lysine-phenylalanine, preferably from the following peptide fragments: valine-citrulline (VC), valine-alanine (VA), and glycine-glycine-phenylalanine-glycine (GGFG); each of the peptide fragments is optionally substituted with one or more substituents selected from -OR₂₀ and -(OCH₂CH₂)ₚO-C₁-C₆ alkyl, wherein R₂₀ is selected from H, C₁-C₆ alkyl, -(CH₂CH₂O)ₜ-C₁-C₆ alkyl, a sulfonic acid group, and a phosphoric acid group, t is an integer from 1 to 20, and p is an integer from 1 to 6.

27. The connector according to any one of claims 21-26, wherein L₄ is selected from:

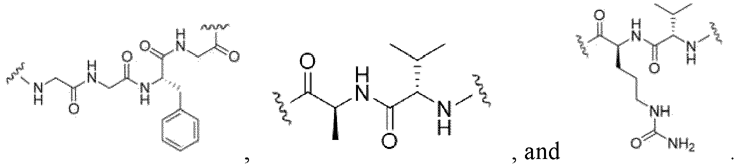

28. The connector according to any one of claims 21-27, wherein
R₆ is H, C₁-C₃ alkoxy, or -(OCH₂CH₂)ₚO-C₁-C₃ alkyl, wherein p is an integer from 1 to 6; preferably, R₆ is H, methoxy, or -(OCH₂CH₂)ₚOCH₃, wherein p is an integer from 1 to 6 or from 1 to 4.

29. The connector according to any one of claims 21-28, wherein the connector is selected from connectors represented by formulas II-1 to XIV-1; in each of the formulas, R, L₂, X, L₃, R₂-R₄, R₁₁-R₁₃, and R₁₅ are as described in any one of claims 1-11.

30. The connector according to any one of claims 21-29, wherein the connector is selected from connectors represented by formulas 1-1 to 46-1.

31. Use, wherein the use is selected from:
use of the linker according to any one of claims 1-13 in the preparation of a linker-drug conjugate or an antibody-drug conjugate, wherein preferably, the linker-drug conjugate is the linker-drug conjugate according to any one of claims 14-17, and the antibody-drug conjugate is the antibody-drug conjugate according to any one of claims 18-20; and
use of the antibody-drug conjugate or the pharmaceutical composition thereof according to any one of claims 18-20 in the preparation of a medicament, wherein preferably, the medicament is used for treating or preventing a disease selected from a cancer, an autoimmune disease, an inflammatory disease, and an infectious disease.
